# EUROPEAN PATENT APPLICATION

(11) **EP 4 282 886 A1**
(43) Date of publication of application: **29.11.2023**
(21) Application number: 22742460.3
(22) Date of filing: 11.01.2022
(51) Int. Cl.: C08F 20/12, G03F 7/004, G03F 7/038, G03F 7/039, G03F 7/20

(54) **ACTINIC-RAY-SENSITIVE OR RADIATION-SENSITIVE RESIN COMPOSITION, ACTINIC-RAY-SENSITIVE OR RADIATION-SENSITIVE FILM, METHOD FOR FORMING PATTERN, METHOD FOR PRODUCING ELECTRONIC DEVICE, COMPOUND, AND RESIN**

(30) Priority: 22.01.2021 JP 2021009187
(71) Applicant: FUJIFILM Corporation, Tokyo 106-8620 (JP)
(72) Inventor: YOSHIMURA Tsutomu, Haibara-gun, Shizuoka 421-0396 (JP); KOJIMA Masafumi, Haibara-gun, Shizuoka 421-0396 (JP); GOTO Akiyoshi, Haibara-gun, Shizuoka 421-0396 (JP); KURUMISAWA Yuma, Haibara-gun, Shizuoka 421-0396 (JP)
(74) Representative: HGF
(86) International application number: PCT/JP2022/000606
(87) International publication number: WO 2022/158338

(57) **Abstract**

Provided are an actinic ray-sensitive or radiation-sensitive resin composition and the like, in which, in a formation of an extremely fine pattern (for example, line-and-space pattern of 30 nm or less, hole pattern of a pore diameter of 30 nm or less, and the like), a roughness performance is excellent and occurrence of pattern defects is suppressed. The actinic ray-sensitive or radiation-sensitive resin composition contains (A) a resin which has a repeating unit derived from a specific compound represented by Formula (1), and (B) a compound which generates an acid by irradiation with an actinic ray or a radiation.

## Description

### BACKGROUND OF THE INVENTION

### 1. Field of the Invention

The present invention relates to an actinic ray-sensitive or radiation-sensitive resin composition, an actinic ray-sensitive or radiation-sensitive film, a pattern forming method, a method for manufacturing an electronic device, a compound and a resin.

### 2. Description of the Related Art

In processes for manufacturing semiconductor devices such as an integrated circuit (IC) and a large scale integrated circuit (LSI), nanofabrication by lithography using a photosensitive composition has been performed.

Examples of the lithographic method include a method in which a resist film is formed with the photosensitive composition, and then the obtained film is exposed and then developed. In particular, in recent years, studies have been conducted on the use of electron beam (EB) and extreme ultraviolet (EUV) light in addition to an ArF excimer laser during exposure, and an actinic ray-sensitive or radiation-sensitive resin composition suitable for EUV exposure has been developed.

In a formation of a resist pattern using EUV (wavelength: 13.5 nm) for the purpose of forming a fine pattern or using electron beam, requirements for various performances are stricter than a case of using ArF (wavelength: 193 nm) light or the like in the related art.

For example, JP2019-99553A discloses a salt represented by a specific formula, a resin having a structural unit derived from the salt, and a resist composition containing the resin and an acid generator.

WO2020/158417A discloses an actinic ray-sensitive or radiation-sensitive resin composition containing a resin and a compound which generates an acid by irradiation with actinic ray or radiation, in which the composition satisfies specific parameters, and the compound is a specific compound.

### SUMMARY OF THE INVENTION

In recent years, miniaturization of a pattern formed by using EUV light or electron beam has been promoted, and further improvement is required in a roughness performance, a defect performance, and the like of the pattern.

Accordingly, an object of the present invention is to provide an actinic ray-sensitive or radiation-sensitive resin composition in which, in a formation of an extremely fine pattern (for example, line-and-space pattern of 30 nm or less, hole pattern of a pore diameter of 30 nm or less, and the like), a roughness performance is excellent and occurrence of pattern defects is suppressed.

Another object of the present invention is to provide an actinic ray-sensitive or radiation-sensitive film formed of the actinic ray-sensitive or radiation-sensitive resin composition, a pattern forming method, and a method for manufacturing an electronic device.

Still another object of the present invention is to provide a compound and a resin.

The present inventors have found that the above-described objects can be achieved by the following configurations.
[1] An actinic ray-sensitive or radiation-sensitive resin composition comprising:
   (A) a resin which has a repeating unit derived from a compound represented by Formula (1); and
   (B) a compound which generates an acid by irradiation with an actinic ray or a radiation. 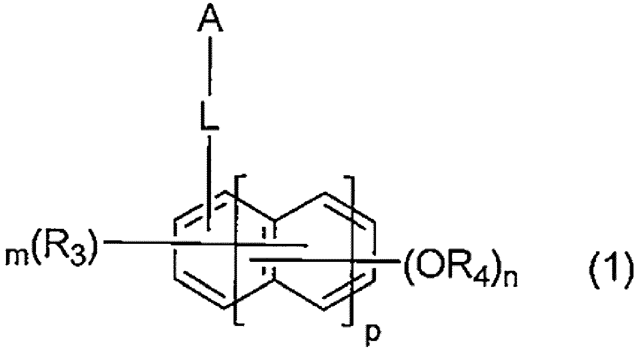 In Formula (1),
      A represents a polymerizable group,
      L represents a divalent linking group having a halogen atom,
      R₃ represents a halogen atom, an amino group, a thiol group, or an organic group,
      R₄ represents a hydrogen atom or an organic group,
      n represents an integer of 1 or more, m represents an integer of 0 or more, and p represents an integer of 0 or more, here, n, m, and p satisfy a relationship of n + m ≤ 5 + 2p, and
      in a case where m represents an integer of 2 or more, a plurality of R₃'s may be the same or different from each other, and in a case where n represents an integer of 2 or more, a plurality of R₄'s may be the same or different from each other.
[2] The actinic ray-sensitive or radiation-sensitive resin composition according to [1],
   in which the compound represented by Formula (1) is a compound represented by Formula (2). 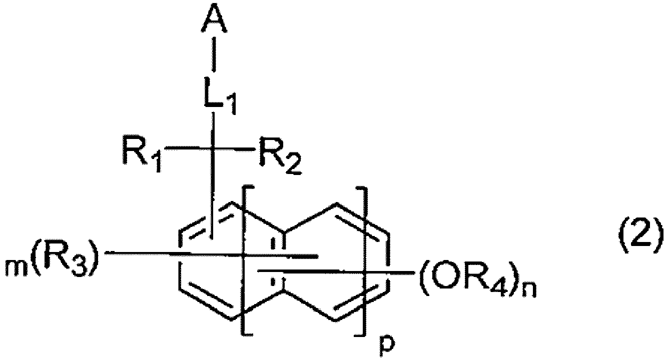 In Formula (2), A has the same meaning as A in Formula (1),
   L₁ represents a single bond or a divalent linking group,
   R₁ represents a halogen atom or an organic group having a halogen atom,
   R₂ represents a hydrogen atom, a halogen atom, a hydroxy group, an amino group, a thiol group, or an organic group, and
   R₃, R₄, n, m, and p have the same meanings as R₃, R₄, n, m, and p in Formula (1), respectively.
[3] The actinic ray-sensitive or radiation-sensitive resin composition according to claim 2,
   in which the compound represented by Formula (2) is a compound represented by Formula (3). 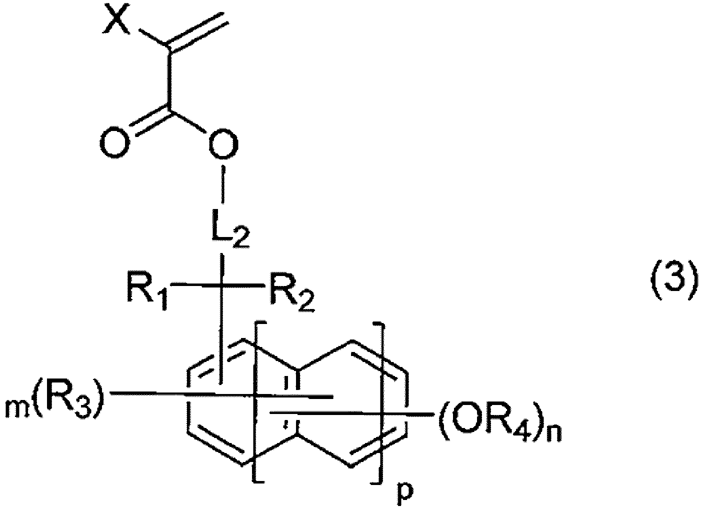 In Formula (3),
   X represents a hydrogen atom or an organic group,
   R₁ and R₂ have the same meanings as R₁ and R₂ in Formula (2), respectively,
   L₂ represents a single bond or a divalent linking group, and
   R₃, R₄, n, m, and p have the same meanings as R₃, R₄, n, m, and p in Formula (1), respectively.
[4] The actinic ray-sensitive or radiation-sensitive resin composition according to any one of [1] to [3],
   in which the compound (B) is an onium salt.
[5] The actinic ray-sensitive or radiation-sensitive resin composition according to any one of [1] to [4],
   in which the resin (A) has a repeating unit represented by Formula (4). 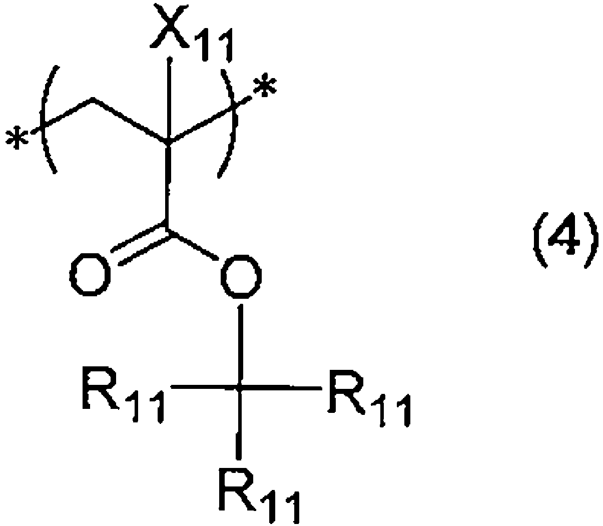 In Formula (4),
   X₁₁ represents a hydrogen atom, a halogen atom, a hydroxy group, or an organic group,
   R₁₁'s each independently represent an alkyl group, and two R₁₁'s may be bonded to each other to form a ring.
[6] An actinic ray-sensitive or radiation-sensitive film formed of the actinic ray-sensitive or radiation-sensitive resin composition according to any one of [1] to [5].
[7] A pattern forming method comprising:
   a step of forming an actinic ray-sensitive or radiation-sensitive film on a substrate using the actinic ray-sensitive or radiation-sensitive resin composition according to any one of [1] to [5];
   a step of exposing the actinic ray-sensitive or radiation-sensitive film; and
   a step of developing the exposed actinic ray-sensitive or radiation-sensitive film with a developer to form a pattern.
[8] A method for manufacturing an electronic device, comprising:
   the pattern forming method according to [7].
[9] A compound represented by Formula (2). 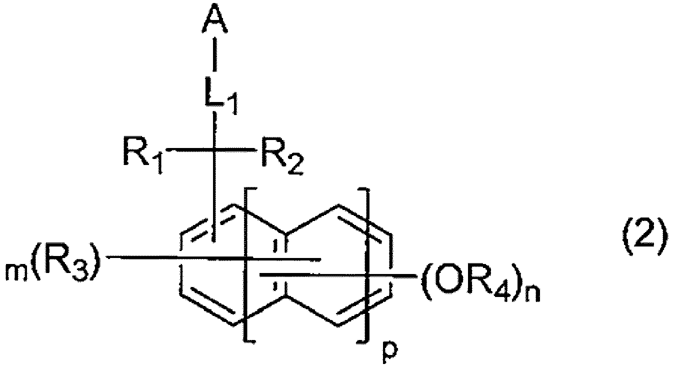 In Formula (2),
   A represents a polymerizable group,
   L₁ represents a single bond or a divalent organic group,
   R₁ represents a halogen atom or an organic group having a halogen atom,
   R₂ represents a hydrogen atom, a halogen atom, a hydroxy group, an amino group, a thiol group, or an organic group,
   R₃ represents a halogen atom, an amino group, a thiol group, or an organic group,
   R₄ represents a hydrogen atom or an organic group,
   n represents an integer of 1 or more, m represents an integer of 0 or more, and p represents an integer of 0 or more, here, n, m, and p satisfy a relationship of n + m ≤ 5 + 2p, and
   in a case where m represents an integer of 2 or more, a plurality of R₃'s may be the same or different from each other, and in a case where n represents an integer of 2 or more, a plurality of R₄'s may be the same or different from each other.
[10] The compound according to [9],
   in which the compound represented by Formula (2) is a compound represented by Formula (3). 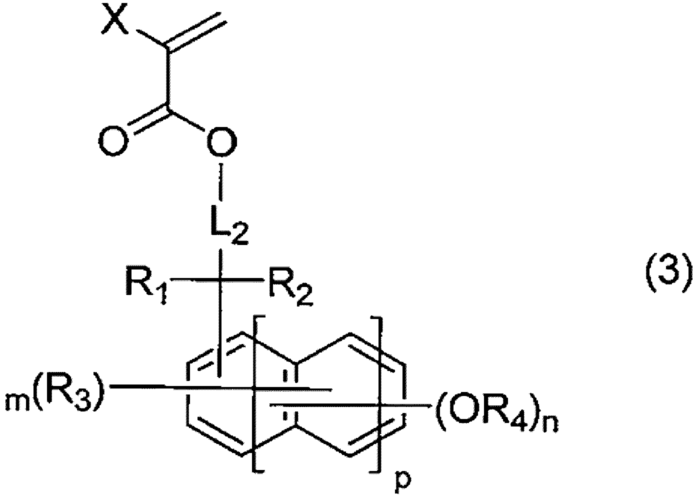 In Formula (3), X represents a hydrogen atom or an organic group,
   R₁ and R₂ have the same meanings as R₁ and R₂ in Formula (2), respectively,
   L₂ represents a single bond or a divalent linking group, and
   R₃, R₄, n, m, and p have the same meanings as R₃, R₄, n, m, and p in Formula (2), respectively.
[11] A resin having a repeating unit derived from the compound according to [9] or [10].

According to the present invention, it is possible to provide an actinic ray-sensitive or radiation-sensitive resin composition in which, in a formation of an extremely fine pattern (for example, line-and-space pattern of 30 nm or less, hole pattern of a pore diameter of 30 nm or less, and the like), a roughness performance is excellent and occurrence of pattern defects is suppressed.

In addition, according to the present invention, it is possible to provide an actinic ray-sensitive or radiation-sensitive film formed of the actinic ray-sensitive or radiation-sensitive resin composition, a pattern forming method, a method for manufacturing an electronic device, a compound, and a resin.

### DESCRIPTION OF THE PREFERRED EMBODIMENTS

Hereinafter, an example of forms for carrying out the present invention will be described.

In the present specification, a numerical value range expressed using "to" means a range that includes the preceding and succeeding numerical values of "to" as a lower limit value and an upper limit value, respectively.

A bonding direction of divalent groups cited in the present specification is not limited unless otherwise specified. For example, in a case where Y in a compound represented by General Formula "X-Y-Z" is -COO-, Y may be -CO-O- or -O-CO-. In addition, the above-described compound may be "X-CO-O-Z" or "X-O-CO-Z".

"(Meth)acryl" in the present specification is a generic term encompassing acryl and methacryl, and means "at least one of acryl or methacryl". Similarly, "(meth)acrylic acid" means "at least one of acrylic acid or methacrylic acid".

"Actinic ray" or "radiation" in the present specification means, for example, a bright line spectrum of a mercury lamp, far ultraviolet rays typified by an excimer laser, extreme ultraviolet rays (EUV light), X-rays, electron beams (EB), or the like. "Light" in the present specification means actinic ray or radiation.

Unless otherwise specified, "exposure" in the present specification encompasses not only exposure by a bright line spectrum of a mercury lamp, far ultraviolet rays typified by an excimer laser, X-rays, EUV light, or the like, but also drawing by particle beams such as electron beams and ion beams.

In the present specification, a weight-average molecular weight (Mw), a number-average molecular weight (Mn), and a dispersity (also referred to as a molecular weight distribution) (Mw/Mn) of a resin are defined as values expressed in terms of polystyrene by means of gel permeation chromatography (GPC) measurement (solvent: tetrahydrofuran, flow amount (amount of a sample injected): 10 µL, columns: TSK gel Multipore HXL-M manufactured by Tosoh Corporation, column temperature: 40°C, flow rate: 1.0 mL/min, and detector: differential refractive index detector) using a GPC apparatus (HLC-8120GPC manufactured by Tosoh Corporation).

1 Å is 1 × 10⁻¹⁰ m.

In the present specification, an acid dissociation constant (pKa) represents a pKa in an aqueous solution, and is specifically a value determined by computation from a value based on a Hammett's substituent constant and database of publicly known literature values, using the following software package 1. Any of the pKa values described in the present specification indicates values determined by computation using the software package.

Software Package 1: Advanced Chemistry Development (ACD/Labs) Software V 8.14 for Solaris (1994 - 2007 ACD/Labs).

On the other hand, the pKa can also be determined by a molecular orbital computation method. Examples of a specific method thereof include a method for performing calculation by computing H⁺ dissociation free energy in a solvent based on a thermodynamic cycle (in the present specification, water is usually used as the solvent, and in a case where a pKa is not determined with water, dimethyl sulfoxide (DMSO) is used).

With regard to a computation method for H⁺ dissociation free energy, the H⁺ dissociation free energy can be computed by, for example, density functional theory (DFT), but various other methods have been reported in literature and the like, and are not limited thereto. There are a plurality of software applications capable of performing DFT, and examples thereof include Gaussian 16.

As described above, the pKa in the present specification refers to a value determined by computation from a value based on a Hammett's substituent constant and database of publicly known literature values, using the software package 1, but in a case where the pKa cannot be calculated by the method, a value obtained by Gaussian 16 based on density functional theory (DFT) shall be adopted.

In notations for a group (atomic group) in the present specification, in a case where the group is cited without specifying that it is substituted or unsubstituted, the group includes both a group having no substituent and a group having a substituent. For example, an "alkyl group" includes not only an alkyl group having no substituent (unsubstituted alkyl group), but also an alkyl group having a substituent (substituted alkyl group). In addition, an "organic group" in the present specification refers to a group including at least one carbon atom.

In addition, in the present specification, the types of substituents, the positions of substituents, and the number of substituents in a case where it is described that "a substituent may be contained" are not particularly limited. The number of substituents may be, for example, one, two, three, or more. Examples of the substituent include a monovalent non-metal atomic group from which a hydrogen atom has been excluded, and the substituent can be selected from the following substituent T, for example.

### (Substituent T)

Examples of the substituent T include halogen atoms such as a fluorine atom, a chlorine atom, a bromine atom, and an iodine atom; alkoxy groups such as a methoxy group, an ethoxy group, and a tert-butoxy group; aryloxy groups such as a phenoxy group and a p-tolyloxy group; alkoxycarbonyl groups such as a methoxycarbonyl group, a butoxycarbonyl group, and a phenoxycarbonyl group; acyloxy groups such as an acetoxy group, a propionyloxy group, and a benzoyloxy group; acyl groups such as an acetyl group, a benzoyl group, an isobutyryl group, an acryloyl group, a methacryloyl group, and a methoxalyl group; alkylsulfanyl groups such as a methylsulfanyl group and a tert-butylsulfanyl group; arylsulfanyl groups such as a phenylsulfanyl group and a p-tolylsulfanyl group; an alkyl group; an alkenyl group; a cycloalkyl group; an aryl group; a heteroaryl group; a hydroxyl group; a carboxyl group; a formyl group; a sulfo group; a cyano group; an alkylaminocarbonyl group; an arylaminocarbonyl group; a sulfonamide group; a silyl group; an amino group; a monoalkylamino group; a dialkylamino group; an arylamino group; a thiol group; and a combination thereof.

### [Actinic Ray-Sensitive or Radiation-Sensitive Resin Composition]

The actinic ray-sensitive or radiation-sensitive resin composition according to the embodiment of the present invention contains a resin (A) having a repeating unit derived from a compound represented by Formula (1), and a compound (B) which generates an acid by irradiation with an actinic ray or a radiation.

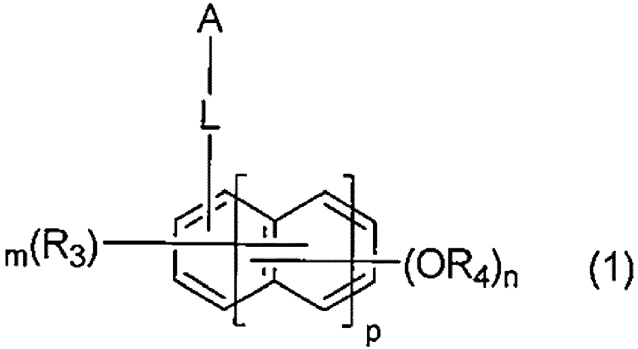

In Formula (1),
A represents a polymerizable group,
L represents a divalent linking group having a halogen atom,
R₃ represents a halogen atom, an amino group, a thiol group, or an organic group,
R₄ represents a hydrogen atom or an organic group,
n represents an integer of 1 or more, m represents an integer of 0 or more, and p represents an integer of 0 or more, here, n, m, and p satisfy a relationship of n + m ≤ 5 + 2p, and
in a case where m represents an integer of 2 or more, a plurality of R₃'s may be the same or different from each other, and in a case where n represents an integer of 2 or more, a plurality of R₄'s may be the same or different from each other.

In the actinic ray-sensitive or radiation-sensitive resin composition according to the embodiment of the present invention, the compound (B) which generates an acid by irradiation with an actinic ray or a radiation and a compound (C) described later may be the same compound.

A mechanism by which the objects of the present invention can be achieved through such configurations is not always clear, but is considered to be as follows by the present inventors.

That is, first, the present inventors have studied in detail on that, in order to achieve the purpose of forming an extremely fine pattern (for example, line-and-space pattern of 30 nm or less, hole pattern of a pore diameter of 30 nm or less, and the like), a halogen atom, which has a particularly large absorption of EUV or electron beam and can be expected to contribute to the achievement of the purpose, is introduced in the resin in the actinic ray-sensitive or radiation-sensitive resin composition (hereinafter, also simply referred to as "composition"), and have considered that, in order to form a good pattern, in an actinic ray-sensitive or radiation-sensitive film (typically, a resist film) formed of the composition, it is important that compatibility between the resin and components other than the resin (acid generator, acid diffusion control agent, and the like) is high.

For example, in a resin having an aromatic ring substituted with a hydroxyl group, with regard to an aspect in which the halogen atom is further substituted to the aromatic ring to introduce the halogen atom into the resin, in the formation of the above-described extremely fine pattern, sufficient roughness performance cannot be obtained and pattern defects are likely to occur. This is because a structure in which the hydroxyl group and the halogen atom are located close to each other is formed in the resin, so that the resins are likely to aggregate with each other through this structure, and it is considered that the mixing of (compatibility between) the resin and the components other than the resin in the resist film is insufficient. More specifically, since the compatibility between the resin and the components other than the resin is insufficient, it is considered that the components other than the resin (acid generator, acid diffusion control agent, and the like) tend to easily aggregate with each other, which leads to easy occurrence of the pattern defects, and in an exposed portion, for example, a reaction between an acid generated from the acid generator and the resin tends to be insufficient, and it is considered that this is related to the fact that the sufficient roughness performance is not obtained.

On the other hand, in the present invention, it is premised that the halogen atom in L of Formula (1) and the group represented by OR₄ of the aromatic ring as a substituent are located at a certain distance from each other. Although the detailed reason is not clear, even in a state in which the repeating unit described from the compound represented by Formula (1) has a halogen atom, compared to the above-described aspect, it is presumed that the resins are difficult to be aggregated with each other.

As a result, since the compatibility between the resin and the components other than the resin in the resist film is high and the aggregation of the components other than the resin (acid generator, acid diffusion control agent, and the like) does not occur easily, defects of the resist are reduced, and in the exposed portion, for example, the reaction between the acid generated from the acid generator and the resin progresses sufficiently, and it is considered that the sufficient roughness performance is obtained.

### [Components of Actinic Ray-Sensitive or Radiation-Sensitive Resin Composition]

Hereinafter, components which can be contained in the actinic ray-sensitive or radiation-sensitive resin composition will be described in detail.

The actinic ray-sensitive or radiation-sensitive resin composition according to the embodiment of the present invention is typically a resist composition, and may be a positive tone resist composition or a negative tone resist composition. In addition, the resist composition may be either a resist composition for alkali development or a resist composition for organic solvent development. The composition according to the embodiment of the present invention is typically a chemically amplified resist composition.

### <(A) Resin>

The actinic ray-sensitive or radiation-sensitive resin composition (hereinafter, also referred to as "composition") contains the resin (A).

The resin (A) is typically an acid-decomposable resin that is a resin in which a polarity increases due to an action of acid, so that a solubility in an alkali developer increases and a solubility in an organic solvent decreases.

The resin (A) has a group which is decomposed by the action of acid to form a polar group (in other words, structure in which a polar group is protected by a leaving group which is eliminated by the action of acid). Such a group (structure) is also called as an acid-decomposable group. A resin having the acid-decomposable group (that is, a resin which has a repeating unit having the acid-decomposable group) has an increased polarity by the action of acid, and thus has an increased solubility in an alkali developer and a decreased solubility in an organic solvent.

### (Repeating Unit Derived From Compound Represented by Formula (1))

The resin (A) has a repeating unit derived from a compound represented by Formula (1) (hereinafter, also referred to as "repeating unit (a1)").

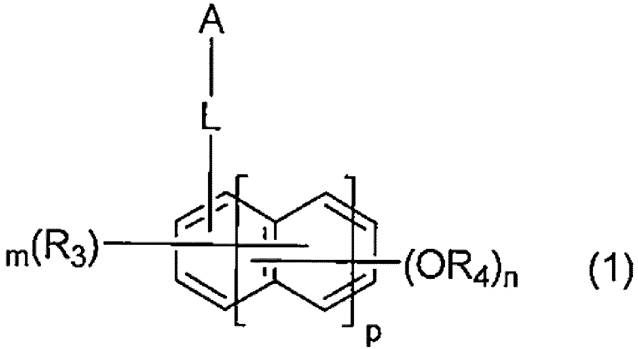

In Formula (1),
A represents a polymerizable group,
L represents a divalent linking group having a halogen atom,
R₃ represents a halogen atom, an amino group, a thiol group, or an organic group,
R₄ represents a hydrogen atom or an organic group,
n represents an integer of 1 or more, m represents an integer of 0 or more, and p represents an integer of 0 or more, here, n, m, and p satisfy a relationship of n + m ≤ 5 + 2p, and
in a case where m represents an integer of 2 or more, a plurality of R₃'s may be the same or different from each other, and in a case where n represents an integer of 2 or more, a plurality of R₄'s may be the same or different from each other.

A positional relationship between L and OR₄ is not particularly limited, but in a case where p is 0, it is preferable that L and OR₄ are in the meta-position or the para-position.

The polymerizable group of A is not particularly limited, and examples thereof include a vinyl group which may have a substituent, and a group represented by Formula (A) is preferable.

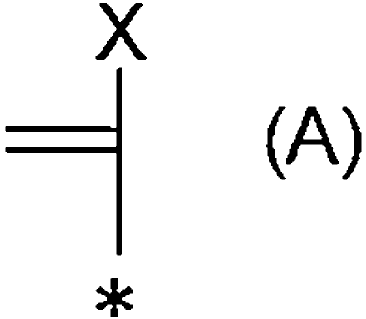

In Formula (A),
X represents a hydrogen atom or an organic group, and
* represents a bonding position.

The organic group is not particularly limited, and examples thereof include an alkyl group, an alkenyl group, a cycloalkyl group, an aryl group, and an alkoxy group.

The alkyl group is not particularly limited, examples thereof include a linear or branched alkyl group having 1 to 12 carbon atoms, and a methyl group or an ethyl group is preferable and a methyl group is more preferable.

The alkenyl group is not particularly limited, examples thereof include a linear or branched alkenyl group having 2 to 12 carbon atoms, and a vinyl group is preferable.

The cycloalkyl group is not particularly limited, and examples thereof include a cycloalkyl group having 3 to 20 carbon atoms. Among these, a monocyclic cycloalkyl group such as a cyclopentyl group and a cyclohexyl group, or a polycyclic cycloalkyl group such as a norbornyl group, a tetracyclodecanyl group, a tetracyclododecanyl group, and an adamantyl group is preferable.

The aryl group is not particularly limited, an aryl group having 6 to 14 carbon atoms is preferable, and examples thereof include a phenyl group, a naphthyl group, and an anthryl group.

The alkoxy group is not particularly limited, examples thereof include a linear or branched alkoxy group having 1 to 12 carbon atoms, and an alkoxy group having 1 to 6 carbon atoms is preferable and an alkoxy group having 1 to 3 carbon atoms is more preferable.

The alkyl group, alkenyl group, cycloalkyl group, aryl group, and alkoxy group may have a substituent.

L represents a divalent linking group having a halogen atom.

Examples of the divalent linking group in the divalent linking group having a halogen atom include -COO-, -CO-, -O-, an alkylene group, a cycloalkylene group, an arylene group, and a linking group in which a plurality of these groups are linked.

The alkylene group is not particularly limited, and may be linear or branched. The number of carbon atoms in the alkylene group is not particularly limited, but is preferably 1 to 10 and more preferably 1 to 3.

The cycloalkylene group is not particularly limited, and may be monocyclic or polycyclic. The number of carbon atoms in the cycloalkylene group is not particularly limited, but is preferably 3 to 10 and more preferably 3 to 6.

The arylene group is not particularly limited, and an arylene group having 6 to 14 carbon atoms is preferable and an arylene group having 6 to 10 carbon atoms is more preferable.

The alkylene group, cycloalkylene group, and arylene group may have a substituent.

As a preferred aspect, the alkylene group, cycloalkylene group, and arylene group may have a halogen atom, a hydroxy group, an amino group, a thiol group, or an organic group. Examples of the organic group include an organic group as R₂ in Formula (2) described later. These groups may further have a substituent.

The divalent linking group having a halogen atom as L has at least one halogen atom, and may have a plurality of halogen atoms.

R₃ represents a halogen atom, an amino group, a thiol group, or an organic group.

Examples of the halogen atom include a fluorine atom, a chlorine atom, a bromine atom, and an iodine atom.

The organic group is not particularly limited, and examples thereof include an alkyl group, an alkenyl group, a cycloalkyl group, and an aryl group.

The alkyl group is not particularly limited, examples thereof include a linear or branched alkyl group having 1 to 12 carbon atoms, and a methyl group or an ethyl group is preferable and a methyl group is more preferable.

The alkenyl group is not particularly limited, examples thereof include a linear or branched alkenyl group having 2 to 12 carbon atoms, and a vinyl group is preferable.

The cycloalkyl group is not particularly limited, and examples thereof include a cycloalkyl group having 3 to 20 carbon atoms. Among these, a monocyclic cycloalkyl group such as a cyclopentyl group and a cyclohexyl group, or a polycyclic cycloalkyl group such as a norbornyl group, a tetracyclodecanyl group, a tetracyclododecanyl group, and an adamantyl group is preferable.

The aryl group is not particularly limited, an aryl group having 6 to 14 carbon atoms is preferable, and examples thereof include a phenyl group, a naphthyl group, and an anthryl group.

The alkyl group, alkenyl group, cycloalkyl group, and aryl group may have a substituent.

The organic group of R₄ has the same meaning as the organic group as R₃ in Formula (1), and the preferred range is also the same.

R₄ is preferably a hydrogen atom.

The aromatic ring in Formula (1) may further have a substituent.

The upper limit of n is not particularly limited, but is usually 5. n is preferably 1 to 3, and more preferably 1 or 2.

The upper limit of m is not particularly limited, but is usually 5. m is preferably 0 to 3, and more preferably 0 to 2.

The upper limit of p is not particularly limited, but is usually 3. p is preferably 0 to 3, and more preferably 0 to 2.

The above-described compound represented by Formula (1) is preferably a compound represented by Formula (2).

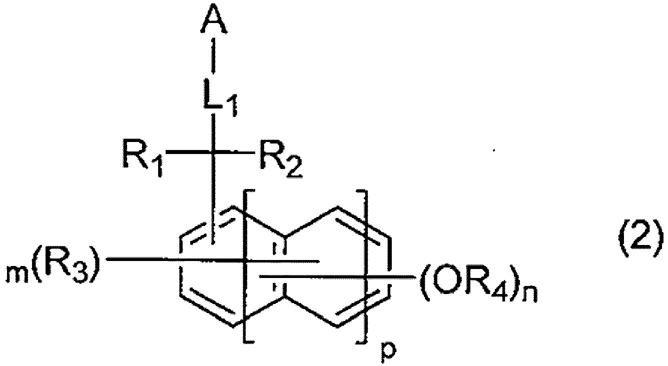

In Formula (2),
A has the same meaning as A in Formula (1),
L₁ represents a single bond or a divalent linking group,
R₁ represents a halogen atom or an organic group having a halogen atom,
R₂ represents a hydrogen atom, a halogen atom, a hydroxy group, an amino group, a thiol group, or an organic group, and
R₃, R₄, n, m, and p have the same meanings as R₃, R₄, n, m, and p in Formula (1), respectively.

In a case where m represents an integer of 2 or more, a plurality of R₃'s may be the same or different from each other, and in a case where n represents an integer of 2 or more, a plurality of R₄'s may be the same or different from each other.

L₁ represents a single bond or a divalent linking group.

Examples of the divalent linking group include -COO-, -CO-, -O-, an alkylene group, a cycloalkylene group, an arylene group, and a linking group in which a plurality of these groups are linked.

The alkylene group is not particularly limited, and may be linear or branched.

The number of carbon atoms in the alkylene group is not particularly limited, but is preferably 1 to 10 and more preferably 1 to 3.

The cycloalkylene group is not particularly limited, and may be monocyclic or polycyclic. The number of carbon atoms in the cycloalkylene group is not particularly limited, but is preferably 3 to 10 and more preferably 3 to 6.

The arylene group is not particularly limited, and an arylene group having 6 to 14 carbon atoms is preferable and an arylene group having 6 to 10 carbon atoms is more preferable.

The alkylene group, cycloalkylene group, and arylene group may have a substituent.

L₁ is preferably a divalent linking group.

R₁ represents a halogen atom or an organic group having a halogen atom.

Examples of the halogen atom include a fluorine atom, a chlorine atom, a bromine atom, and an iodine atom.

The organic group in the organic group having a halogen atom is not particularly limited, and examples thereof include an alkyl group, a cycloalkyl group, an aryl group, and an alkoxy group.

The alkyl group is not particularly limited, examples thereof include a linear or branched alkyl group having 1 to 12 carbon atoms, and an alkyl group having 1 to 6 carbon atoms is preferable and an alkyl group having 1 to 3 carbon atoms is more preferable.

The cycloalkyl group is not particularly limited, and examples thereof include a cycloalkyl group having 3 to 20 carbon atoms. Among these, a monocyclic cycloalkyl group such as a cyclopentyl group and a cyclohexyl group, or a polycyclic cycloalkyl group such as a norbornyl group, a tetracyclodecanyl group, a tetracyclododecanyl group, and an adamantyl group is preferable.

The aryl group is not particularly limited, an aryl group having 6 to 14 carbon atoms is preferable, and examples thereof include a phenyl group, a naphthyl group, and an anthryl group.

The alkoxy group is not particularly limited, examples thereof include a linear or branched alkoxy group having 1 to 12 carbon atoms, and an alkoxy group having 1 to 6 carbon atoms is preferable and an alkoxy group having 1 to 3 carbon atoms is more preferable.

The alkyl group, cycloalkyl group, aryl group, and alkoxy group may further have a substituent. As a preferred aspect, the substituent is not particularly limited, but for example, a halogen atom, a hydroxy group, or an alkyloxycarbonyl group is preferable.

An alkyl group in the alkyloxycarbonyl group is not particularly limited, examples thereof include a linear or branched alkyl group having 1 to 12 carbon atoms, and an alkyl group having 1 to 6 carbon atoms is preferable and an alkyl group having 1 to 3 carbon atoms is more preferable. The alkyloxycarbonyl group may further have a substituent. The substituent is not particularly limited, but for example, a halogen atom is preferable.

The organic group having a halogen atom as R₁ has at least one halogen atom, and may have a plurality of halogen atoms.

R₂ represents a hydrogen atom, a halogen atom, a hydroxy group, an amino group, a thiol group, or an organic group.

Examples of the halogen atom include a fluorine atom, a chlorine atom, a bromine atom, and an iodine atom.

The organic group is not particularly limited, and examples thereof include an alkyl group, a cycloalkyl group, an aryl group, and an alkoxy group.

The alkyl group is not particularly limited, examples thereof include a linear or branched alkyl group having 1 to 12 carbon atoms, and an alkyl group having 1 to 6 carbon atoms is preferable and an alkyl group having 1 to 3 carbon atoms is more preferable.

The cycloalkyl group is not particularly limited, and examples thereof include a cycloalkyl group having 3 to 20 carbon atoms. Among these, a monocyclic cycloalkyl group such as a cyclopentyl group and a cyclohexyl group, or a polycyclic cycloalkyl group such as a norbornyl group, a tetracyclodecanyl group, a tetracyclododecanyl group, and an adamantyl group is preferable.

The aryl group is not particularly limited, an aryl group having 6 to 14 carbon atoms is preferable, and examples thereof include a phenyl group, a naphthyl group, and an anthryl group.

The alkoxy group is not particularly limited, examples thereof include a linear or branched alkoxy group having 1 to 12 carbon atoms, and an alkoxy group having 1 to 6 carbon atoms is preferable and an alkoxy group having 1 to 3 carbon atoms is more preferable.

The alkyl group, cycloalkyl group, aryl group, and alkoxy group may further have a substituent. As a preferred aspect, the substituent is not particularly limited, but for example, a halogen atom, a hydroxy group, or an alkyloxycarbonyl group is preferable.

An alkyl group in the alkyloxycarbonyl group is not particularly limited, examples thereof include a linear or branched alkyl group having 1 to 12 carbon atoms, and an alkyl group having 1 to 6 carbon atoms is preferable and an alkyl group having 1 to 3 carbon atoms is more preferable. The alkyloxycarbonyl group may further have a substituent. The substituent is not particularly limited, but for example, a halogen atom is preferable.

R₂ is preferably a hydrogen atom, a halogen atom, or an organic group.

R₄ is preferably a hydrogen atom.

The aromatic ring in Formula (2) may further have a substituent.

The above-described compound represented by Formula (2) is preferably a compound represented by Formula (3).

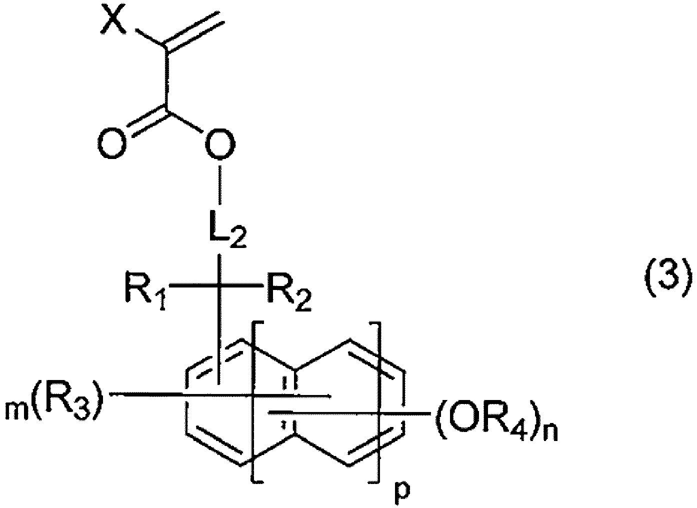

In Formula (3),
X represents a hydrogen atom or an organic group,
R₁ and R₂ have the same meanings as R₁ and R₂ in Formula (2), respectively,
L₂ represents a single bond or a divalent linking group, and
R₃, R₄, n, m, and p have the same meanings as R₃, R₄, n, m, and p in Formula (1), respectively.

In a case where m represents an integer of 2 or more, a plurality of R₃'s may be the same or different from each other. In a case where n represents an integer of 2 or more, a plurality of R₄'s may be the same or different from each other.

X represents a hydrogen atom or an organic group.

The organic group as X has the same meaning as the organic group of X in Formula (A) described above, and the preferred range is also the same.

L₂ represents a single bond or a divalent linking group.

Examples of the divalent linking group include -COO-, -CO-, -O-, an alkylene group, a cycloalkylene group, an arylene group, and a linking group in which a plurality of these groups are linked.

The alkylene group is not particularly limited, and may be linear or branched.

The number of carbon atoms in the alkylene group is not particularly limited, but is preferably 1 to 10 and more preferably 1 to 3.

The cycloalkylene group is not particularly limited, and may be monocyclic or polycyclic. The number of carbon atoms in the cycloalkylene group is not particularly limited, but is preferably 3 to 10 and more preferably 3 to 6.

The arylene group is not particularly limited, and an arylene group having 6 to 14 carbon atoms is preferable and an arylene group having 6 to 10 carbon atoms is more preferable.

The alkylene group, cycloalkylene group, and arylene group may have a substituent.

L₂ is preferably a single bond or an alkylene group.

R₂ is preferably a hydrogen atom, a halogen atom, or an organic group.

R₄ is preferably a hydrogen atom.

The aromatic ring in Formula (3) may further have a substituent.

Examples of the compound represented by Formula (1) are shown below, but the present invention is not limited thereto.

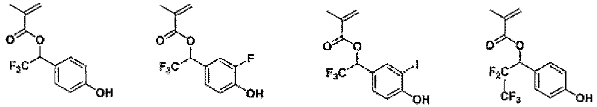

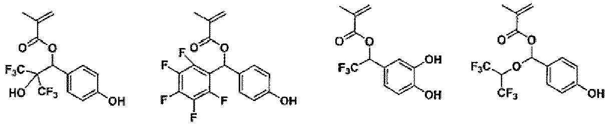

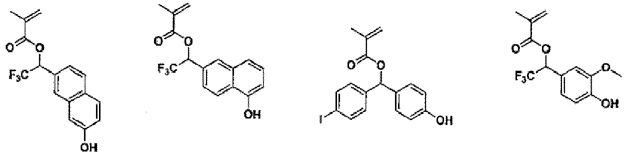

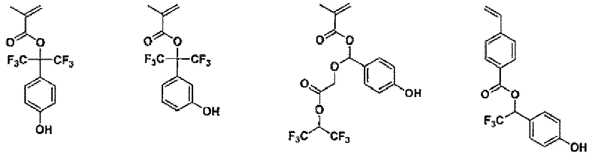

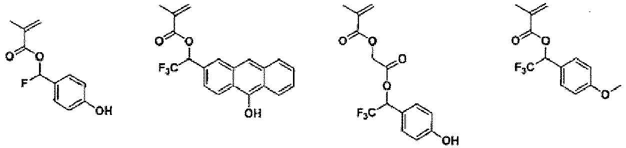

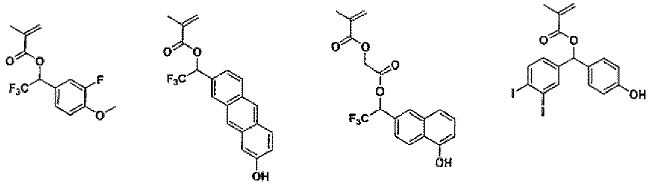

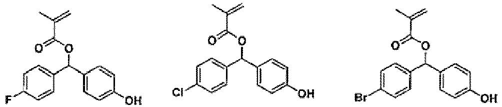

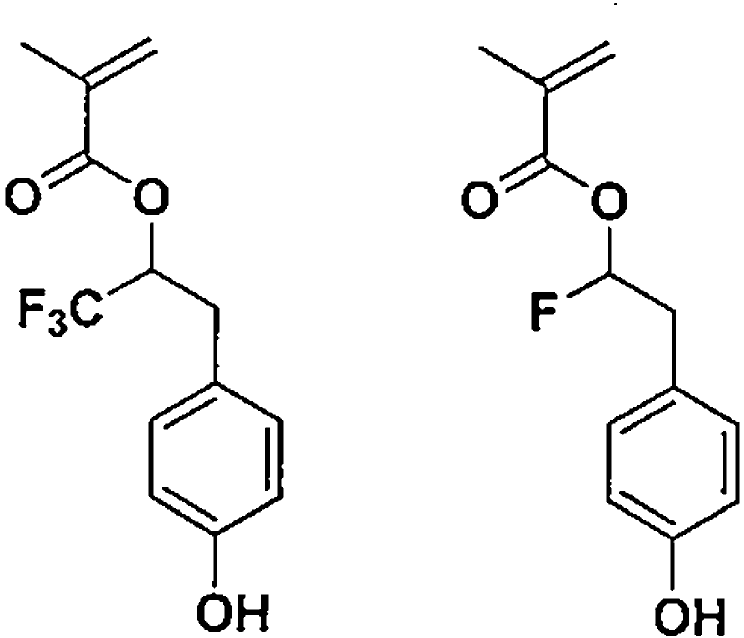

The resin (A) may use one kind of the repeating unit (a1) alone or may use two or more kinds thereof.

In the resin (A), a content of the repeating unit (a1) is preferably 5% to 70% by mole, more preferably 5% to 55% by mole, and still more preferably 5% to 30% by mole with respect to all repeating units of the resin (A).

### (Repeating Unit (a2) having Acid-decomposable Group)

The resin (A) may further include a repeating unit having an acid-decomposable group (also referred to as a "repeating unit (a2)").

The acid-decomposable group preferably has a structure in which a polar group is protected by a group (leaving group) which is eliminated by the action of acid.

Examples of the polar group include an acidic group (typically, a group which dissociates in a 2.38%-by-mass tetramethylammonium hydroxide aqueous solution), such as a carboxyl group, a phenolic hydroxyl group, a fluorinated alcohol group, a sulfonic acid group, a sulfonamide group, a sulfonylimide group, an (alkylsulfonyl)(alkylcarbonyl)methylene group, an (alkylsulfonyl)(alkylcarbonyl)imide group, a bis(alkylcarbonyl)methylene group, a bis(alkylcarbonyl)imide group, a bis(alkylsulfonyl)methylene group, a bis(alkylsulfonyl)imide group, a tris(alkylcarbonyl)methylene group, and a tris(alkylsulfonyl)methylene group; and an alcoholic hydroxyl group.

The alcoholic hydroxyl group is a hydroxyl group bonded to a hydrocarbon group and refers to a hydroxyl group other than a hydroxyl group (phenolic hydroxyl group) directly bonded to an aromatic ring, and an aliphatic alcohol group (for example, a hexafluoroisopropanol group and the like) having an α-position substituted with an electron withdrawing group such as a fluorine atom is excluded as the hydroxyl group. The alcoholic hydroxyl group is preferably a hydroxyl group having an acid dissociation constant (pKa) from 12 to 20.

Among those, as the polar group, a carboxyl group, a phenolic hydroxyl group, a fluorinated alcohol group (preferably, a hexafluoroisopropanol group), or a sulfonic acid group is preferable.

Examples of the group (leaving group) that is eliminated by the action of acid include groups represented by Formulae (Y1) to (Y4).

Formula (Y1): -C(Rx₁)(Rx₂)(Rx₃)

Formula (Y2): -C(=O)OC(Rx₁)(Rx₂)(Rx₃)

Formula (Y3): -C(R₃₆)(R₃₇)(OR₃₈)

Formula (Y4): -C(Rn)(H)(Ar)

In Formula (Y1) and Formula (Y2), Rx₁ to Rx₃ each independently represent a (linear or branched) alkyl group, an (linear or branched) alkenyl group, a (monocyclic or polycyclic) cycloalkyl group, or a (monocyclic or polycyclic) aryl group.

Among these, it is preferable that Rx₁ to Rx₃ each independently represent a linear or branched alkyl group, and it is more preferable that Rx₁ to Rx₃ each independently represent a linear alkyl group.

Two of Rx₁ to Rx₃ may be bonded to each other to form a monocycle or a polycycle.

The alkyl group of Rx₁ to Rx₃ is not particularly limited, and examples thereof include an alkyl group having 1 to 20 carbon atoms. Among these, an alkyl group having 1 to 4 carbon atoms, such as a methyl group, an ethyl group, an n-propyl group, an isopropyl group, an n-butyl group, an isobutyl group, and a t-butyl group is preferable.

The alkenyl group of Rx₁ to Rx₃ is not particularly limited, and examples thereof include an alkenyl group having 2 to 20 carbon atoms. Among these, an alkenyl having 2 to 4 carbon atoms, such as a vinyl group, a propenyl group, and an isopropenyl group, is preferable.

The cycloalkyl group of Rx₁ to Rx₃ is not particularly limited, and examples thereof include a cycloalkyl group having 3 to 20 carbon atoms. Among these, a monocyclic cycloalkyl group such as a cyclopentyl group and a cyclohexyl group, or a polycyclic cycloalkyl group such as a norbornyl group, a tetracyclodecanyl group, a tetracyclododecanyl group, and an adamantyl group is preferable.

As the aryl group of Rx₁ to Rx₃, an aryl group having 6 to 14 carbon atoms is preferable, and examples thereof include a phenyl group, a naphthyl group, and an anthryl group.

The alkyl group, alkenyl group, cycloalkyl group, and aryl group may have a substituent.

As a cycloalkyl group formed by the bonding of two of Rx₁ to Rx₃, a monocyclic cycloalkyl group such as a cyclopentyl group and a cyclohexyl group, or a polycyclic cycloalkyl group such as a norbornyl group, a tetracyclodecanyl group, a tetracyclododecanyl group, and an adamantyl group is preferable, and a monocyclic cycloalkyl group having 5 or 6 carbon atoms is more preferable.

In the cycloalkyl group formed by the bonding of two of Rx₁ to Rx₃, for example, one of methylene groups constituting the ring may be replaced with a heteroatom such as an oxygen atom, or with a group having a heteroatom, such as a carbonyl group.

The cycloalkyl group formed by the bonding of two of Rx₁ to Rx₃ may have a substituent.

In Formula (Y3), R₃₆ to R₃₈ each independently represent a hydrogen atom or a monovalent organic group. R₃₇ and R₃₈ may be bonded to each other to form a ring. Examples of the monovalent organic group include an alkyl group, a cycloalkyl group, an aryl group, an aralkyl group, and an alkenyl group. It is also preferable that R₃₆ is a hydrogen atom.

As Formula (Y3), a group represented by Formula (Y3-1) is preferable.

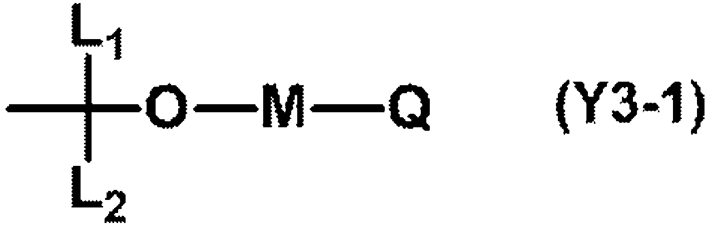

Here, L₁ and L₂ each independently represent a hydrogen atom, an alkyl group, a cycloalkyl group, an aryl group, or a group formed by a combination thereof (for example, a group formed by a combination of an alkyl group and an aryl group).

M represents a single bond or a divalent linking group.

Q represents an alkyl group which may include a heteroatom, a cycloalkyl group which may include a heteroatom, an aryl group which may include a heteroatom, an amino group, an ammonium group, a mercapto group, a cyano group, an aldehyde group, or a group formed by a combination thereof (for example, a group formed by a combination of an alkyl group and a cycloalkyl group).

In the alkyl group and the cycloalkyl group, for example, one of methylene groups may be replaced with a heteroatom such as an oxygen atom or with a group having a heteroatom, such as a carbonyl group.

It is preferable that one of L₁ or L₂ is a hydrogen atom, and the other is an alkyl group, a cycloalkyl group, an aryl group, or a group formed by a combination of an alkylene group and an aryl group.

At least two of Q, M, or L₁ may be bonded to each other to form a ring (preferably a 5- or 6-membered ring).

From the viewpoint of pattern miniaturization, L₂ is preferably a secondary or tertiary alkyl group, and more preferably a tertiary alkyl group. Examples of the secondary alkyl group include an isopropyl group, a cyclohexyl group, and a norbornyl group, and examples of the tertiary alkyl group include a tert-butyl group and an adamantane group. In these aspects, since a glass transition temperature (Tg) and an activation energy are increased, it is possible to suppress fogging in addition to ensuring a film hardness.

In Formula (Y4), Ar represents an aromatic ring group. Rn represents an alkyl group, a cycloalkyl group, or an aryl group. Rn and Ar may be bonded to each other to form a non-aromatic ring. Ar is more preferably an aryl group.

The resin (A) preferably has an acetal structure.

The acid-decomposable group preferably has an acetal structure. The acetal structure is, for example, a structure in which a polar group such as a carboxyl group, a phenolic hydroxyl group, and a fluorinated alcohol group is protected with the group represented by Formula (Y3) described above.

As the repeating unit having an acid-decomposable group, a repeating unit represented by Formula (A) is preferable.

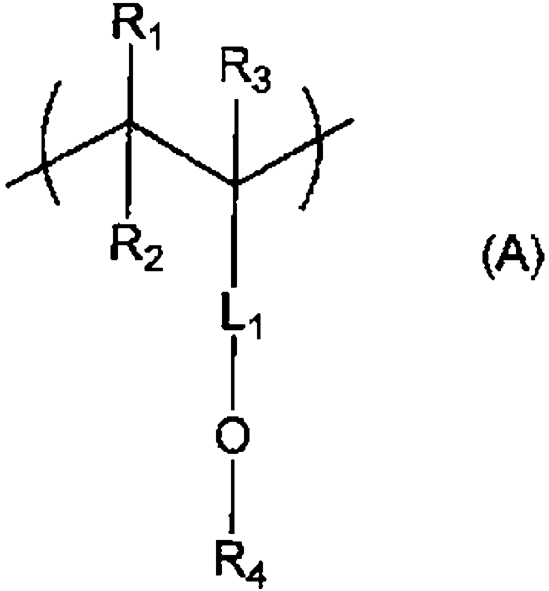

L₁ represents a divalent linking group, R₁ to R₃ each independently represent a hydrogen atom or a monovalent substituent, and R₄ represents a group which is eliminated through decomposition by the action of acid.

L₁ represents a divalent linking group. Examples of the divalent linking group include -CO-, -O-, -S-, -SO-, -SO₂-, a hydrocarbon group (for example, an alkylene group, a cycloalkylene group, an alkenylene group, an arylene group, and the like), and a linking group in which a plurality of these groups are linked. Among these, L₁ is preferably -CO- or an arylene group.

As the arylene group, a phenylene group is preferable.

The alkylene group may be linear or branched. The number of carbon atoms in the alkylene group is not particularly limited, but is preferably 1 to 10 and more preferably 1 to 3.

R₁ to R₃ each independently represent a hydrogen atom or a monovalent substituent. Examples of the monovalent substituent include an alkyl group, a cycloalkyl group, and a halogen atom.

The alkyl group may be linear or branched. The number of carbon atoms in the alkyl group is not particularly limited, but is preferably 1 to 10 and more preferably 1 to 3.

The cycloalkyl group may be monocyclic or polycyclic. The number of carbon atoms in the cycloalkyl group is preferably 3 to 8.

Examples of the halogen atom include a fluorine atom, a chlorine atom, a bromine atom, and an iodine atom.

R₄ represents a group (leaving group) which is eliminated by the action of acid.

Among these, examples of the leaving group include the groups represented by Formulae (Y1) to (Y4) described above, and the group represented by Formula (Y3) described above is preferable.

In a case where each of the above-described groups has a substituent, examples of the substituent include an alkyl group (having 1 to 4 carbon atoms), a halogen atom, a hydroxyl group, an alkoxy group (having 1 to 4 carbon atoms), a carboxyl group, and an alkoxycarbonyl group (having 2 to 6 carbon atoms). The substituent preferably has 8 or less carbon atoms.

As the repeating unit having an acid-decomposable group, a repeating unit represented by General Formula (AI) is also preferable.

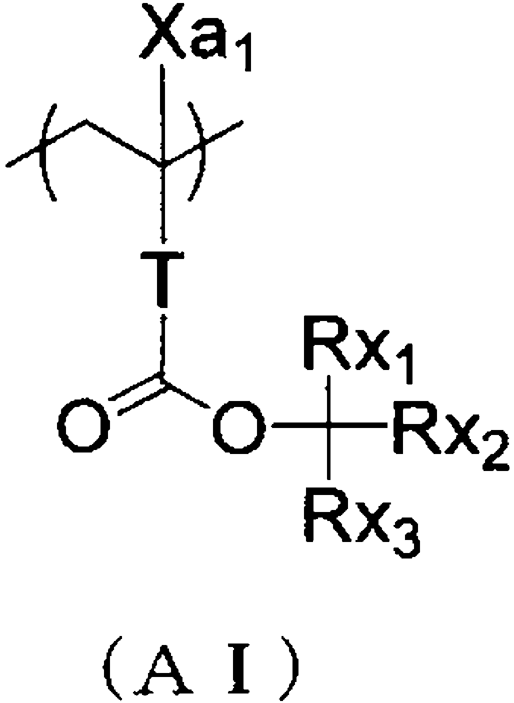

In General Formula (AI),
Xa₁ represents a hydrogen atom or an alkyl group,
T represents a single bond or a divalent linking group, and
Rx₁ to Rx₃ each independently represent a (linear or branched) alkyl group, a (linear or branched) alkenyl group, a (monocyclic or polycyclic) cycloalkyl group, or a (monocyclic or polycyclic) aryl group, here, in a case where all of Rx₁ to Rx₃ are (linear or branched) alkyl groups, it is preferable that at least two of Rx₁, Rx₂, or Rx₃ are methyl groups.

Two of Rx₁ to Rx₃ may be bonded to each other to form a (monocyclic or polycyclic) cycloalkyl group.

Examples of the alkyl group represented by Xa₁ include a methyl group and a group represented by -CH₂-R₁₁. R₁₁ represents a halogen atom (a fluorine atom and the like), a hydroxyl group, or a monovalent organic group, examples thereof include an alkyl group having 5 or less carbon atoms and an acyl group having 5 or less carbon atoms. Among these, an alkyl group having 3 or less carbon atoms is preferable, and a methyl group is more preferable. Xa₁ is preferably a hydrogen atom, a methyl group, a trifluoromethyl group, or a hydroxymethyl group.

Examples of the divalent linking group of T include an alkylene group, an aromatic ring group, a -COO-Rt- group, and an -O-Rt- group. In the formulae, Rt represents an alkylene group or a cycloalkylene group.

T is preferably a single bond or a -COO-Rt- group. In a case where T represents a -COO-Rt-group, Rt is preferably an alkylene group having 1 to 5 carbon atoms, and more preferably a -CH₂- group, a -(CH₂)₂- group, or a -(CH₂)₃- group.

The alkyl group of Rx₁ to Rx₃ is not particularly limited, and examples thereof include an alkyl group having 1 to 20 carbon atoms. Among these, an alkyl group having 1 to 4 carbon atoms, such as a methyl group, an ethyl group, an n-propyl group, an isopropyl group, an n-butyl group, an isobutyl group, and a t-butyl group is preferable.

The alkenyl group of Rx₁ to Rx₃ is not particularly limited, and examples thereof include an alkenyl group having 2 to 20 carbon atoms. Among these, an alkenyl having 2 to 4 carbon atoms, such as a vinyl group, a propenyl group, and an isopropenyl group, is preferable.

The cycloalkyl group of Rx₁ to Rx₃ is not particularly limited, and examples thereof include a cycloalkyl group having 3 to 20 carbon atoms. Among these, a monocyclic cycloalkyl group such as a cyclopentyl group and a cyclohexyl group, or a polycyclic cycloalkyl group such as a norbornyl group, a tetracyclodecanyl group, a tetracyclododecanyl group, and an adamantyl group is preferable.

The aryl group of Rx₁ to Rx₃ is not particularly limited, but an aryl group having 6 to 14 carbon atoms, such as a phenyl group, a naphthyl group, and an anthracenyl group, is preferable.

As the cycloalkyl group formed by the bonding of two of Rx₁ to Rx₃, a monocyclic cycloalkyl group such as a cyclopentyl group and a cyclohexyl group is preferable, and in addition, a polycyclic cycloalkyl group such as a norbornyl group, a tetracyclodecanyl group, a tetracyclododecanyl group, and an adamantyl group is also preferable. Among these, a monocyclic cycloalkyl group having 5 or 6 carbon atoms is preferable.

In the cycloalkyl group formed by the bonding of two of Rx₁ to Rx₃, for example, one of methylene groups constituting the ring may be replaced with a heteroatom such as an oxygen atom, or with a group having a heteroatom, such as a carbonyl group.

With regard to the repeating unit represented by General Formula (AI), for example, an aspect in which Rx₁ is a methyl group or an ethyl group and Rx₂ and Rx₃ are bonded to each other to form the above-described cycloalkyl group is preferable.

In a case where each of the above-described groups has a substituent, examples of the substituent include an alkyl group (having 1 to 4 carbon atoms), a halogen atom, a hydroxyl group, an alkoxy group (having 1 to 4 carbon atoms), a carboxyl group, and an alkoxycarbonyl group (having 2 to 6 carbon atoms). The substituent preferably has 8 or less carbon atoms.

The repeating unit represented by General Formula (AI) is preferably an acid-decomposable tertiary alkyl (meth)acrylate ester-based repeating unit (a repeating unit in which Xa₁ represents a hydrogen atom or a methyl group and T represents a single bond).

As a preferred aspect, the resin (A) contains a repeating unit represented by Formula (4).

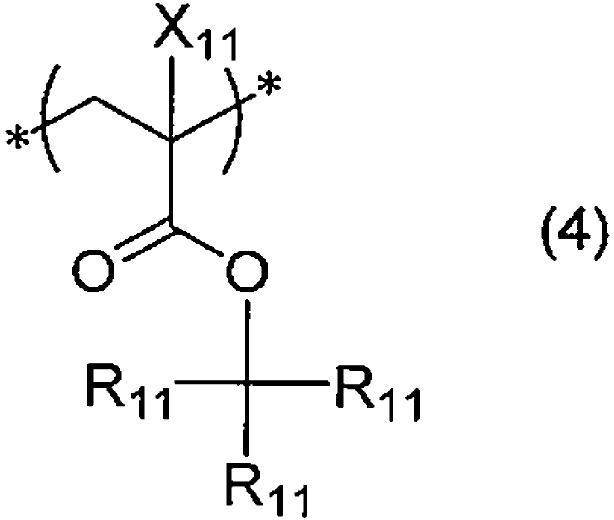

In Formula (4),
X₁₁ represents a hydrogen atom, a halogen atom, a hydroxy group, or an organic group,
R₁₁'s each independently represent an alkyl group, and two R₁₁'s may be bonded to each other to form a ring.

Examples of the halogen atom of X₁₁ include a fluorine atom, a chlorine atom, a bromine atom, and an iodine atom.

The organic group of X₁₁ is not particularly limited, and examples thereof include an alkyl group, a cycloalkyl group, and an aryl group.

The alkyl group is not particularly limited, examples thereof include a linear or branched alkyl group having 1 to 12 carbon atoms, and a methyl group or an ethyl group is preferable and a methyl group is more preferable.

The cycloalkyl group is not particularly limited, and examples thereof include a cycloalkyl group having 3 to 20 carbon atoms. Among these, a monocyclic cycloalkyl group such as a cyclopentyl group and a cyclohexyl group, or a polycyclic cycloalkyl group such as a norbornyl group, a tetracyclodecanyl group, a tetracyclododecanyl group, and an adamantyl group is preferable.

The aryl group is not particularly limited, an aryl group having 6 to 14 carbon atoms is preferable, and examples thereof include a phenyl group, a naphthyl group, and an anthryl group.

The alkyl group, cycloalkyl group, and aryl group may have -O-, -COO-, or an alkylene group.

The alkyl group, cycloalkyl group, and aryl group may further have a substituent. The substituent is not particularly limited, but a halogen atom is preferable as a preferred aspect.

The alkyl group as R₁₁ is not particularly limited, and examples thereof include linear or branched alkyl groups. The number of carbon atoms in the alkyl group is preferably 1 to 12 and more preferably 1 to 10.

The alkyl group may further have a substituent. The substituent is not particularly limited, and examples of a preferred aspect thereof include an alkoxy group (having 1 to 4 carbon atoms), an acyl group (for example, an acetyl group), a hydroxy group, and a halogen atom.

Two R₁₁'s may be bonded to each other to form a ring. Specifically, two R₁₁'s may be bonded to each other to form a (monocyclic or polycyclic) cycloalkyl group.

As the cycloalkyl group formed by the bonding of two R₁₁'s, a monocyclic cycloalkyl group such as a cyclopentyl group and a cyclohexyl group is preferable, and in addition, a polycyclic cycloalkyl group such as a norbornyl group, a tetracyclodecanyl group, a tetracyclododecanyl group, and an adamantyl group is also preferable. Among these, a monocyclic cycloalkyl group having 5 or 6 carbon atoms is preferable.

In the cycloalkyl group formed by the bonding of two R₁₁'s, for example, one of methylene groups constituting the ring may be replaced with a heteroatom such as an oxygen atom, or with a group having a heteroatom, such as a carbonyl group.

The resin (A) may include only one kind of the repeating unit having an acid-decomposable group or a combination of two or more kinds thereof.

A content of the repeating unit having an acid-decomposable group included in the resin (A) (in a case where a plurality of the repeating units having an acid-decomposable group are present, a total content thereof) is preferably 10% to 90% by mole, more preferably 20% to 80% by mole, and still more preferably 25% to 75% by mole with respect to all repeating units of the resin (A).

The total content of the repeating unit (a1) and the repeating unit (a2) (in a case where a plurality of the repeating units (a1) and a plurality of the repeating units (a2) are present, the sum thereof) included in the resin (A) is preferably 60% by mole or more, more preferably 70% or mole or more, and still more preferably 80% by mole or more with respect to all repeating units of the resin (A). In a case where the resin (A) has only the repeating unit (a1) and the repeating unit (a2), the total amount of the repeating unit (a1) and the repeating unit (a2) included in the resin (A) is 100% by mole.

### (Repeating Unit (a3))

It is preferable that the resin (A) further has a repeating unit (a3) having an acid group different from that of the above-described repeating unit (a1).

As the acid group included in the repeating unit (a3), at least one group selected from the group consisting of a carboxylic acid group, a sulfonic acid group, a phenolic hydroxyl group, and a fluoroalkyl alcohol group is preferable, and a phenolic hydroxyl group or a fluoroalkyl alcohol group is more preferable.

### · Repeating Unit having Phenolic Hydroxyl Group

A repeating unit having a phenolic hydroxyl group is preferably a repeating unit represented by General Formula (B2).

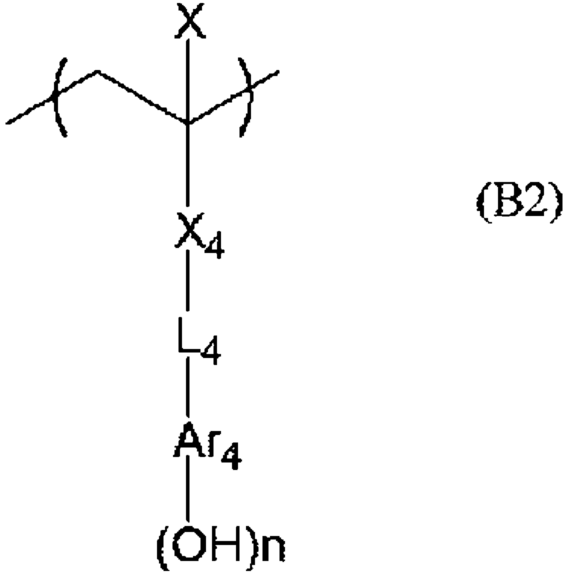

In General Formula (B2), X represents a hydrogen atom, an alkyl group, or a halogen atom.

The above-described alkyl group may be linear or branched. The number of carbon atoms in the above-described alkyl group is preferably 1 to 10.

A substituent of the above-described alkyl group is preferably a hydroxyl group or a halogen atom. In a case where the above-described alkyl group has a substituent, it is preferable that the alkyl group has only a hydroxyl group and/or a halogen atom as the substituent. The above-described alkyl group is preferably -CH₃.

In General Formula (B2), X₄ represents a single bond, -COO-, or -CONR₆₄-, and R₆₄ represents a hydrogen atom or an alkyl group (may be linear or branched; preferably having 1 to 5 carbon atoms). A carbonyl carbon in -COO- is preferably directly bonded to a main chain of the repeating unit.

In General Formula (B2), L₄ represents a single bond or an alkylene group (may be linear or branched; preferably having 1 to 20 carbon atoms).

In General Formula (B2), Ar₄ represents an (n+1)-valent aromatic ring group. As the above-described aromatic ring group, an arylene group having 6 to 18 carbon atoms, such as a benzene ring group, a naphthalene ring group, and an anthracene ring group, or an aromatic ring group including a heterocyclic ring, such as a thiophene ring group, a furan ring group, a pyrrole ring group, a benzothiophene ring group, a benzofuran ring group, a benzopyrrole ring group, a triazine ring group, an imidazole ring group, a benzimidazole ring group, a triazole ring group, a thiadiazole ring group, and a thiazole ring group, is preferable, and a benzene ring group is more preferable.

In General Formula (B2), n represents an integer of 1 to 5. The (n+1)-valent aromatic ring group may further have a substituent.

Examples of a substituent which can be included in the alkyl group of R₆₄, the alkylene group of L₄, and the (n+1)-valent aromatic ring group of Ar₄, each described above, include a halogen atom (preferably, a fluorine atom); alkoxy groups such as a methoxy group, an ethoxy group, a hydroxyethoxy group, a propoxy group, a hydroxypropoxy group, and a butoxy group; and aryl groups such as a phenyl group. In addition, examples of the substituent which can be included in the (n+1)-valent aromatic ring group of Ar₄ also include an alkyl group (may be linear or branched; preferably having 1 to 20 carbon atoms).

Specific examples of the repeating unit having a phenolic hydroxyl group will be shown below, but the present invention is not limited thereto. In the formulae, a represents 1 or 2.

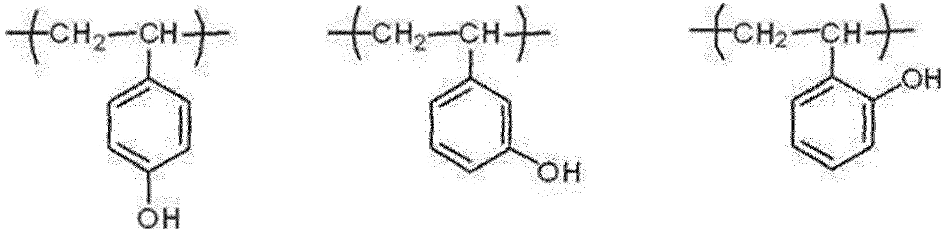

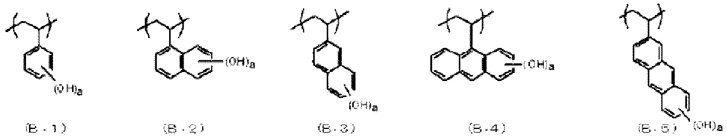

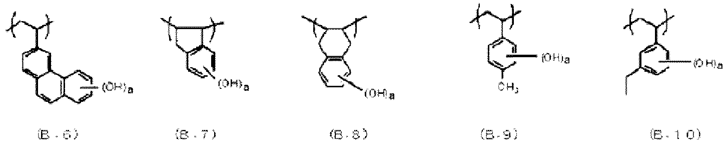

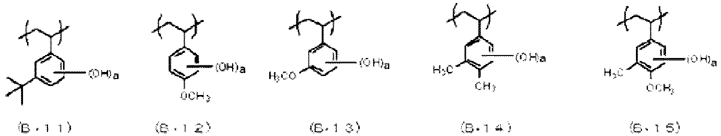

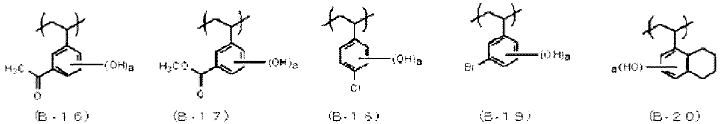

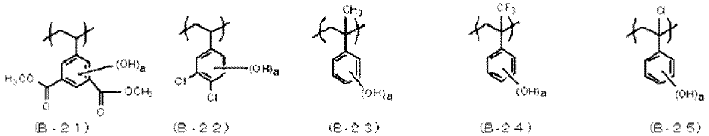

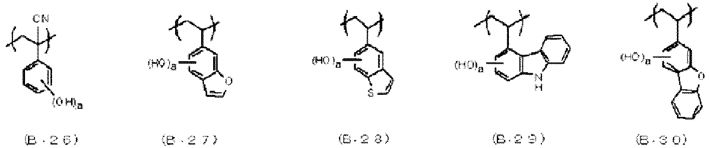

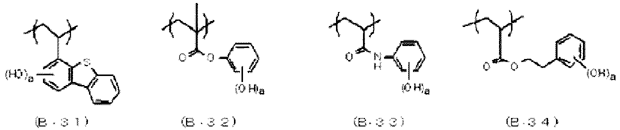

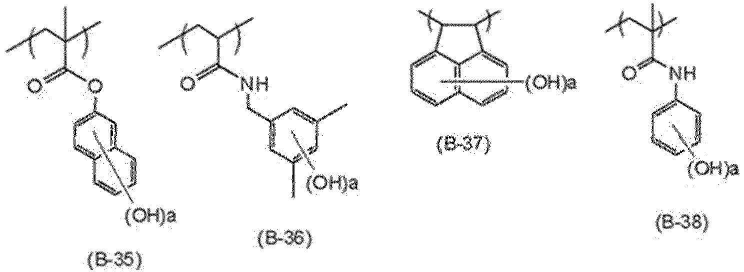

Among the above-described repeating units, repeating units specifically shown below are preferable. In the formulae, R represents a hydrogen atom or a methyl group, and a represents an integer of 1 to 3.

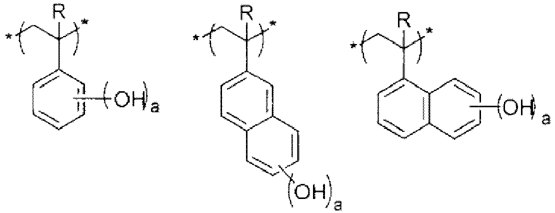

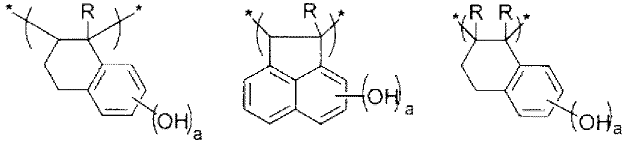

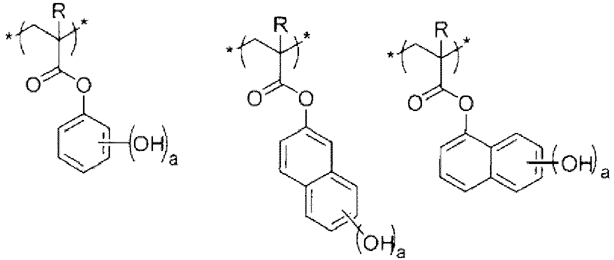

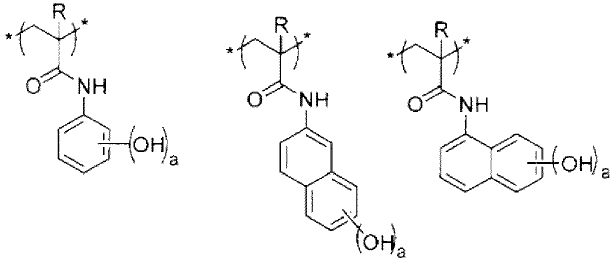

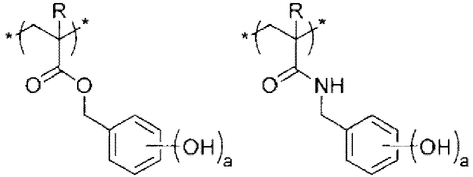

### · Repeating Unit having Fluoroalkyl Alcohol Group

As a repeating unit having a fluoroalkyl alcohol group, a repeating unit having a hexafluoroisopropanol group is preferable.

Examples of a repeating unit corresponding to the repeating unit having a fluoroalkyl alcohol group will be shown below, but the present invention is not limited thereto.

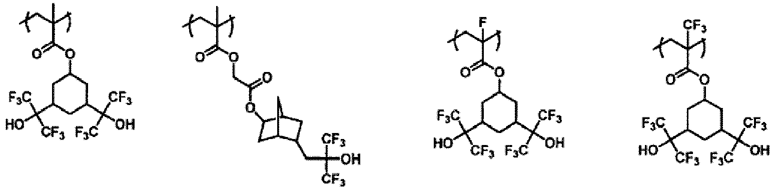

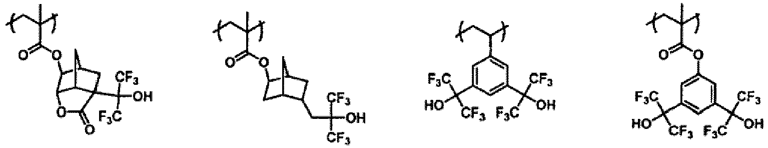

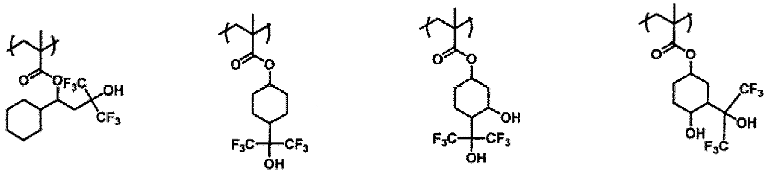

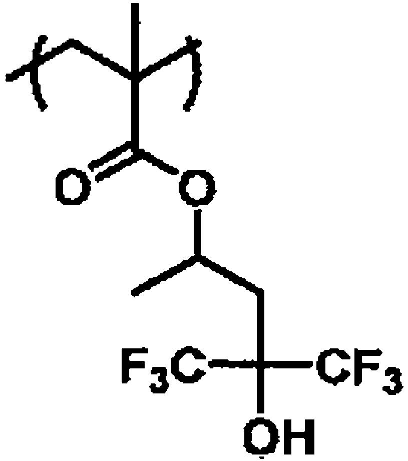

Specific examples of a monomer corresponding to a repeating unit (a3) having an acid group other than the phenolic hydroxyl group and the fluoroalkyl alcohol group will be shown below, but the present invention is not limited thereto.

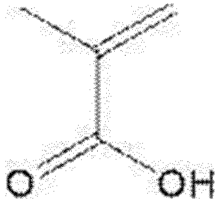

In a case where the resin (A) has the repeating unit (a3), a content thereof is preferably 1% to 30% by mole, more preferably 5% to 25% by mole, and still more preferably 5% to 20% by mole with respect to all repeating units of the resin (A).

The repeating unit (a3) may be used alone or in combination of two or more kinds thereof.

### (Repeating Unit (a4) having Polar Group)

The resin (A) may further include a repeating unit having a polar group (also referred to as "repeating unit (a4)"), which is different from the repeating unit (a1).

As the polar group included in the repeating unit (a4), at least one group selected from the group consisting of an ester group, a sulfonate group, a sulfonamide group, a carbonate group, a carbamate group, an alcoholic hydroxyl group (excluding the fluorinated alkyl alcohol group in the above-described repeating unit (a2)), a sulfoxide group, a sulfonyl group, a ketone group, an imide group, an amide group, a sulfonimide group, a cyano group, a nitro group, and an ether group is preferable; at least one group selected from the group consisting of a lactone group, a sultone group, a sultam group, an alcoholic hydroxyl group (excluding the fluorinated alkyl alcohol group in the above-described repeating unit (a2)), and a cyclic carbonate group is more preferable; a lactone group or a sultone group is still more preferable; and a lactone group is particularly preferable.

That is, the repeating unit (a4) preferably has a lactone structure or a sultone structure, and particularly preferably has a lactone structure.

As the lactone structure or the sultone structure, any structure which has a lactone ring or sultone ring may be used, but a lactone structure having a 5- to 7-membered ring or a sultone structure having a 5- to 7-membered ring is preferable.

A lactone structure in which another ring is fused with the 5- to 7-membered lactone ring so as to form a bicyclo structure or a spiro structure is also preferable. A sultone structure in which another ring is fused with a 5- to 7-membered sultone ring so as to form a bicyclo structure or a spiro structure is also preferable.

Among these, the resin (A) more preferably has a repeating unit having a lactone structure represented by any one of General Formulae (LC1-1) to (LC1-22) or a sultone structure represented by any one of General Formulae (SL1-1) to (SL1-3). In addition, the lactone structure or the sultone structure may be bonded directly to the main chain.

Among these, the lactone structure represented by General Formula (LC1-1), General Formula (LC1-4), General Formula (LC1-5), General Formula (LC1-8), General Formula (LC1-16), General Formula (LC1-21), or General Formula (LC1-22), or the sultone structure represented by General Formula (SL1-1) is preferable.

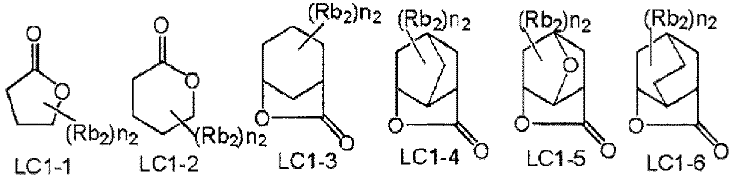

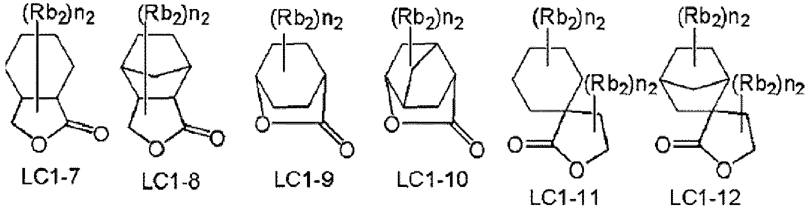

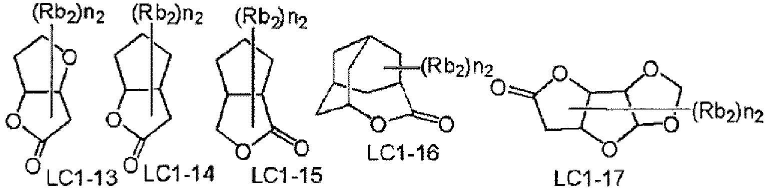

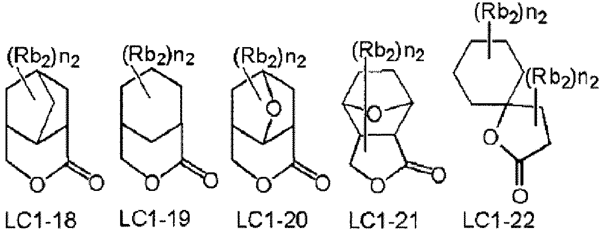

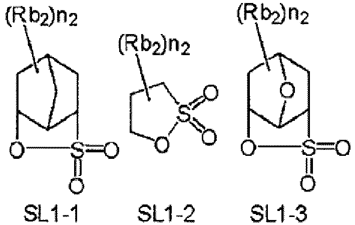

The lactone structure or the sultone structure may or may not have a substituent (Rb₂). As the substituent (Rb₂), an alkyl group having 1 to 8 carbon atoms, a cycloalkyl group having 4 to 7 carbon atoms, an alkoxy group having 1 to 8 carbon atoms, an alkoxycarbonyl group having 2 to 8 carbon atoms, a carboxyl group, a halogen atom, a hydroxyl group, a cyano group, or the like is preferable, and an alkyl group having 1 to 4 carbon atoms or a cyano group is more preferable. n₂ represents an integer of 0 to 4. In a case where n₂ is 2 or more, the substituents (Rb₂) which are present in a plural number may be the same or different from each other. In addition, the substituents (Rb₂) which are present in a plural number may be bonded to each other to form a ring.

As the repeating unit having a lactone structure or a sultone structure, a repeating unit represented by General Formula (LS 1) is preferable.

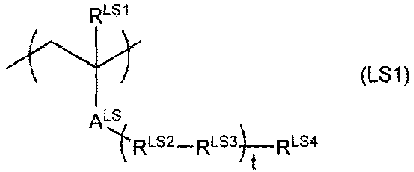

In General Formula (LS1),
A^{LS} represents an ester bond (a group represented by -COO-) or an amide bond (a group represented by -CONH-).
t is a repetition number of the structure represented by -R^{LS2}-R^{LS3}-, represents an integer of 0 to 5, and is preferably 0 or 1 and more preferably 0. In a case where t is 0, (-R^{LS2}-R^{LS3}-)t is a single bond.
R^{LS2} represents an alkylene group, a cycloalkylene group, or a combination thereof. In a case where R^{LS2}'s are present in a plural number, the R^{LS2}'s which are present in a plural number may be the same or different from each other.

The alkylene group or cycloalkylene group of R^{LS2} may have a substituent.

R^{LS3} represents a single bond, an ether bond, an ester bond, an amide bond, a urethane bond, or a urea bond. In a case where R^{LS3}'s are present in a plural number, the R^{LS3}'s which are present in a plural number may be the same or different from each other.

Among these, R^{LS3} is preferably an ether bond or an ester bond, and more preferably an ester bond.

R^{LS4} represents a monovalent organic group having a lactone structure or a sultone structure.

Among these, any of the structures represented by General Formulae (LC1-1) to (LC1-22) and the structures represented by General Formulae (SL1-1) to (SL1-3) described above are preferably a group obtained by removing one hydrogen atom from one carbon atom constituting the lactone structure or the sultone structure. It is preferable that the above-described carbon atom from which one hydrogen atom is removed is not a carbon atom constituting the substituent (Rb₂).

R^{LS1} represents a hydrogen atom, a halogen atom, or a monovalent organic group (preferably a methyl group).

Examples of a monomer corresponding to the repeating unit having at least one selected from the group consisting of a lactone structure and a sultone structure will be shown below.

In the following examples, a methyl group bonded to a vinyl group may be replaced with a hydrogen atom, a halogen atom, or a monovalent organic group.

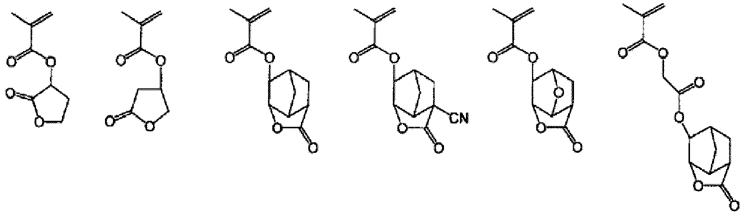

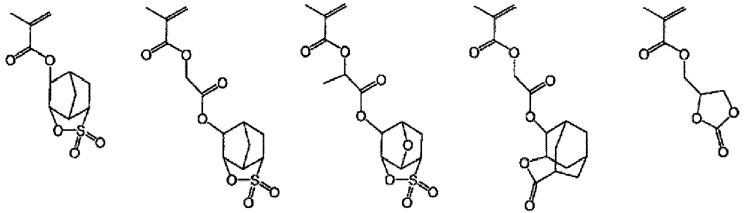

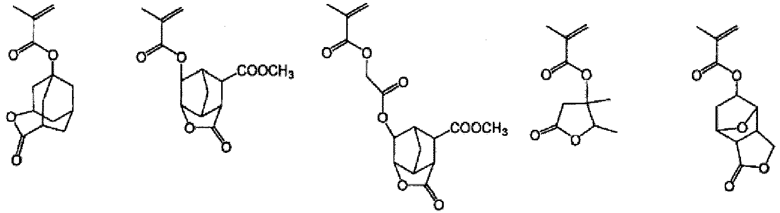

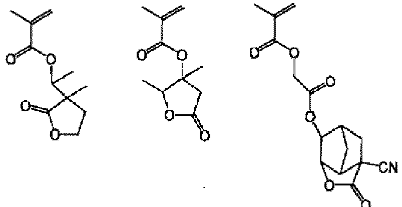

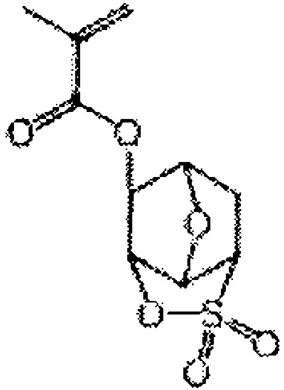

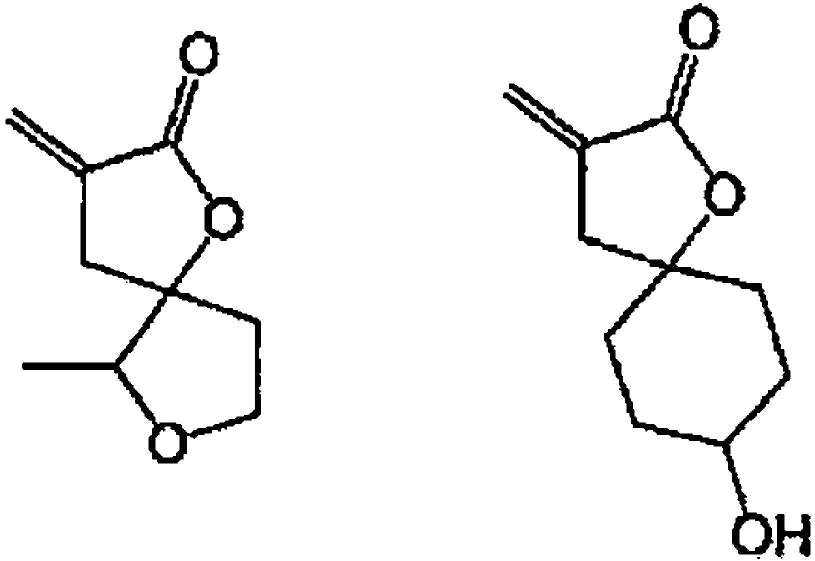

The repeating unit (a4) is also preferably a repeating unit having a carbonate group.

The carbonate group in the repeating unit having a carbonate group is preferably included in a cyclic carbonate ester group.

The repeating unit having a carbonate group is preferably a repeating unit represented by General Formula (A4).

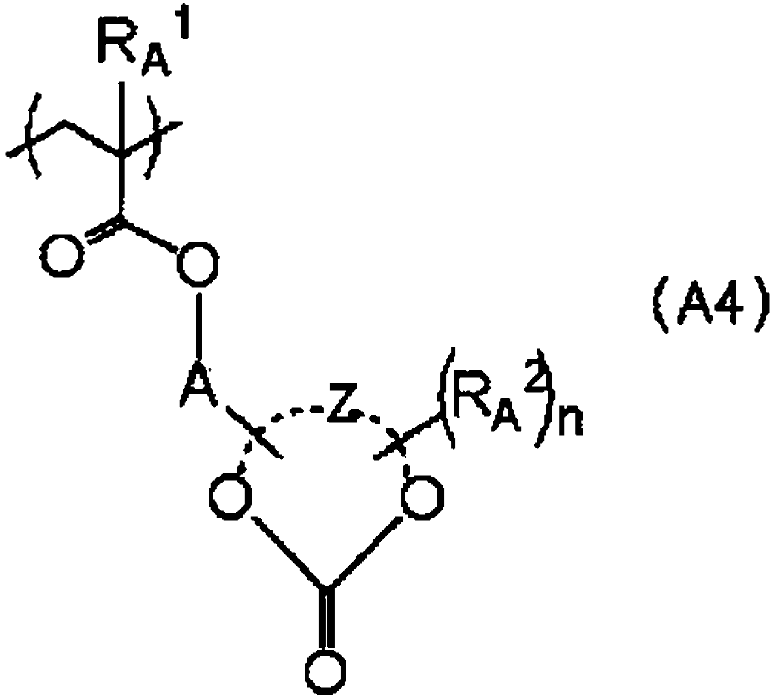

In General Formula (A4),
R_{A}¹ represents a hydrogen atom, a halogen atom, or a monovalent organic group (preferably a methyl group).
n represents an integer of 0 or more (preferably 0 to 3).
R_{A}² represents a substituent. In a case where n is 2 or more, R_{A}² which are present in a plural number may be the same or different from each other.
A represents a single bond or a divalent linking group. As the above-described divalent linking group, an alkylene group (preferably having 1 to 4 carbon atoms), a divalent linking group having a monocyclic or polycyclic alicyclic hydrocarbon structure, an ether group, an ester group, a carbonyl group, a carboxylic acid group, or a divalent group formed by a combination thereof is preferable.
Z represents an atomic group which forms a monocycle or polycycle with a group represented by -O-CO-O- in the formula.

The monocycle or polycycle which is formed by Z with the group represented by -O-CO-O- in the formula is preferably a 5-membered cyclic carbonate ester group, and more preferably a cyclic carbonate ester structure represented by General Formula (CC1-1).

That is, it is preferable that A in General Formula (A4) is bonded to a group formed by removing one hydrogen atom from a ring member atom of the cyclic carbonate ester structure represented by General Formula (CC1-1).

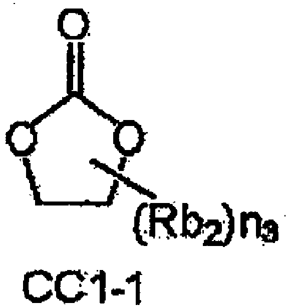

A cyclic carbonate ester structure moiety in General Formula (CC1-1) may have the substituent (Rb2). Preferred examples of the substituent (Rb2) include an alkyl group having 1 to 8 carbon atoms, a cycloalkyl group having 4 to 7 carbon atoms, an alkoxy group having 1 to 8 carbon atoms, an alkoxycarbonyl group having 1 to 8 carbon atoms, a carboxylic acid group, a halogen atom (preferably a fluorine atom), a hydroxyl group, and a cyano group. n3 represents an integer of 0 or 1.

The resin (A) may have a repeating unit having a polar group other than the lactone group, sultone group, or carbonate group described above. The repeating unit having a polar group other than the lactone group, sultone group, or carbonate group described above is preferably a repeating unit having an alicyclic hydrocarbon structure substituted with the above-described polar group. With regard to the alicyclic hydrocarbon structure substituted with the polar group, the alicyclic hydrocarbon structure is preferably a cyclohexyl group, an adamantyl group, or a norbornane group.

Specific examples of a monomer corresponding to the repeating unit having a polar group will be shown below, but the present invention is not limited to these specific examples.

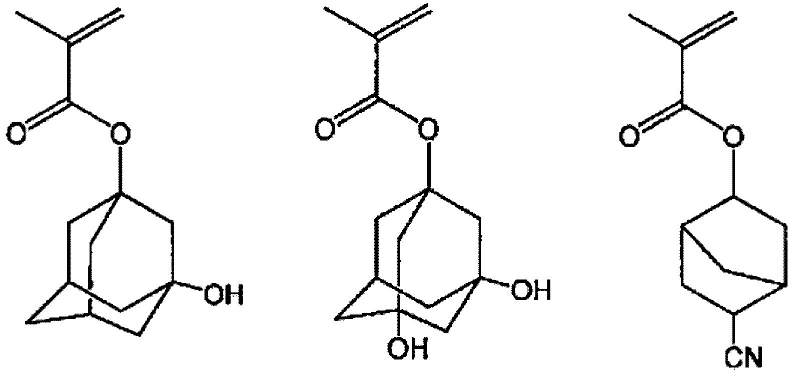

It is also preferable that the resin (A) has, as the repeating unit (a4), a repeating unit described in paragraphs [0370] to [0433] of the specification of US2016/0070167A1.

In a case where the resin (A) includes the repeating unit (a4), the type of the repeating unit (a4) included in the resin (A) may be one kind or two or more kinds.

In a case where the resin (A) includes the repeating unit (a4), a content of the repeating unit (a4) included in the resin (A) (in a case where a plurality of the repeating units (a4) are present, a total content thereof) is preferably 5% to 50% by mole, more preferably 10% to 45% by mole, and still more preferably 10% to 30% by mole with respect to all repeating units of the resin (A).

### (Other Repeating Units)

In addition, the resin (A) may further include a repeating unit (a5) represented by Formula (D).

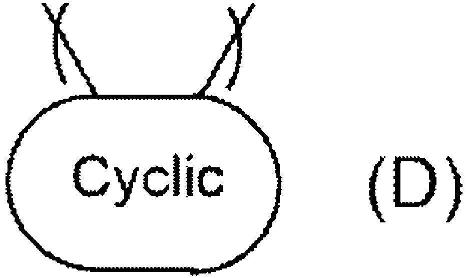

In Formula (D), "cyclic" is a group which forms a main chain as a cyclic structure. The number of ring-constituting atoms is not particularly limited.

Examples of the repeating unit represented by Formula (D) include the following repeating units.

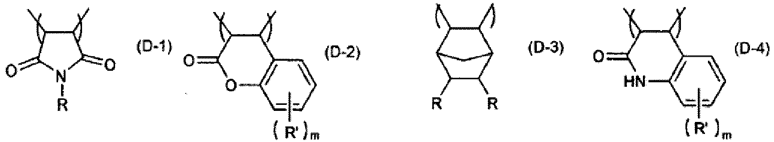

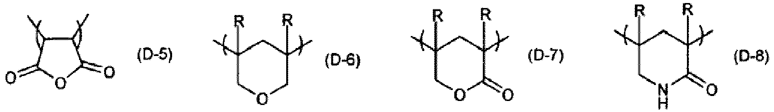

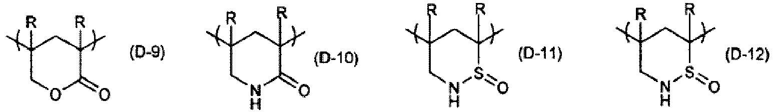

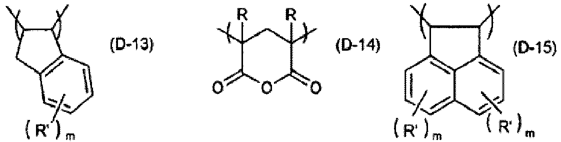

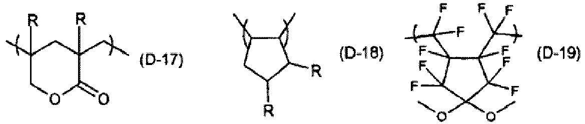

In the formulae, R's each independently represent a hydrogen atom, an alkyl group, a cycloalkyl group, an aryl group, an aralkyl group, an alkenyl group, a hydroxyl group, an alkoxy group, an acyloxy group, a cyano group, a nitro group, an amino group, a halogen atom, an ester group (-OCOR" or -COOR"; R" is an alkyl group or fluorinated alkyl group having 1 to 20 carbon atoms), or a carboxylic acid group. The alkyl group, the cycloalkyl group, the aryl group, the aralkyl group, and the alkenyl group described above may each have a substituent. In addition, the hydrogen atom bonded to the carbon atom in the group represented by R may be substituted with a fluorine atom or an iodine atom. In the formula, R"s each independently represent an alkyl group, a cycloalkyl group, an aryl group, an aralkyl group, an alkenyl group, a hydroxyl group, an alkoxy group, an acyloxy group, a cyano group, a nitro group, an amino group, a halogen atom, an ester group (-OCOR" or -COOR"; R" is an alkyl group or fluorinated alkyl group having 1 to 20 carbon atoms), or a carboxylic acid group. The alkyl group, the cycloalkyl group, the aryl group, the aralkyl group, and the alkenyl group described above may each have a substituent. In addition, a hydrogen atom bonded to the carbon atom in the group represented by R' may be substituted with a fluorine atom or an iodine atom.
m represents an integer of 0 or more. An upper limit of m is not particularly limited, but is 2 or less in many cases and 1 or less in more cases.

In a case where the resin (A) includes a repeating unit (repeating unit (a5)) represented by Formula (D), a content thereof is preferably 1% to 50% by mole, more preferably 3% to 40% by mole, and still more preferably 5% to 30% by mole with respect to all repeating units of the resin (A).

In addition, the resin (A) may further include a repeating unit (a6) represented by Formula (E).

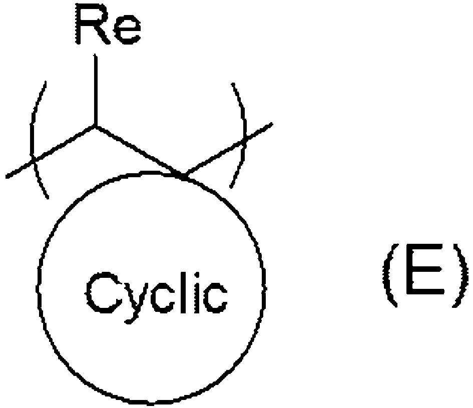

In Formula (E), Re's each independently represent a hydrogen atom or an organic group. Examples of the organic group include an alkyl group, a cycloalkyl group, an aryl group, an aralkyl group, and an alkenyl group, which may have a substituent.

"cyclic" is a cyclic group including a carbon atom of a main chain. The number of atoms included in the cyclic group is not particularly limited.

Examples of the repeating unit represented by Formula (E) include the following repeating units.

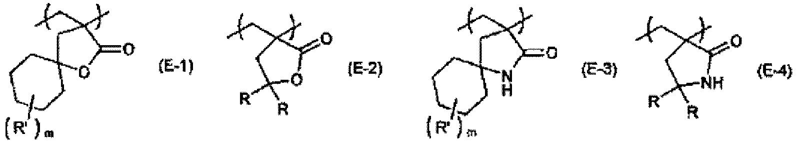

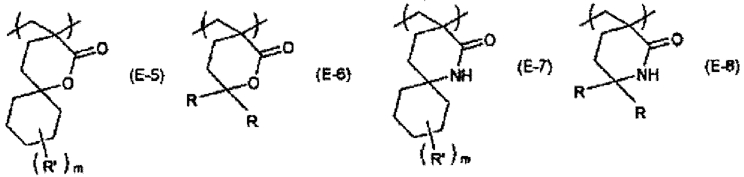

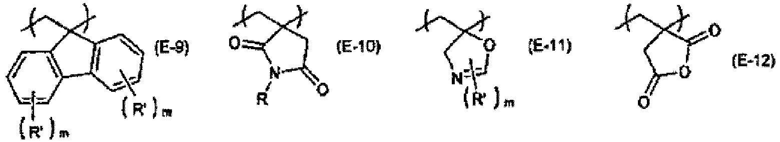

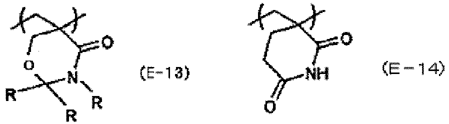

In the formula, R's each independently represent a hydrogen atom, an alkyl group, a cycloalkyl group, an aryl group, an aralkyl group, an alkenyl group, a hydroxyl group, an alkoxy group, an acyloxy group, a cyano group, a nitro group, an amino group, a halogen atom, an ester group (-OCOR" or -COOR"; R" is an alkyl group or fluorinated alkyl group having 1 to 20 carbon atoms), or a carboxylic acid group. The alkyl group, the cycloalkyl group, the aryl group, the aralkyl group, and the alkenyl group described above may each have a substituent. In addition, the hydrogen atom bonded to the carbon atom in the group represented by R may be substituted with a fluorine atom or an iodine atom.

R"s each independently represent a hydrogen atom, an alkyl group, a cycloalkyl group, an aryl group, an aralkyl group, an alkenyl group, a hydroxyl group, an alkoxy group, an acyloxy group, a cyano group, a nitro group, an amino group, a halogen atom, an ester group (-OCOR" or -COOR"; R" is an alkyl group or fluorinated alkyl group having 1 to 20 carbon atoms), or a carboxylic acid group. The alkyl group, the cycloalkyl group, the aryl group, the aralkyl group, and the alkenyl group described above may each have a substituent. In addition, a hydrogen atom bonded to the carbon atom in the group represented by R' may be substituted with a fluorine atom or an iodine atom.
m represents an integer of 0 or more. An upper limit of m is not particularly limited, but is 2 or less in many cases and 1 or less in more cases.

In addition, in Formula (E-2), Formula (E-4), Formula (E-6), Formula (E-8), and Formula (E-12), two R's may be bonded to each other to form a ring.

In a case where the resin (A) includes the repeating unit (a6) represented by Formula (E), a content thereof is preferably 1% to 50% by mole, more preferably 3% to 40% by mole, and still more preferably 5% to 30% by mole with respect to all repeating units of the resin (A).

The resin (A) may include, as the other repeating units different from the repeating unit (a1), a repeating unit other than the above-described repeating units as long as the effects of the present invention are not impaired, and for example, may have a repeating unit having a photoacid generating group.

The repeating unit having a photoacid generating group is not particularly limited, but is preferably a repeating unit (repeating unit (a7)) represented by General Formula (A7).

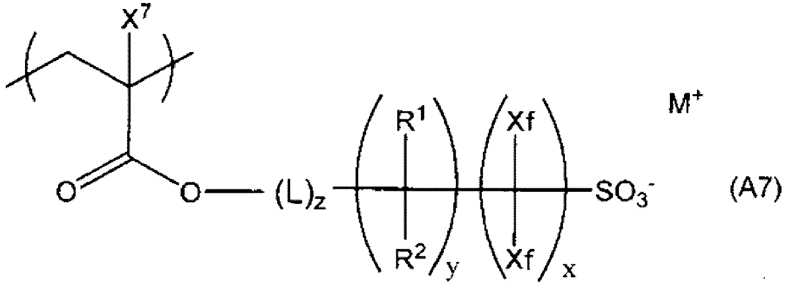

In General Formula (A7), two Xf's each independently represent a hydrogen atom, a fluorine atom, or an alkyl group substituted with at least one fluorine atom ((preferably CF₃). At least one of two Xf's is preferably a non-hydrogen atom.

The above-described alkyl group may be linear or branched. The number of carbon atoms in the above-described alkyl group is preferably 1 to 10. The above-described alkyl group preferably has only a fluorine atom as a substituent.

In General Formula (A7), R¹ and R² each independently represent a hydrogen atom, a fluorine atom, or an alkyl group. In a case where there is a plurality of R¹'s and R²'s, R¹'s and R²'s may each be the same or different from each other.

The above-described alkyl group may be linear or branched. The number of carbon atoms in the above-described alkyl group is preferably 1 to 10. A substituent of the above-described alkyl group is preferably a fluorine atom. In a case where the above-described alkyl group has a substituent, it is preferable that the alkyl group has only a fluorine atom as the substituent.

In General Formula (A7), L represents a divalent linking group, and in a case where a plurality of L's are present, L's may be the same or different from each other.

Examples of the divalent linking group of L include -COO-, -CO-, -O-, -S-, -SO-, -SO₂-, an alkylene group, a cycloalkylene group, an alkenylene group, a linking group consisting of a plurality of these groups linked to each other, and the like. The total number of carbon atoms in these linking groups is preferably 12 or less.

In General Formula (A7), x represents an integer of 1 to 20, y represents an integer of 0 to 10, and z represents an integer of 0 to 10.

In General Formula (A7), X⁷ represents a hydrogen atom, an alkyl group, or a halogen atom.

Examples of the alkyl group in a case where X⁷ represents an alkyl group include a linear or branched alkyl group having 1 to 12 carbon atoms, and a methyl group, an ethyl group, an i-propyl group, or an n-propyl group is preferable, a methyl group or an ethyl group is more preferable, and a methyl group is still more preferable.

In a case where the above-described alkyl group has a substituent, examples of the substituent include substituents described in the above substituent T.

Examples of the halogen atom in a case where X⁷ represents a halogen atom include a fluorine atom, a chlorine atom, a bromine atom, and an iodine atom, and a fluorine atom is preferable.

In General Formula (A7), M⁺ represents a cation. As for details of M⁺, for example, the same cations as M⁺ in General Formula (PA-1) described later can be used.

In a case where the resin (A) has the repeating unit (a7), a content thereof is preferably 1% to 40% by mole, more preferably 1% to 25% by mole, and still more preferably 1% to 15% by mole with respect to all repeating units of the resin (A).

The repeating unit (a7) may be used alone or in combination of two or more kinds thereof.

The resin (A) can be synthesized in accordance with an ordinary method (for example, a radical polymerization).

A weight-average molecular weight of the resin (A) as a value expressed in terms of polystyrene by a GPC method is preferably 1,000 to 200,000, more preferably 3,000 to 20,000, and still more preferably 4,500 to 15,000. In a case of setting the weight-average molecular weight of the resin (A) to 1,000 to 200,000, it is possible to prevent deterioration of heat resistance and dry etching resistance, and it is also possible to prevent deterioration of film-forming properties due to deterioration of developability and an increase in viscosity.

A dispersity (molecular weight distribution) of the resin (A) is usually 1 to 5, preferably 1 to 3, more preferably 1.2 to 3.0, and still more preferably 1.2 to 2.0. As the dispersity is smaller, resolution and resist shape are excellent, and a side wall of a resist pattern is smoother and roughness is excellent.

In the composition according to the embodiment of the present invention, a content of the resin (A) is preferably 10.0% to 99.9% by mass, and more preferably 20.0% to 99.0% by mass in the total solid content.

In addition, as a preferred aspect, in the composition according to the embodiment of the present invention, the content of the resin (A) is preferably 25.0% to 99.9% by mass, and more preferably 60.0% to 99.0% by mass in the total solid content.

In addition, the resin (A) may be used alone or in combination of two or more kinds thereof.

As long as effects of the present invention are not impaired, the composition according to the embodiment of the present invention may contain a resin (also referred to as a resin (A')) not having the repeating unit (a1), in addition to the resin (A).

The resin (A') is not particularly limited as long as it is a resin which does not have the repeating unit (a1), and examples thereof include a resin which, in the resin (A), does not have the repeating unit (a1).

In a case where the composition according to the embodiment of the present invention contains the resin (A'), a ratio of the content of the resin (A) and the content of the resin (A') in the composition according to the embodiment of the present invention is preferably 9:1 to 8:2 in a mass ratio.

### [Compound Represented by Formula (2)]

The present invention also relates to a compound represented by Formula (2).

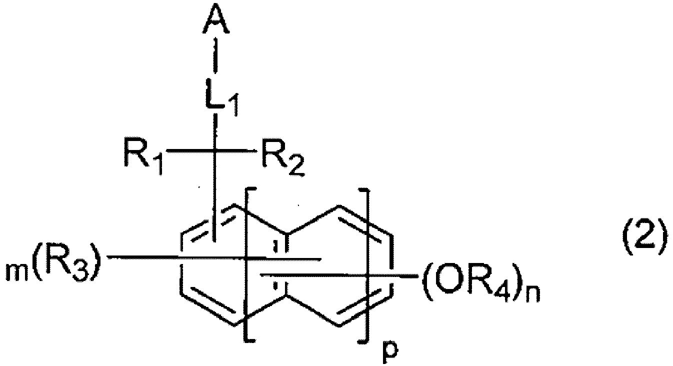

In Formula (2),
A represents a polymerizable group,
L₁ represents a single bond or a divalent organic group,
R₁ represents a halogen atom or an organic group having a halogen atom,
R₂ represents a hydrogen atom, a halogen atom, a hydroxy group, an amino group, a thiol group, or an organic group,
R₃ represents a halogen atom, an amino group, a thiol group, or an organic group,
R₄ represents a hydrogen atom or an organic group,
n represents an integer of 1 or more, m represents an integer of 0 or more, and p represents an integer of 0 or more, here, n, m, and p satisfy a relationship of n + m ≤ 5 + 2p, and
in a case where m represents an integer of 2 or more, a plurality of R₃'s may be the same or different from each other, and in a case where n represents an integer of 2 or more, a plurality of R₄'s may be the same or different from each other.

A, L₁, R₁, R₂, R₃, R₄, n, m, and p have the same meanings as A, L₁, R₁, R₂, R₃, R₄, n, m, and p in Formula (2) of the above-described resin (A) contained in the composition according to the embodiment of the present invention, respectively.

The above-described compound represented by Formula (2) is preferably a compound represented by Formula (3).

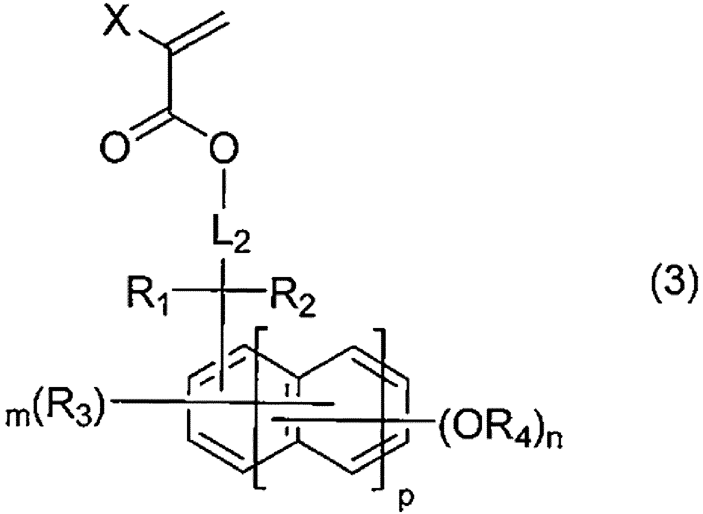

In Formula (3),
X represents a hydrogen atom or an organic group,
R₁ and R₂ have the same meanings as R₁ and R₂ in Formula (2), respectively,
L₂ represents a single bond or a divalent linking group, and
R₃, R₄, n, m, and p have the same meanings as R₃, R₄, n, m, and p in Formula (2), respectively,
in a case where m represents an integer of 2 or more, a plurality of R₃'s may be the same or different from each other, and in a case where n represents an integer of 2 or more, a plurality of R₄'s may be the same or different from each other.

X and L₂ have the same meanings as X and L₂ in Formula (3) of the above-described resin (A) contained in the composition according to the embodiment of the present invention, respectively.

The compound represented by Formula (2) can be synthesized by the following method.

As an example, M-70 which will be described later is used to description.

First, 4-tert-butoxybenzaldehyde is dissolved in tetrahydrofuran, and trifluoromethyltrimethylsilane and tetrabutylammonium are added thereto and stirred. Methacrylic anhydride is further added to the obtained solution, and the mixture is heated and stirred, purified by liquid separation, and concentrated to obtain an intermediate. After heating and stirring the intermediate with 4M hydrochloric acid water in an acetone solvent, liquid separation purification, concentration, crystallization, and drying are performed to synthesize M-70.

Compounds other than M-70 can also be synthesized in the same manner.

In addition, the present invention also relates to a resin having a repeating unit derived from the above-described compound represented by Formula (2) or a repeating unit derived from the above-described compound represented by Formula (3).

Examples of the resin include a resin which has, in the above-described resin (A) contained in the composition according to the embodiment of the present invention, a repeating unit derived from the above-described compound represented by Formula (2) or a repeating unit derived from the above-described compound represented by Formula (3).

### <(B) Compound which Generates Acid by Irradiation with Actinic Ray or Radiation>

The composition according to the embodiment of the present invention contains a compound which generates an acid by irradiation with actinic ray or radiation (also referred to as a photoacid generator or a photoacid generator (B)). The photoacid generator is a compound which generates an acid by exposure (preferably exposure to EUV light).

The photoacid generator may be in a form of a low-molecular-weight compound or a form incorporated into a part of a polymer. In addition, a combination of the form of a low-molecular-weight compound and the form incorporated into a part of a polymer may also be used.

In a case where the photoacid generator is in the form of a low-molecular-weight compound, a molecular weight is preferably 3000 or less, more preferably 2000 or less, and still more preferably 1000 or less.

In a case where the photoacid generator is in the form incorporated into a part of a polymer, it may be incorporated into the part of the resin (A) or into a resin which is different from the resin (A).

In the present invention, the photoacid generator is preferably in the form of a low-molecular-weight compound.

The photoacid generator is not particularly limited, but a compound which generates an organic acid by irradiation with EUV light is preferable, and a photoacid generator having a fluorine atom or an iodine atom in the molecule is more preferable.

Examples of the above-described organic acid include sulfonic acids (an aliphatic sulfonic acid, an aromatic sulfonic acid, a camphor sulfonic acid, and the like), carboxylic acids (an aliphatic carboxylic acid, an aromatic carboxylic acid, an aralkylcarboxylic acid, and the like), a carbonylsulfonylimide acid, a bis(alkylsulfonyl)imide acid, and a tris(alkylsulfonyl)methide acid.

In the present invention, a strength of the acid generated from the photoacid generator (B) is not particularly limited, but for example, a pKa of the generated acid is preferably -12.00 to 1.00, more preferably -7.00 to 0.50, and still more preferably -5.00 to 0.00.

A volume of the acid generated from the photoacid generator is not particularly limited, but from the viewpoint of suppressing diffusion of the acid generated by exposure into the non-exposed portion and improving resolution, the volume is preferably 240 Å³ or more, more preferably 305 Å³ or more, still more preferably 350 Å³ or more, and particularly preferably 400 Å³ or more.

From the viewpoint of sensitivity or solubility in an application solvent, the volume of the acid generated from the photoacid generator is preferably 1,500 Å³ or less, more preferably 1,000 Å³ or less, and still more preferably 700 Å³ or less.

The value of the above-described volume is obtained using "WinMOPAC" manufactured by Fujitsu Limited. For the computation of the value of the above-described volume, first, the chemical structure of an acid according to each example is input, next, using this structure as an initial structure, the most stable conformation of each acid is determined by molecular force field computation using a Molecular Mechanics (MM) 3 method, and thereafter with respect to the most stable conformation, molecular orbital calculation using a Parameterized Model number (PM) 3 method is performed, whereby the "accessible volume" of each acid can be computed.

A structure of the acid generated from the photoacid generator is not particularly limited, but from the viewpoint that the diffusion of the acid is suppressed and the resolution is improved, it is preferable that an interaction between the acid generated from the photoacid generator and the resin (A) is strong. From this viewpoint, in a case where the acid generated from the photoacid generator is an organic acid, it is preferable that a polar group is further included, in addition to an organic acid group such as a sulfonic acid group, a carboxylic acid group, a carbonylsulfonylimide acid group, a bissulfonylimide acid group, and a trissulfonylmethide acid group.

Examples of the polar group include an ether group, an ester group, an amide group, an acyl group, a sulfo group, a sulfonyloxy group, a sulfonamide group, a thioether group, a thioester group, a urea group, a carbonate group, a carbamate group, a hydroxyl group, and a mercapto group.

The number of polar groups included in the generated acid is not particularly limited, and is preferably 1 or more and more preferably 2 or more. However, from the viewpoint of suppressing excessive development, the number of polar groups is preferably less than 6 and more preferably less than 4.

The photoacid generator is preferably a photoacid generator which generates an acid as exemplified below. In some of the examples, the computed value of the volume is added (unit: Å³).

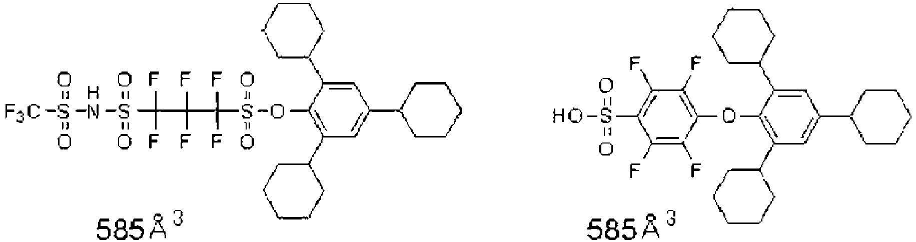

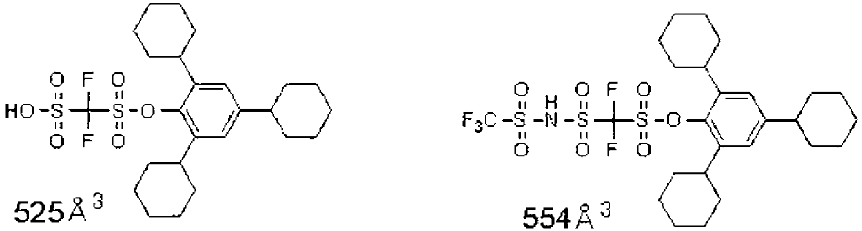

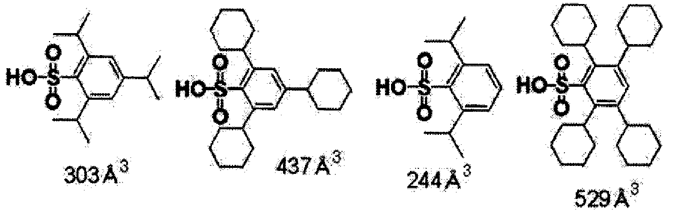

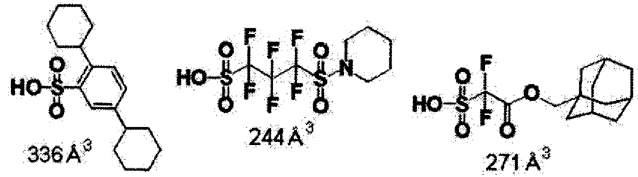

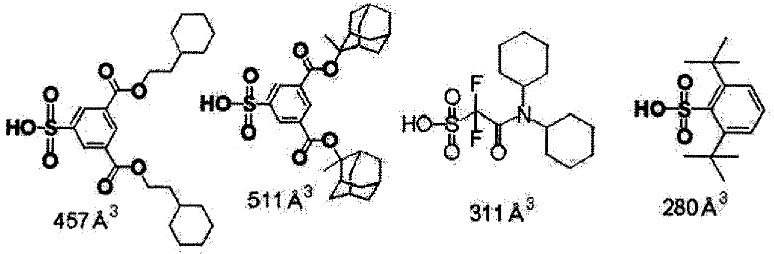

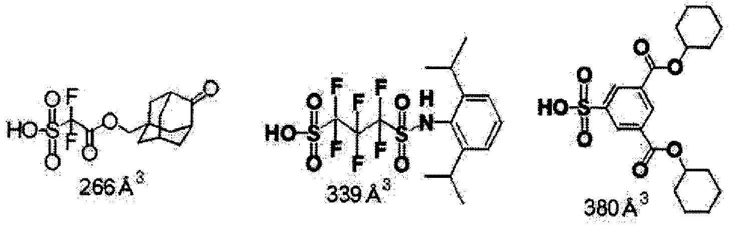

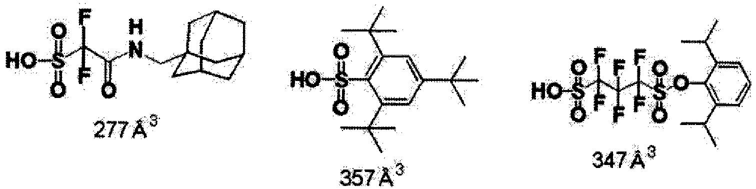

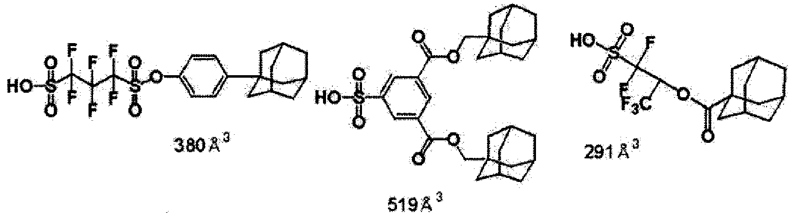

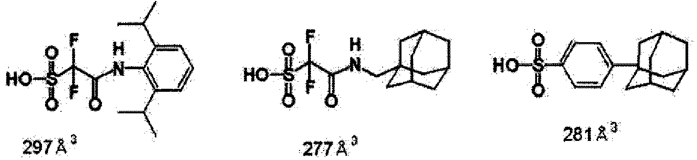

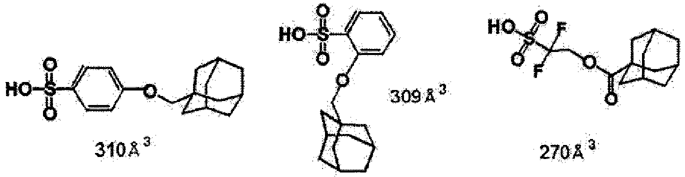

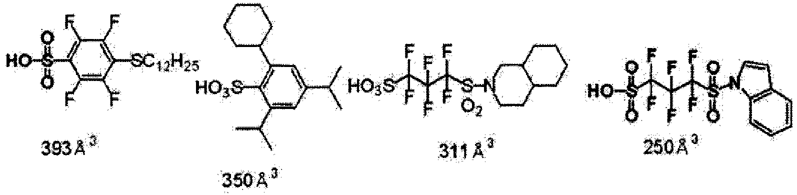

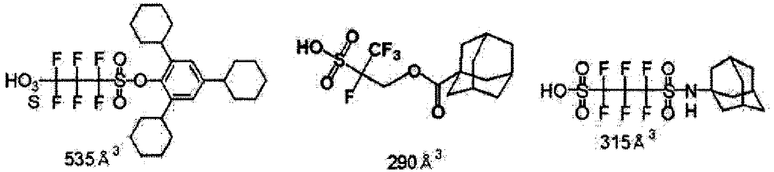

The photoacid generator is not particularly limited, and may be a photoacid generator having a non-anionic structure which does not have an anion or a cation or a photoacid generator having an anion or a cation, a so-called onium salt.

From the viewpoint that the effects of the present invention are more excellent, the photoacid generator is preferably an onium salt.

The photoacid generator preferably has a halogen atom in a cationic moiety.

Examples of the halogen atom include a fluorine atom, a chlorine atom, a bromine atom, and an iodine atom.

### (Compound Represented by General Formula (PA-1))

The photoacid generator preferably includes a compound represented by General Formula (PA-1).

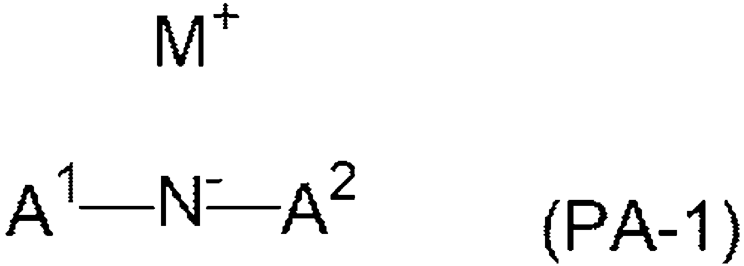

In General Formula (PA-1), A¹ and A² each independently represent -SO₂-R^{P} or -CO-R^{P}. R^{P} represents an organic group.

Two R^{P}'s present in General Formula (PA-1) may be the same or different from each other.

The number of carbon atoms in the two R^{P}'s present in General Formula (PA-1) is preferably 1 to 25 and more preferably 1 to 15, respectively.

The number of atoms in the two R^{P}'s present in General Formula (PA-1), in which hydrogen atoms are excluded, is preferably 2 to 30 and more preferably 4 to 20, respectively.

R^{P} is preferably a group represented by General Formula (RF).

-L^{RF}-R^{RF} (RF)

In General Formula (RF), L^{RF} represents a single bond or a divalent linking group.

Examples of the above-described divalent linking group include -COO-, -CONH-, -CO-, -O-, -S-, -SO-, -SO₂-, an alkylene group (may be linear or branched; preferably having 1 to 6 carbon atoms), a cycloalkylene group (preferably having 3 to 15 carbon atoms), an alkenylene group (may be linear or branched; preferably having 2 to 6 carbon atoms), and a divalent linking group formed by a combination of a plurality of these groups.

In addition, as a substituent which can be included in these divalent linking groups if possible, a halogen atom is preferable and a fluorine atom is more preferable. For example, it is also preferable that the above-described alkylene group (including an alkylene group which can be included in the divalent linking group in which a plurality of the groups are combined) is a perfluoroalkylene group.

The above-described divalent linking group is preferably -alkylene group-COO- or -alkylene group-SO₂-. It is preferable that the -alkylene group-COO- and -alkylene group-SO₂- have the alkylene group on the N- side.

In General Formula (RF), R^{RF} represents a cycloalkyl group or an alkyl group.

In a case where R^{RF} is a cycloalkyl group, the cycloalkyl group may be a monocycle or a polycycle.

The number of carbon atoms in the above-described cycloalkyl group is preferably 3 to 15 and more preferably 5 to 10.

Examples of the above-described cycloalkyl group include a norbornyl group, a decalinyl group, and an adamantyl group.

As a substituent which may be included in the above-described cycloalkyl group, an alkyl group (may be linear or branched; preferably having 1 to 5 carbon atoms) is preferable. It is also preferable that the above-described cycloalkyl group does not have any other substituent.

One or more of carbon atoms which are ring member atoms of the above-described cycloalkyl group may be replaced with a carbonyl carbon atom and/or heteroatom. For example, in the cycloalkyl group, a carbon atom (-CH<) bonded to L^{RF} may be replaced with a nitrogen atom (-N<).

In a case where R^{RF} is an alkyl group, the alkyl group may be linear or branched.

The number of carbon atoms in the above-described alkyl group is preferably 1 to 10 and more preferably 1 to 5.

As a substituent which may be included in the above-described alkyl group, a cycloalkyl group, a fluorine atom, or a cyano group is preferable. It is also preferable that the above-described alkyl group does not have any other substituent.

Examples of the cycloalkyl group as the substituent include those of the cycloalkyl group described in a case where R^{RF} is the cycloalkyl group.

In a case where the above-described alkyl group has a fluorine atom as the substituent, the above-described alkyl group may or may not be a perfluoroalkyl group. In a case where the above-described alkyl group has a fluorine atom as the substituent, it is also preferable that a part or all of the above-described alkyl groups are perfluoromethyl groups.

In General Formula (PA-1), the two R^{P}'s included in "A¹-N⁻-A²" may be bonded to each other to form a ring.

In General Formula (PA-1), M⁺ represents a cation.

The cation of M⁺ is preferably an organic cation.

The above-described organic cations are each independently preferably a cation (cation (ZaI)) represented by General Formula (ZaI) or a cation (cation (ZaII)) represented by General Formula (ZaII).

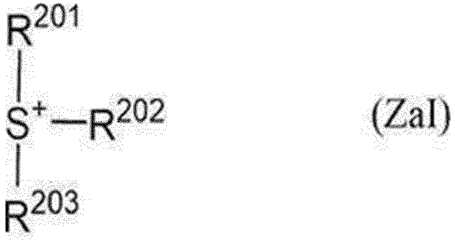

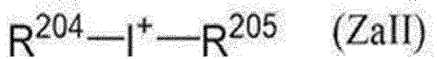

In General Formula (ZaI),
R²⁰¹, R²⁰², and R²⁰³ each independently represent an organic group.

The number of carbon atoms in the organic group of R²⁰¹, R²⁰², and R²⁰³ is usually 1 to 30, and preferably 1 to 20. In addition, two of R²⁰¹ to R²⁰³ may be bonded to each other to form a ring structure, and the ring structure may include an oxygen atom, a sulfur atom, an ester group, an amide group, or a carbonyl group in the ring. Examples of the group formed by the bonding of two of R²⁰¹ to R²⁰³ include an alkylene group (for example, a butylene group and a pentylene group) and -CH₂-CH₂-O-CH₂-CH₂-.

Examples of the cation in General Formula (ZaI) include a cation (ZaI-1), a cation (ZaI-2), a cation (cation (ZaI-3b)) represented by General Formula (ZaI-3b), and a cation (cation (ZaI-4b)) represented by General Formula (ZaI-4b), each of which will be described later.

First, the cation (ZaI-1) will be described.

The cation (ZaI-1) is an arylsulfonium cation in which at least one of R²⁰¹, R²⁰², or R²⁰³ of General Formula (ZaI) is an aryl group.

In the arylsulfonium cation, all of R²⁰¹ to R²⁰³ may be aryl groups, or some of R²⁰¹ to R²⁰³ may be an aryl group and the rest may be an alkyl group or a cycloalkyl group.

In addition, one of R²⁰¹ to R²⁰³ may be an aryl group, the remaining two of R²⁰¹ to R²⁰³ may be bonded to each other to form a ring structure, and an oxygen atom, a sulfur atom, an ester group, an amide group, or a carbonyl group may be included in the ring. Examples of the group formed by the bonding of two of R²⁰¹ to R²⁰³ include an alkylene group (for example, a butylene group, a pentylene group, or -CH₂-CH₂-O-CH₂-CH₂-) in which one or more methylene groups may be substituted with an oxygen atom, a sulfur atom, an ester group, an amide group, and/or a carbonyl group.

Examples of the arylsulfonium cation include a triarylsulfonium cation, a diarylalkylsulfonium cation, an aryldialkylsulfonium cation, a diarylcycloalkylsulfonium cation, and an aryldicycloalkylsulfonium cation.

The aryl group included in the arylsulfonium cation is preferably a phenyl group or a naphthyl group, and more preferably a phenyl group. The aryl group may be an aryl group which has a heterocyclic structure having an oxygen atom, a nitrogen atom, a sulfur atom, or the like. Examples of the heterocyclic structure include a pyrrole residue, a furan residue, a thiophene residue, an indole residue, a benzofuran residue, and a benzothiophene residue. In a case where the arylsulfonium cation has two or more aryl groups, the two or more aryl groups may be the same or different from each other.

The alkyl group or the cycloalkyl group included in the arylsulfonium cation as necessary is preferably a linear alkyl group having 1 to 15 carbon atoms, a branched alkyl group having 3 to 15 carbon atoms, or a cycloalkyl group having 3 to 15 carbon atoms, and examples thereof include a methyl group, an ethyl group, a propyl group, an n-butyl group, a sec-butyl group, a t-butyl group, a cyclopropyl group, a cyclobutyl group, and a cyclohexyl group.

The aryl group, alkyl group, and cycloalkyl group of R²⁰¹ to R²⁰³ may have a substituent.

The substituents which may be included in the aryl group, the alkyl group, and the cycloalkyl group of R²⁰¹ to R²⁰¹ are not particularly limited, but each independently preferably an alkyl group (for example, having 1 to 15 carbon atoms), a cycloalkyl group (for example, having 3 to 15 carbon atoms), an aryl group (for example, having 6 to 14 carbon atoms), an alkoxy group (for example, having 1 to 15 carbon atoms), a cycloalkylalkoxy group (for example, having 1 to 15 carbon atoms), a halogen atom, a hydroxyl group, or a phenylthio group.

The substituent may further have a substituent if possible, and may be in a form of an alkyl halide group such as a trifluoromethyl group, for example, in which an alkyl group has a halogen atom as a substituent.

Next, the cation (ZaI-2) will be described.

The cation (ZaI-2) is a cation in which R²⁰¹ to R²⁰³ in Formula (ZaI) are each independently a cation representing an organic group having no aromatic ring. Here, the aromatic ring also encompasses an aromatic ring including a heteroatom.

The number of carbon atoms in the organic group as R²⁰¹ to R²⁰³, which has no aromatic ring, is generally 1 to 30, and preferably 1 to 20.

R²⁰¹ to R²⁰¹ are each independently preferably an alkyl group, a cycloalkyl group, an allyl group, or a vinyl group, more preferably a linear or branched 2-oxoalkyl group, a 2-oxocycloalkyl group, or an alkoxycarbonylmethyl group, and still more preferably a linear or branched 2-oxoalkyl group.

Examples of the alkyl group and cycloalkyl group of R²⁰¹ to R²⁰¹ include a linear alkyl group having 1 to 10 carbon atoms or branched alkyl group having 3 to 10 carbon atoms (for example, a methyl group, an ethyl group, a propyl group, a butyl group, and a pentyl group), and a cycloalkyl group having 3 to 10 carbon atoms (for example, a cyclopentyl group, a cyclohexyl group, and a norbornyl group).

R²⁰¹ to R²⁰³ may further be substituted with a halogen atom, an alkoxy group (for example, having 1 to 5 carbon atoms), a hydroxyl group, a cyano group, or a nitro group.

Next, the cation (ZaI-3b) will be described.

The cation (ZaI-3b) is a cation represented by General Formula (ZaI - 3b).

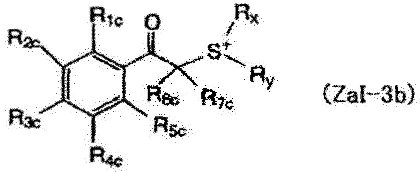

In General Formula (ZaI-3b),
R_{1c} to R_{5c} each independently represent a hydrogen atom, an alkyl group, a cycloalkyl group, an aryl group, an alkoxy group, an aryloxy group, an alkoxycarbonyl group, an alkylcarbonyloxy group, a cycloalkylcarbonyloxy group, a halogen atom, a hydroxyl group, a nitro group, an alkylthio group, or an arylthio group.
R_{6c} and R_{7c} each independently represent a hydrogen atom, an alkyl group (a t-butyl group and the like), a cycloalkyl group, a halogen atom, a cyano group, or an aryl group.
Rₓ and R_{y} each independently represent an alkyl group, a cycloalkyl group, a 2-oxoalkyl group, a 2-oxocycloalkyl group, an alkoxycarbonylalkyl group, an allyl group, or a vinyl group.

Any two or more of R_{1c}, ... , or R_{5c}, R_{5c} and R_{6c}, R_{6c} and R_{7c}, R_{5c} and Rₓ, and Rₓ and R_{y} may each be bonded to each other to form a ring, and the rings may each independently include an oxygen atom, a sulfur atom, a ketone group, an ester bond, or an amide bond.

Examples of the above-described ring include an aromatic or non-aromatic hydrocarbon ring, an aromatic or non-aromatic heterocyclic ring, and a polycyclic fused ring formed by a combination of two or more of these rings. Examples of the ring include a 3- to 10-membered ring, and the ring is preferably a 4- to 8-membered ring and more preferably a 5- or 6-membered ring.

Examples of the group formed by the bonding of any two or more of R_{1c}, ... , or R_{5c}, R_{6c} and R_{7c}, and Rₓ and R_{y} include an alkylene group such as a butylene group and a pentylene group. A methylene group in this alkylene group may be substituted with a heteroatom such as an oxygen atom.

As the group formed by the bonding of R_{5c} and R_{6c}, and R_{5c} and Rₓ, a single bond or an alkylene group is preferable. Examples of the alkylene group include a methylene group and an ethylene group.

Next, the cation (ZaI-4b) will be described. The cation (ZaI-4b) is a cation represented by General Formula (ZaI-4b).

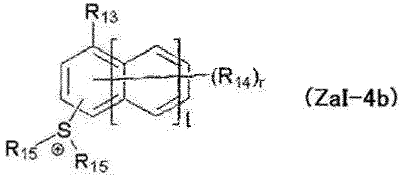

In General Formula (ZaI-4b),
1 represents an integer of 0 to 2.
r represents an integer of 0 to 8.
R₁₃ represents a hydrogen atom, a fluorine atom, a hydroxyl group, an alkyl group, an alkoxy group, an alkoxycarbonyl group, or a group having a cycloalkyl group (which may be the cycloalkyl group itself or a group including the cycloalkyl group in a part thereof). These groups may have a substituent.
R₁₄ represents a hydroxyl group, an alkyl group, an alkoxy group, an alkoxycarbonyl group, an alkylcarbonyl group, an alkylsulfonyl group, a cycloalkylsulfonyl group, or a group having a cycloalkyl group (which may be the cycloalkyl group itself or a group including the cycloalkyl group in a part thereof). These groups may have a substituent. In a case where R₁₄'s are present in a plural number, R₁₄'s each independently represent the above-described group such as a hydroxyl group.
R₁₅'s each independently represent an alkyl group, a cycloalkyl group, or a naphthyl group. These groups may have a substituent. Two R₁₅'s may be bonded to each other to form a ring. In a case where two R₁₅'s are bonded to each other to form a ring, a ring skeleton may include a heteroatom such as an oxygen atom and a nitrogen atom. In one aspect, it is preferable that two R₁₅'s are alkylene groups and are bonded to each other to form a ring structure.

In General Formula (ZaI-4b), the alkyl group of R₁₃, R₁₄, and R₁₅ is linear or branched. The number of carbon atoms in the alkyl group is preferably 1 to 10. The alkyl group is more preferably a methyl group, an ethyl group, an n-butyl group, a t-butyl group, or the like.

Next, General Formula (ZaII) will be described. In General Formula (ZaII), R²⁰⁴ and R²⁰⁵ each independently represent an aryl group, an alkyl group, or a cycloalkyl group.

The aryl group of R²⁰⁴ and R²⁰⁵ is preferably a phenyl group or a naphthyl group, and more preferably a phenyl group. The aryl group of R²⁰⁴ and R²⁰⁵ may be an aryl group which has a heterocyclic ring having an oxygen atom, a nitrogen atom, a sulfur atom, or the like. Examples of a skeleton of the aryl group having a heterocyclic ring include pyrrole, furan, thiophene, indole, benzofuran, and benzothiophene.

The alkyl group and cycloalkyl group of R²⁰⁴ and R²⁰⁵ are preferably a linear alkyl group having 1 to 10 carbon atoms or a branched alkyl group having 3 to 10 carbon atoms (for example, a methyl group, an ethyl group, a propyl group, a butyl group, and a pentyl group), or a cycloalkyl group having 3 to 10 carbon atoms (for example, a cyclopentyl group, a cyclohexyl group, and a norbornyl group).

The aryl group, the alkyl group, and the cycloalkyl group of R²⁰⁴ and R²⁰⁵ may each independently have a substituent. Examples of the substituent which may be included in each of the aryl group, the alkyl group, and the cycloalkyl group of R²⁰⁴ and R²⁰⁵ include an alkyl group (for example, having 1 to 15 carbon atoms), a cycloalkyl group (for example, having 3 to 15 carbon atoms), an aryl group (for example, having 6 to 15 carbon atoms), an alkoxy group (for example, having 1 to 15 carbon atoms), a halogen atom, a hydroxyl group, and a phenylthio group.

### (Other Photoacid Generators)

In the composition according to the embodiment of the present invention, a photoacid generator other than those described above may be used.

Examples of other photoacid generators include a compound (onium salt) represented by "M⁺Z⁻ (M⁺ represents a cation and Z⁻ represents an anion)".

In the compound represented by "M⁺ Z⁻", M⁺ represents a cation, and examples thereof include the same cation as the cation in General Formula (PA-1).

In the compound represented by "M⁺ Z⁻", Z⁻ represents an anion, and an anion having a significantly low ability to case a nucleophilic reaction is preferable.

Examples of the above-described anion include a sulfonate anion (an aliphatic sulfonate anion such as a fluoroalkyl sulfonate anion, an aromatic sulfonate anion, a camphor sulfonate anion, and the like), a carboxylate anion (an aliphatic carboxylate anion, an aromatic carboxylate anion, an aralkyl carboxylate anion, and the like), and a tris(alkylsulfonyl)methide anion.

An aliphatic moiety in the aliphatic sulfonate anion and the aliphatic carboxylate anion may be an alkyl group or a cycloalkyl group, and a linear or branched alkyl group having 1 to 30 carbon atoms or a cycloalkyl group having 3 to 30 carbon atoms is preferable.

An aromatic ring group in the aromatic sulfonate anion and the aromatic carboxylate anion is preferably an aryl group having 6 to 14 carbon atoms, and examples thereof include a phenyl group, a tolyl group, and a naphthyl group.

Examples of the substituent which can be included in the alkyl group, cycloalkyl group, and aryl group mentioned above include a nitro group, a halogen atom such as a fluorine atom, a carboxylic acid group, a hydroxyl group, an amino group, a cyano group, an alkoxy group (preferably having 1 to 15 carbon atoms), a cycloalkyl group (preferably having 3 to 15 carbon atoms), an aryl group (preferably having 6 to 14 carbon atoms), an alkoxycarbonyl group (preferably having 2 to 7 carbon atoms), an acyl group (preferably having 2 to 12 carbon atoms), an alkoxycarbonyloxy group (preferably having 2 to 7 carbon atoms), an alkylthio group (preferably having 1 to 15 carbon atoms), an alkylsulfonyl group (preferably having 1 to 15 carbon atoms), an alkyliminosulfonyl group (preferably having 1 to 15 carbon atoms), an aryloxysulfonyl group (preferably having 6 to 20 carbon atoms), an alkylaryloxysulfonyl group (preferably having 7 to 20 carbon atoms), a cycloalkylaryloxysulfonyl group (preferably having 10 to 20 carbon atoms), an alkyloxyalkyloxy group (preferably having 5 to 20 carbon atoms), and a cycloalkylalkyloxyalkyloxy group (preferably having 8 to 20 carbon atoms).

An aralkyl group in the aralkyl carboxylate anion is preferably an aralkyl group having 7 to 12 carbon atoms, and examples thereof include a benzyl group, a phenethyl group, a naphthylmethyl group, a naphthylethyl group, and a naphthylbutyl group.

An alkyl group in the tris(alkylsulfonyl)methide anion is preferably an alkyl group having 1 to 5 carbon atoms. Examples of a substituent of these alkyl group include a halogen atom, an alkyl group substituted with a halogen atom, an alkoxy group, an alkylthio group, an alkyloxysulfonyl group, an aryloxysulfonyl group, and a cycloalkylaryloxysulfonyl group, and a fluorine atom or an alkyl group substituted with a fluorine atom is preferable.

A plurality of sulfonate anions may be bonded to each other through a linking group. A plurality of sulfonate anions may be bonded to each other through a linking group. In addition, a plurality of tris(alkylsulfonyl)methide anions may be bonded to each other through a linking group.

Examples of other non-nucleophilic anions include fluorinated phosphorus (for example, PF₆⁻), fluorinated boron (for example, BF₄⁻), and fluorinated antimony (for example, SbF₆⁻).

As the non-nucleophilic anion, an aliphatic sulfonate anion in which at least α-position of sulfonic acid is substituted with a fluorine atom, an aromatic sulfonate anion substituted with a fluorine atom or a group having a fluorine atom, or a tris(alkylsulfonyl)methide anion in which an alkyl group is substituted with a fluorine atom is preferable. Among these, a perfluoroaliphatic sulfonate anion (preferably having 4 to 8 carbon atoms) or benzenesulfonate anion having a fluorine atom is more preferable, and a nonafluorobutanesulfonate anion, a perfluorooctanesulfonate anion, a pentafluorobenzenesulfonate anion, or a 3,5-bis(trifluoromethyl)benzenesulfonate anion is still more preferable.

From the viewpoint of acid strength, it is preferable that the pKa of the generated acid is -1 or less to improve the sensitivity.

In addition, as the non-nucleophilic anion, an anion represented by General Formula (AN1) is also preferable.

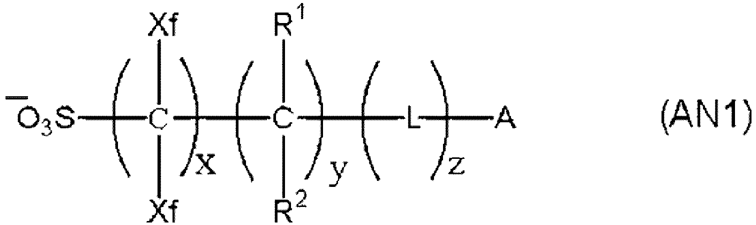

In the formula, Xf's each independently represent a fluorine atom or an alkyl group substituted with at least one fluorine atom.

R¹ and R² each independently represent a hydrogen atom, a fluorine atom, or an alkyl group. In a case where a plurality of R¹'s and R²'s are present, R¹'s and R²'s may each be the same or different from each other.

L represents a divalent linking group, and in a case where a plurality of L's are present, L's may be the same or different from each other.

A represents a cyclic organic group.

x represents an integer of 1 to 20, y represents an integer of 0 to 10, and z represents an integer of 0 to 10.

General Formula (AN1) will be described in more detail.

The number of carbon atoms in the alkyl group as the alkyl group substituted with a fluorine atom of Xf is preferably 1 to 10 and more preferably 1 to 4. In addition, the alkyl group as the alkyl group substituted with a fluorine atom of Xf is preferably a perfluoroalkyl group.

Xf is preferably a fluorine atom or a perfluoroalkyl group having 1 to 4 carbon atoms. Examples of Xf include a fluorine atom, CF₃, C₂F₅, C₃F₇, C₄F₉, CH₂CF₃, CH₂CH₂CF₃, CH₂C₂F₅, CH₂CH₂C₂F₅, CH₂C₃F₇, CH₂CH₂C₃F₇, CH₂C₄F₉, and CH₂CH₂C₄F₉. Among these, a fluorine atom or CF₃ is preferable. In particular, it is preferable that both Xf's are fluorine atoms.

The alkyl group of R¹ and R² may have a substituent (preferably a fluorine atom), and the number of carbon atoms in the substituent is preferably 1 to 4. The substituent is preferably a perfluoroalkyl group having 1 to 4 carbon atoms. Examples of the alkyl group of R¹ and R², which has the substituent, include CF₃, C₂F₅, C₃F₇, C₄F₉, C₅F₁₁, C₆F₁₃, C₇F₁₅, C₈F₁₇, CH₂CF₃, CH₂CH₂CF₃, CH₂C₂F₅, CH₂CH₂C₂F₅, CH₂C₃F₇, CH₂CH₂C₃F₇, CH₂C₄F₉, and CH₂CH₂C₄F₉. Among these, CF₃ is preferable.

R¹ and R² are preferably a fluorine atom or CF₃.
x is preferably an integer of 1 to 10 and more preferably 1 to 5.
y is preferably an integer of 0 to 4 and more preferably 0.
z is preferably an integer of 0 to 5 and more preferably an integer of 0 to 3.

Examples of the divalent linking group of L include -COO-, -CO-, -O-, -S-, -SO-, -SO₂-, an alkylene group, a cycloalkylene group, an alkenylene group, a linking group consisting of a plurality of these groups linked to each other, and the like. The total number of carbon atoms in these linking groups is preferably 12 or less. Among these, -COO-, -CO-, or -O- is preferable, and -COO- is more preferable.

The cyclic organic group of A is not particularly limited as long as it has a cyclic structure, and examples thereof include an alicyclic group, an aromatic ring group, and a heterocyclic group (including not only an aromatic heterocyclic group but also a non-aromatic heterocyclic group).

The alicyclic group may be a monocycle or a polycycle, and is preferably a monocyclic cycloalkyl group such as a cyclopentyl group, a cyclohexyl group, and a cyclooctyl group, or a polycyclic cycloalkyl group such as a norbornyl group, a tricyclodecanyl group, a tetracyclodecanyl group, a tetracyclododecanyl group, and an adamantyl group. Among these, from the viewpoint that diffusivity in the film in a post-exposure heating step can be suppressed and mask error enhancement factor (MEEF) is improved, an alicyclic group having a bulky structure and having 7 or more carbon atoms, such as a norbornyl group, a tricyclodecanyl group, a tetracyclodecanyl group, a tetracyclododecanyl group, and an adamantyl group is preferable.

Examples of the aromatic ring group include a benzene ring, a naphthalene ring, a phenanthrene ring, and an anthracene ring.

Examples of the heterocyclic group include groups derived from a furan ring, a thiophene ring, a benzofuran ring, a benzothiophene ring, a dibenzofuran ring, a dibenzothiophene ring, or a pyridine ring. Among these, groups derived from a furan ring, a thiophene ring, or a pyridine ring are preferable.

In addition, examples of the cyclic organic group also include a lactone structure, and specific examples thereof include the lactone structures represented by General Formulae (LC1-1) to (LC1-22) described above.

The above-described cyclic organic group may have a substituent. Examples of the above-described substituent include an alkyl group (may be linear or branched, or may include a cyclic structure; preferably having 1 to 12 carbon atoms), a cycloalkyl group (may be either a monocycle or a polycycle; in a case where the cycloalkyl group is a polycycle, the cycloalkyl group may be a spiro ring; preferably having 3 to 20 carbon atoms), an aryl group (preferably having 6 to 14 carbon atoms), a hydroxyl group, an alkoxy group, an ester group, an amide group, a urethane group, a ureido group, a thioether group, a sulfonamide group, and a sulfonic acid ester group. A carbon constituting the cyclic organic group (carbon contributing to ring formation) may be a carbonyl carbon.

In addition, the photoacid generator may be a betaine compound having a structure that has a cationic moiety and an anionic moiety and both are linked by a covalent bond.

With regard to the photoacid generator, reference can be made to paragraphs [0368] to [0377] of JP2014-41328A and paragraphs [0240] to [0262] of JP2013-228681A (corresponding to paragraph [0339] of US2015/004533A), the contents of which are incorporated herein by reference.

In addition, specific preferred examples thereof include the following compounds. In the following compounds, the anion and the cation can be optionally exchanged.

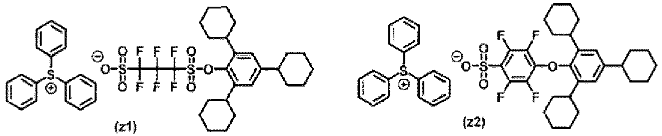

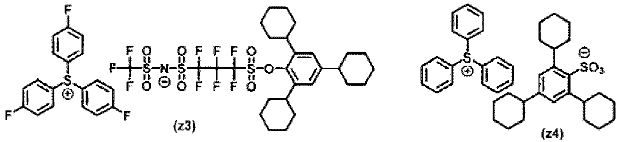

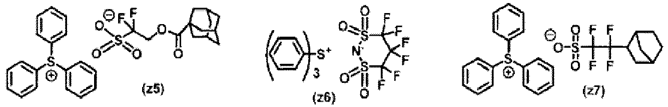

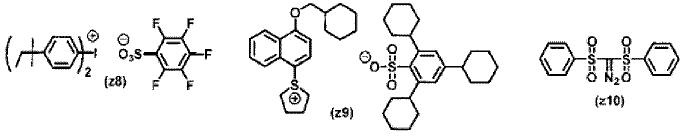

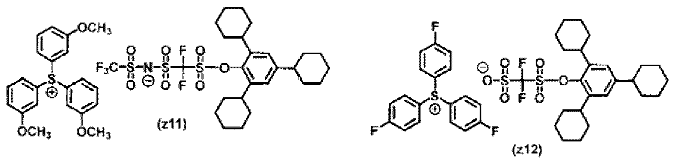

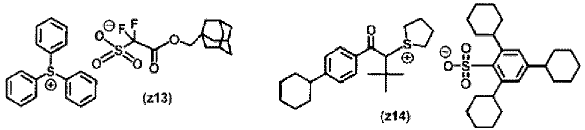

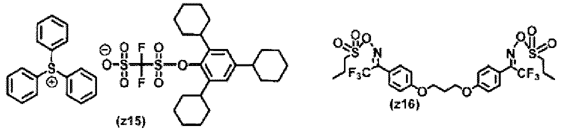

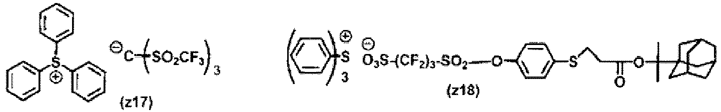

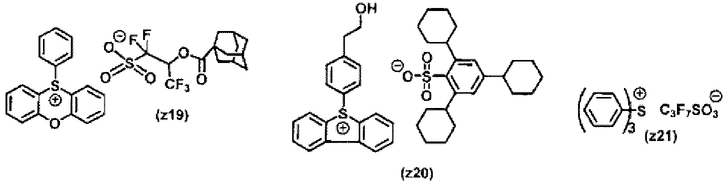

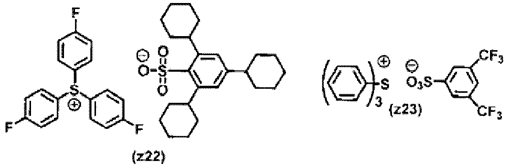

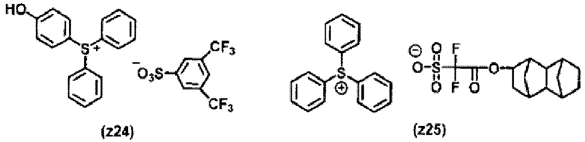

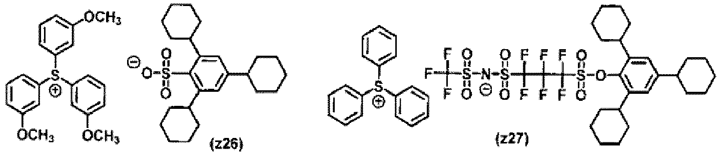

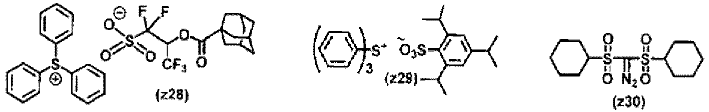

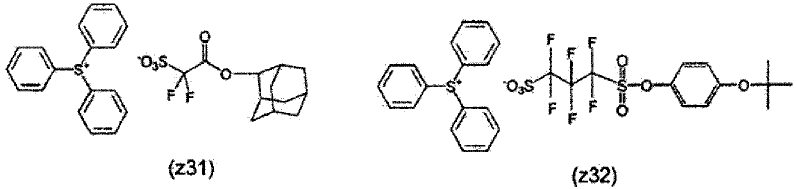

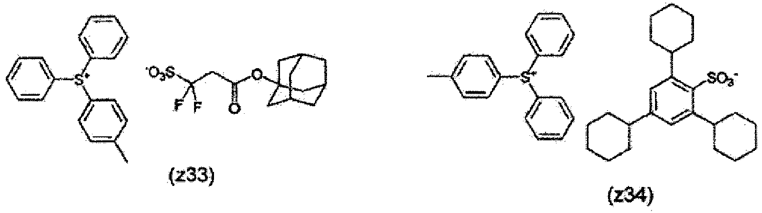

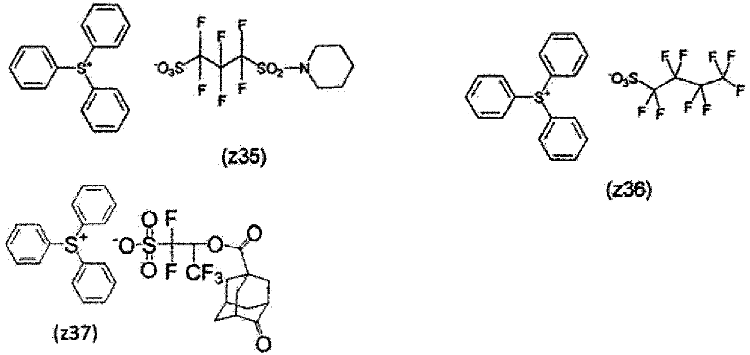

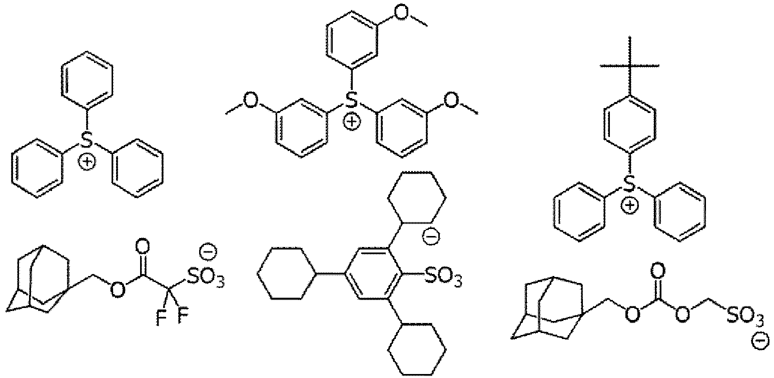

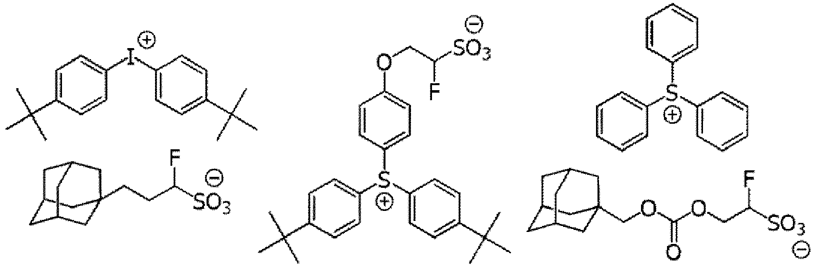

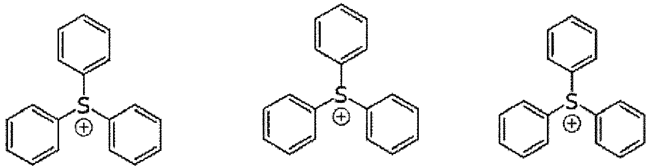

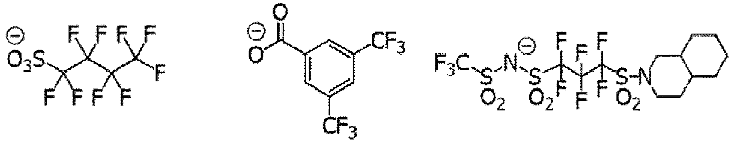

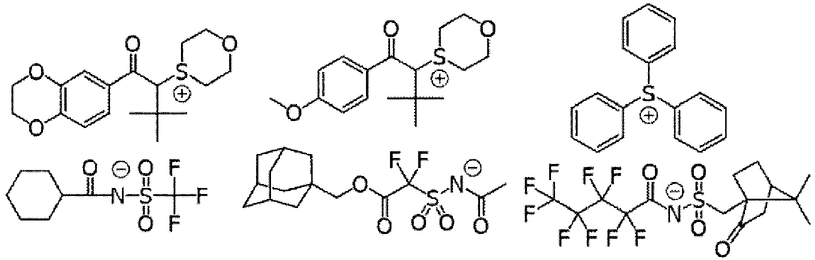

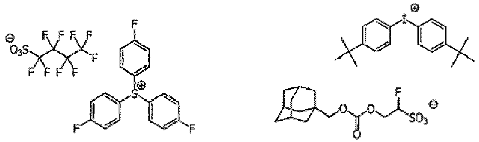

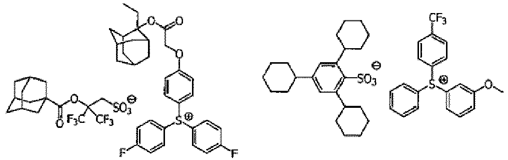

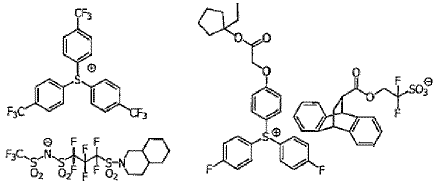

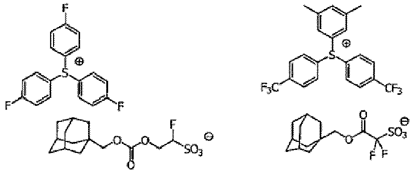

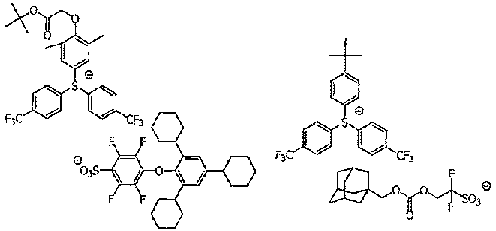

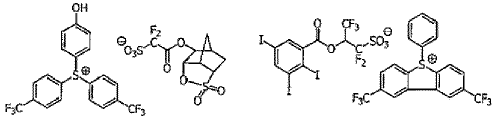

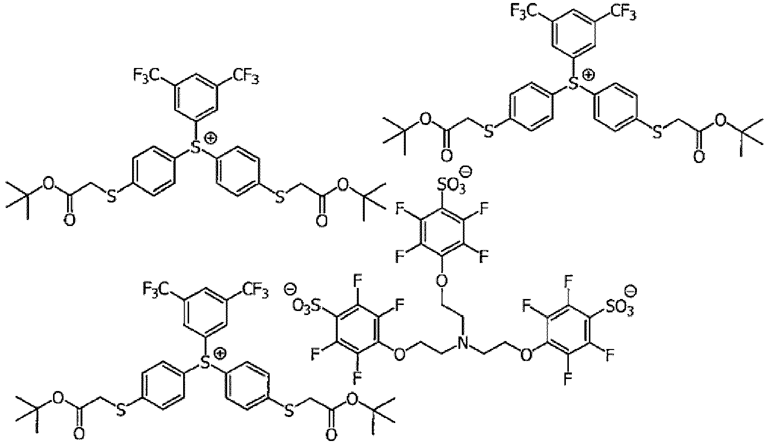

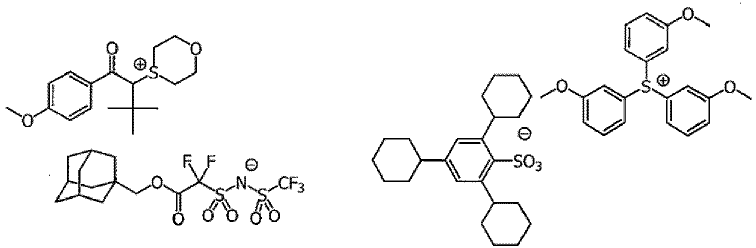

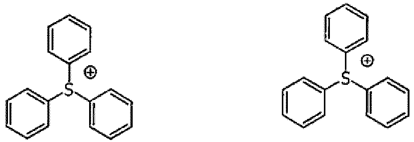

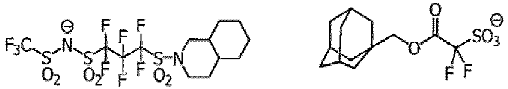

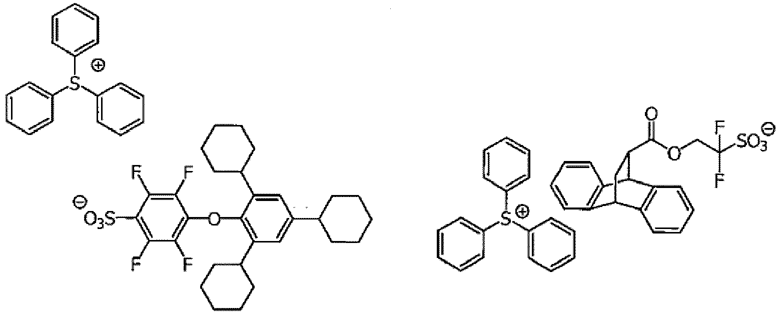

A content of the photoacid generator in the composition according to the embodiment of the present invention is not particularly limited, but from the viewpoint that the effects of the present invention are more excellent, the content is preferably 5.0% by mass or more, more preferably 9.0% by mass or more, and still more preferably 15.0% by mass or more with respect to the total solid content of the composition. In addition, the above-described content is preferably 90.0% by mass or less, more preferably 80.0% by mass or less, and still more preferably 70.0% by mass or less.

The photoacid generators may be used alone or in combination of two or more kinds thereof.

### <(C) Compound which is Decomposed by Irradiation with Actinic Ray or Radiation to Reduce Acid-trapping Property>

The composition according to the embodiment of the present invention may contain, as the acid diffusion control agent, (C) a compound which is decomposed by irradiation with actinic ray or radiation to reduce acid-trapping property (also referred to as a compound (C), a photodegradable quencher, or a photodegradable quencher (C)).

The compound (C) acts as a quencher which suppresses a reaction of the resin (A) in the non-exposed portion due to excessively generated acids by trapping the acids generated from the photoacid generator (B) and the like upon exposure.

The compound (C) is a compound that generates an acid which is relatively weak with respect to the acid generated from the photoacid generator (B). That is, the compound (C) is a compound which generates an acid having a larger pKa than the acid generated from the photoacid generator (B).

A difference between the pKa of the acid generated from the compound (C) and the pKa of the acid generated from the photoacid generator (B) (value obtained by subtracting the pKa of the acid generated from the compound (C) from the pKa of the acid generated from the photoacid generator (B)) is preferably 1.00 or more, more preferably 1.00 to 10.00, still more preferably 1.00 to 5.00, and even more preferably 1.00 to 3.00.

In addition, the pKa of the acid generated from the compound (C) varies depending on the type of the photoacid generator (B) used, but for example, is preferably -4.00 to 14.00, more preferably -2.00 to 12.00, and still more preferably -1.00 to 5.00.

The compound (C) may be a non-ionic compound or an ionic compound.

As a preferred aspect, the ionic compound (C) contains a halogen atom in the anionic moiety. Examples of the halogen atom include a fluorine atom, a chlorine atom, a bromine atom, and an iodine atom. Among these, a fluorine atom is preferable.

As a preferred aspect of the compound (C), among the compounds exemplified as the above-described photoacid generator (B) (preferably, the above-described compound represented by General Formula (PA-1) and the onium salt represented by M⁺Z⁻), a compound that generates an acid which is relatively weak relative to the acid generated from the photoacid generator (B) used can be selected and used.

It is also preferable that the ionic compound as the compound (C) contains a halogen atom in a cationic moiety.

The ionic compound as the compound (C) is preferably a compound represented by General Formulae (C1) to (C3).

### (Compound Represented by General Formula (C1))

R^{C1}-L^{C1}-SO₃⁻ M_{C}⁺ (C1)

In General Formula (C1),
R^{C1} represents a cycloalkyl group or an aryl group,
E^{C1} represents a single bond, an alkylene group, a cycloalkylene group, -O-, -C(=O)-, or a divalent linking group of a combination of these groups, and
M_{C}⁺ represents an organic cation.

In General Formula (C1),
R^{C1} represents a cycloalkyl group or an aryl group.

As the cycloalkyl group in a case where R^{C1} represents a cycloalkyl group, a cycloalkyl group having 3 to 15 carbon atoms is preferable, and a cycloalkyl group having 5 to 10 carbon atoms is more preferable. The cycloalkyl group may be a monocycle or a polycycle.

Examples of the cycloalkyl group include a norbornyl group, a decalinyl group, and an adamantyl group, and an adamantyl group is preferable.

As the aryl group in a case where R^{C1} represents an aryl group, an aryl group having 6 to 15 carbon atoms is preferable.

Examples of the aryl group include a phenyl group and a naphthyl group, and a phenyl group is preferable.

R^{C1} may have a substituent.

As a preferred aspect, in a case where R^{C1} has a substituent, examples of the substituent include a fluorine atom, a group having a fluorine atom, a hydroxyl group, an alkyl group, and a halogen atom other than the fluorine atom.

As the group having a fluorine atom, a fluorine-substituted alkyl group or a fluorine-substituted cycloalkyl group is preferable.

As the fluorine-substituted alkyl group, a fluoroalkyl group having 1 to 5 carbon atoms is preferable. Specific examples thereof include a trifluoromethyl group, a pentafluoroethyl group, and a nonafluorobutyl group, and a trifluoromethyl group is preferable.

As the fluorine-substituted cycloalkyl group, a fluorocycloalkyl group having 3 to 15 carbon atoms is preferable. Specific examples thereof include a fluorocyclohexyl group, a fluorocyclopentyl group, and a fluoroadamantyl group, and a fluorocyclohexyl group is preferable.

Examples of the alkyl group include a chain or branched alkyl group having 1 to 5 carbon atoms.

Examples of the halogen atom other than the fluorine atom include a chlorine atom, a bromine atom, and an iodine atom.

As a preferred aspect, at least one of R^{C1} or L^{C1} is substituted with a fluorine atom or a group having a fluorine atom.

As a preferred aspect, R^{C1} is preferably a polycyclic cycloalkyl group or an aryl group, which is substituted with a fluorine atom or a group having a fluorine atom.

In General Formula (C1),
L^{C1} represents a single bond, an alkylene group, a cycloalkylene group, -O-, -C(=O)-, or a divalent linking group of a combination of these groups.

Examples of the alkylene group in a case where L^{C1} represents an alkylene group include a chain or branched alkylene group having 1 to 20 carbon atoms.

Examples of the chain or branched alkylene group having 1 to 20 carbon atoms include a methylene group, an ethylene group, an n-propylene group, an i-propylene group, an n-butylene group, and an n-pentylene group, and a methylene group, an ethylene group, an n-propylene group, or an n-butylene group is preferable.

Examples of the cycloalkylene group in a case where L^{C1} represents a cycloalkylene group include a monocyclic or polycyclic cycloalkylene group having 3 to 20 carbon atoms.

Examples of the monocyclic or polycyclic cycloalkylene group having 3 to 20 carbon atoms include an adamantylene group, a cyclohexylene group, a cyclopentylene group, a cycloheptylene group, and a norbonylene group, and an adamantylene group, a cyclohexylene group, or a norbonylene group is preferable.

L^{C1} may have a substituent.

As a preferred aspect, examples of the substituent in a case where L^{C1} has a substituent include the above-described substituent in the case where R^{C1} has a substituent.

L^{C1} is preferably a single bond or an alkylene group, and more preferably a single bond or an n-butylene group.

In General Formula (C1),
M_{C}⁺ represents an organic cation.

M_{C}⁺ is preferably a cation represented by General Formula (ZcI) or General Formula (ZcII).

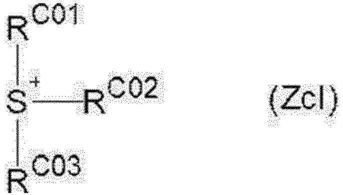

In General Formula (ZcI),
R^{C01}, R^{C02}, and R^{C03} each independently represent an organic group.

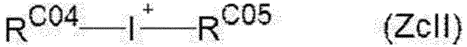

In General Formula (ZcII),
R^{C04} and R^{C05} each independently represent an aryl group, an alkyl group, or a cycloalkyl group.

In General Formula (ZcI),
R^{C01}, R^{C02}, and R^{C03} each independently represent an organic group.

The number of carbon atoms in the organic group of R^{C01}, R^{C02}, and R^{C03} is usually 1 to 30, and preferably 1 to 20. In addition, two of R^{C01} to R^{C03} may be bonded to each other to form a ring structure, and the ring structure may include an oxygen atom, a sulfur atom, an ester group, an amide group, or a carbonyl group in the ring. Examples of the group formed by the bonding of two of R^{C01} to R^{C03} include an alkylene group (for example, a butylene group and a pentylene group) and -CH₂-CH₂-O-CH₂-CH₂-.

The cation in General Formula (ZcI) is not particularly limited, and examples thereof include the cation (ZaI-1), the cation (ZaI-2), the cation (cation (ZaI-3b)) represented by General Formula (ZaI-3b), and the cation (cation (ZaI-4b)) represented by General Formula (ZaI-4b), each of which is described in the above photoacid generator (B).

In General Formula (ZcII),
R^{C04} and R^{C05} each independently represent an aryl group, an alkyl group, or a cycloalkyl group.

The aryl group of R^{C04} and R^{C05} is preferably a phenyl group or a naphthyl group, and more preferably a phenyl group. The aryl group of R^{C04} and R^{C05} may be an aryl group which has a heterocyclic ring having an oxygen atom, a nitrogen atom, a sulfur atom, or the like. Examples of a skeleton of the aryl group having a heterocyclic ring include pyrrole, furan, thiophene, indole, benzofuran, and benzothiophene.

The alkyl group and cycloalkyl group of R^{C04} and R^{C05} are preferably a linear alkyl group having 1 to 10 carbon atoms or a branched alkyl group having 3 to 10 carbon atoms (for example, a methyl group, an ethyl group, a propyl group, a butyl group, and a pentyl group), or a cycloalkyl group having 3 to 10 carbon atoms (for example, a cyclopentyl group, a cyclohexyl group, and a norbornyl group).

The aryl group, the alkyl group, and the cycloalkyl group of R^{C04} and R^{C05} may each independently have a substituent. Examples of the substituent which may be included in each of the aryl group, the alkyl group, and the cycloalkyl group of R^{C04} and R^{C05} include an alkyl group (for example, having 1 to 15 carbon atoms), a cycloalkyl group (for example, having 3 to 15 carbon atoms), an aryl group (for example, having 6 to 15 carbon atoms), an alkoxy group (for example, having 1 to 15 carbon atoms), a halogen atom, a hydroxyl group, and a phenylthio group.

### (Compound Represented by General Formula (C2))

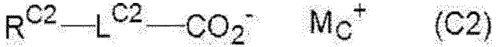

In General Formula (C2),
R^{C2} represents a cycloalkyl group or an aryl group.
L^{C2} represents a single bond, an alkylene group, a cycloalkylene group, -O-, -C(=O)-, or a divalent linking group of a combination of these groups.
M_{C}⁺ represents an organic cation.

In General Formula (C2),
R^{C2} represents a cycloalkyl group or an aryl group.

As the cycloalkyl group in a case where R^{C2} represents a cycloalkyl group, a cycloalkyl group having 3 to 15 carbon atoms is preferable, and a cycloalkyl group having 5 to 10 carbon atoms is more preferable. The cycloalkyl group may be a monocycle or a polycycle.

Examples of the cycloalkyl group include a norbornyl group, a decalinyl group, and an adamantyl group, and an adamantyl group is preferable.

As the aryl group in a case where R^{C2} represents an aryl group, an aryl group having 6 to 15 carbon atoms is preferable.

Examples of the aryl group include a phenyl group and a naphthyl group, and a phenyl group is preferable.

R^{C2} may have a substituent.

As a preferred aspect, examples of the substituent in a case where R^{C2} has a substituent include the substituent as the above-described preferred aspect in the case where R^{C1} in General Formula (C1) has a substituent.

R^{C2} is preferably a polycyclic cycloalkyl group, a polycyclic cycloalkyl group substituted with a fluorine atom or a group having a fluorine atom, or an aryl group substituted with a fluorine atom or a group having a fluorine atom, and more preferably an adamantyl group or a phenyl group substituted with a fluorine atom or a group having a fluorine atom.

In General Formula (C2),
L^{C2} represents a single bond, an alkylene group, a cycloalkylene group, -O-, -C(=O)-, or a divalent linking group of a combination of these groups.

Examples of the alkylene group in a case where L^{C2} represents an alkylene group include a chain or branched alkylene group having 1 to 20 carbon atoms.

Examples of the chain or branched alkylene group having 1 to 20 carbon atoms include a methylene group, an ethylene group, an n-propylene group, an i-propylene group, an n-butylene group, and an n-pentylene group, and a methylene group, an ethylene group, an n-propylene group, or an n-butylene group is preferable.

Examples of the cycloalkylene group in a case where L^{C2} represents a cycloalkylene group include a monocyclic or polycyclic cycloalkylene group having 3 to 20 carbon atoms.

Examples of the monocyclic or polycyclic cycloalkylene group having 3 to 20 carbon atoms include an adamantylene group, a cyclohexylene group, a cyclopentylene group, a cycloheptylene group, and a norbonylene group, and an adamantylene group, a cyclohexylene group, or a norbonylene group is preferable.

L^{C2} may have a substituent.

As a preferred aspect, examples of the substituent in a case where L^{C2} has a substituent include the substituent as the above-described preferred aspect in the case where R^{C2} has a substituent.

In a case where R^{C2} represents an aryl group, L^{C2} is preferably a single bond.

In a case where R^{C2} represents a cycloalkyl group, L^{C2} is preferably an alkylene group, -O-, -C(=O)-, or a divalent linking group of a combination of these groups, and preferably an alkylene group substituted with a fluorine atom or a group having a fluorine atom, -O-, -C(=O)-, or a divalent linking group of a combination of these groups.

As a preferred aspect, at least one of R^{C2} or L^{C2} is substituted with a fluorine atom or a group having a fluorine atom. The group having a fluorine atom is as described above.

M_{C}⁺ represents an organic cation.

M_{C}⁺ is preferably the cation represented by General Formula (ZcI) or General Formula (ZcII) described above.

### (Compound Represented by General Formula (C3))

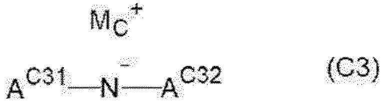

In General Formula (C3),
A^{C31} and A^{C32} each independently represent -SO₂-R^{PC1} or -CO-R^{PC2},
R^{PC1} and R^{PC2} represent an organic group, and
M_{C}⁺ represents an organic cation.

In General Formula (C3), A^{C31} and A^{C32} each independently represent -SO₂-R^{PC1} or -CO-R^{PC2}.

R^{PC1} and R^{PC2} represent an organic group.

R^{PC1} and R^{PC2} are each preferably -L^{C31}-R^{C31} and -L^{C32}-R^{C32}.

R^{C31} and R^{C32} each independently represent an alkyl group or a cycloalkyl group.

As the alkyl group in a case where R^{C31} or R^{C32} represents an alkyl group, a chain or branched alkyl group having 1 to 10 carbon atoms is preferable, and an alkyl group having 1 to 5 carbon atoms is more preferable.

Examples of the alkyl group include a methyl group, an ethyl group, an n-propyl group, and an i-propyl group, and a methyl group or an ethyl group is preferable and a methyl group is more preferable.

As the cycloalkyl group in a case where R^{C31} or R^{C32} represents a cycloalkyl group, a cycloalkyl group having 3 to 15 carbon atoms is preferable, and a cycloalkyl group having 5 to 10 carbon atoms is more preferable. The cycloalkyl group may be a monocycle or a polycycle.

Examples of the cycloalkyl group include a cyclohexyl group, a norbornyl group, a decalinyl group, and an adamantyl group, and a cyclohexyl group or an adamantyl group is preferable.

One or more of the carbon atoms which are ring member atoms of the above-described cycloalkyl group may be replaced with a carbonyl carbon atom.

R^{C31} or R^{C32} may have a substituent.

As a preferred aspect, examples of the substituent in a case where R^{C31} or R^{C32} has a substituent include the substituent as the above-described preferred aspect in the case where R^{C1} in General Formula (C1) described above has a substituent.

R^{C31} and R^{C32} are each independently preferably an alkyl group, an alkyl group substituted with a fluorine atom or a group having a fluorine atom, or a cycloalkyl group, and more preferably a methyl group, a trifluoromethyl group, a cyclohexyl group, or an adamantyl group.

L^{C31} and L^{C32} each independently represent a single bond or a divalent linking group.

It is preferable that L^{C31} and L^{C32} each independently represent a single bond, an alkylene group, a cycloalkylene group, -O-, -C(=O)-, or a divalent linking group of a combination of these groups.

Examples of the alkylene group in a case where L^{C31} or L^{C32} represents an alkylene group include a chain or branched alkylene group having 1 to 20 carbon atoms.

Examples of the chain or branched alkylene group having 1 to 20 carbon atoms include a methylene group, an ethylene group, an n-propylene group, an i-propylene group, an n-butylene group, and an n-pentylene group, and a methylene group, an ethylene group, an n-propylene group, or an n-butylene group is preferable.

Examples of the cycloalkylene group in a case where L^{C31} or L^{C32} represents a cycloalkylene group include a monocyclic or polycyclic cycloalkylene group having 3 to 20 carbon atoms.

Examples of the monocyclic or polycyclic cycloalkylene group having 3 to 20 carbon atoms include an adamantylene group, a cyclohexylene group, a cyclopentylene group, a cycloheptylene group, and a norbonylene group, and an adamantylene group, a cyclohexylene group, or a norbonylene group is preferable.

L^{C31} or L^{C32} may have a substituent.

As a preferred aspect, examples of the substituent in a case where L^{C31} or L^{C32} has a substituent include the substituent as the above-described preferred aspect in the case where R^{C31} or R^{C32} has a substituent.

It is preferable that L^{C31} and L^{C32} each independently represent a single bond, an alkylene group, or a fluorine-substituted alkylene group.

As a preferred aspect, at least one of A^{C31} or A^{C32} is substituted with a fluorine atom or a group having a fluorine atom.

In General Formula (C3), M_{C}⁺ represents an organic cation.

M_{C}⁺ is preferably the cation represented by General Formula (ZcI) or General Formula (ZcII) described above.

Preferred specific examples of the above-described compound (C) include the following compounds. In the following compounds, the anion and the cation can be optionally exchanged.

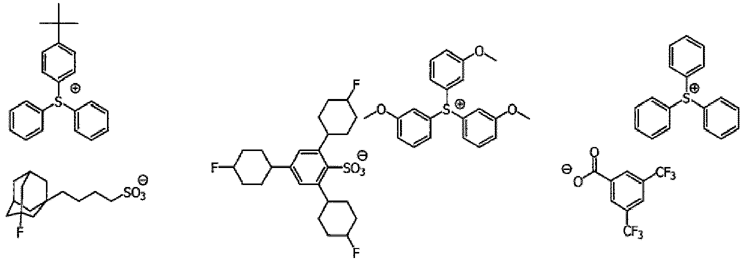

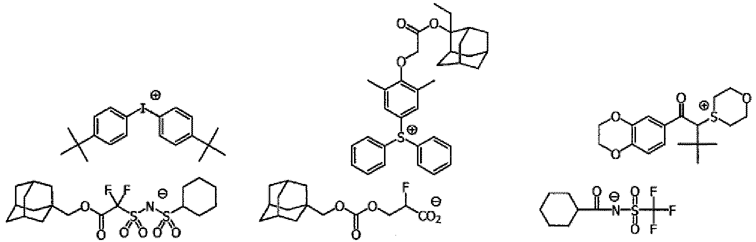

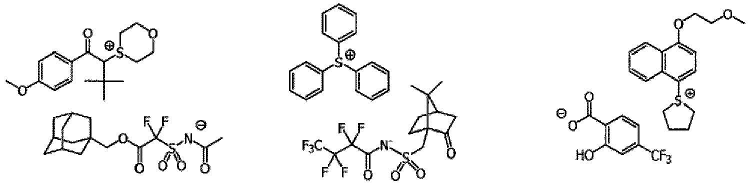

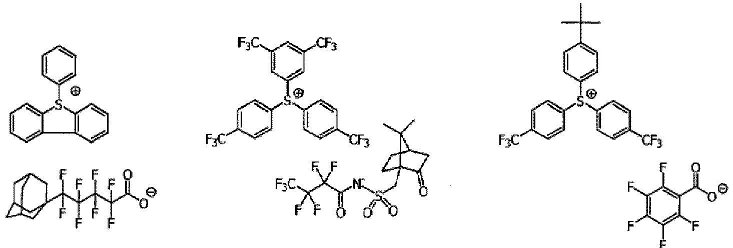

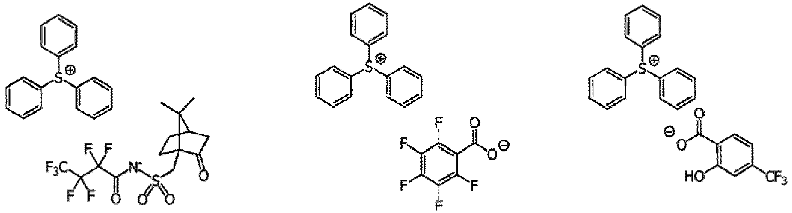

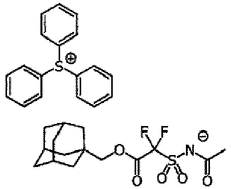

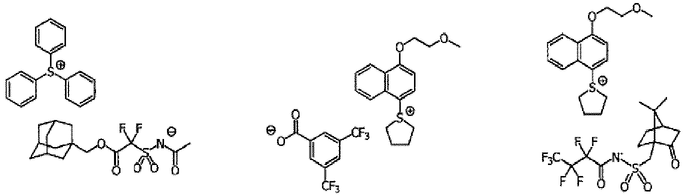

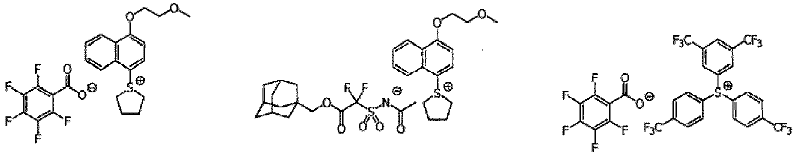

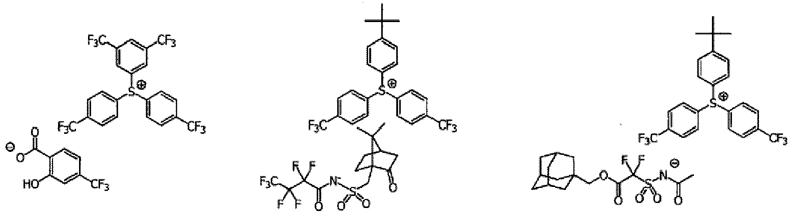

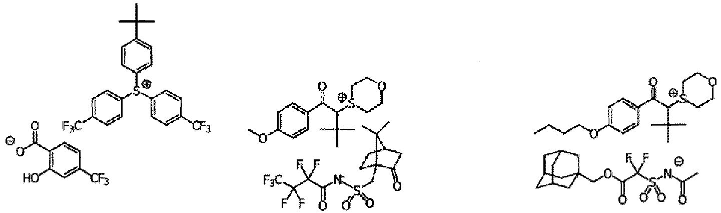

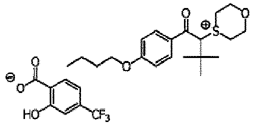

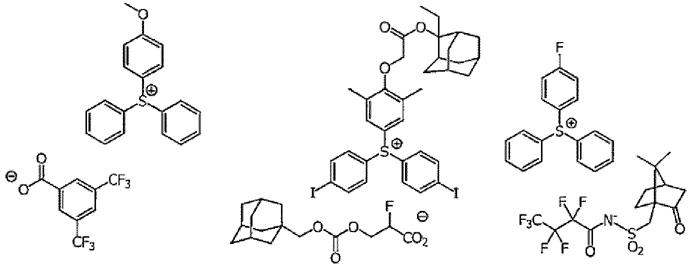

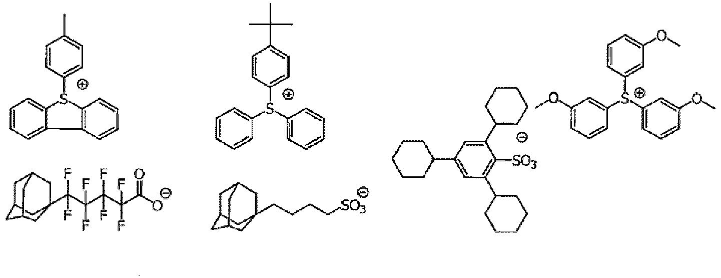

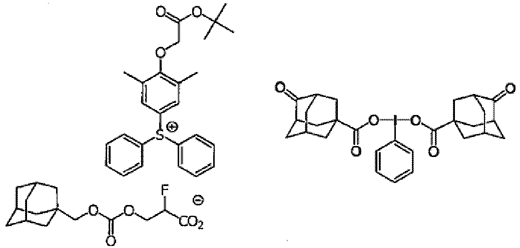

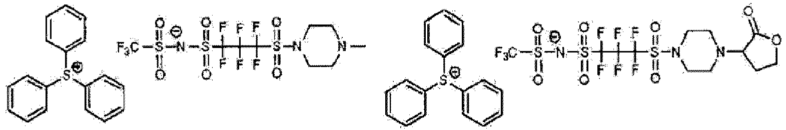

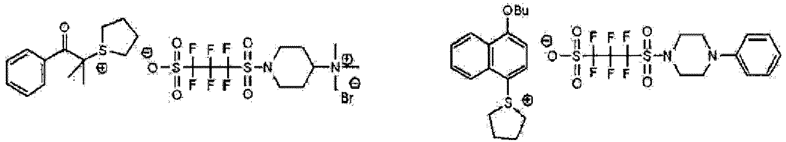

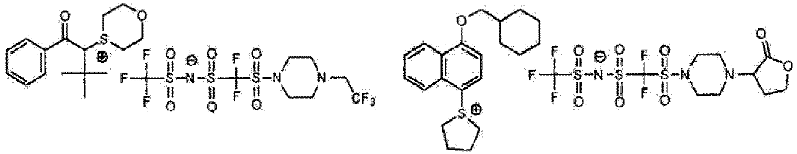

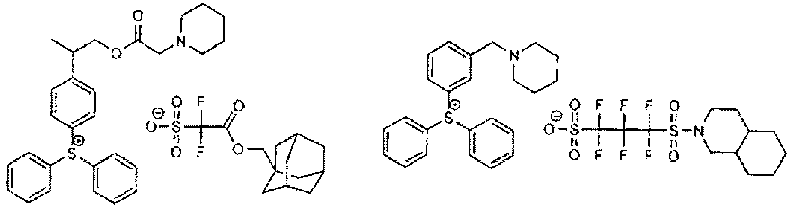

A content of the compound (C) in the composition according to the embodiment of the present invention is not particularly limited, but from the viewpoint that the effects of the present invention are more excellent, the content is preferably 1.0% by mass or more, more preferably 2.0% by mass or more, and still more preferably 5.0% by mass or more with respect to the total solid content of the composition. In addition, the above-described content is preferably 40.0% by mass or less, more preferably 35.0% by mass or less, and still more preferably 30% by mass or less.

The compound (C) may be used alone or in combination of two or more kinds thereof.

### <(D) Compound which Generates Acid by Irradiation with Actinic Ray or Radiation and is Decomposed by Irradiation with Actinic Ray or Radiation to Reduce Acid-trapping Property >

The composition according to the embodiment of the present invention may contain a compound which is the photoacid generator (B) and the compound (C), that is, (D) a compound which generates an acid by irradiation with actinic ray or radiation is decomposed by irradiation with actinic ray or radiation to reduce acid-trapping property (also referred to a compound (D), a photoacid generator-linked quencher, or a photoacid generator-linked quencher (D)). In other words, the compound (D) is a compound which has a structure having a function corresponding to the photoacid generator (B) and a structure having a function corresponding to the compound (C).

The compound (D) is a preferred aspect of the photoacid generator (B) and is also a preferred aspect of the compound (C).

As described above, in the present invention, the compound (C) is a compound that generates an acid which is relatively weak with respect to the acid generated from the photoacid generator (B). That is, the compound (C) is a compound which generates an acid having a larger pKa than the acid generated from the photoacid generator (B). In a case where the photoacid generator (B) and the compound (C) are the same compound (D), in the compound (D), the pKa of the acid generated from the structure having a function corresponding to the compound (C) is larger than the pKa of the acid generated from the structure having a function corresponding to the photoacid generator (B).

The compound (D) may be a non-ionic compound or an ionic compound.

### (Compound Represented by General Formula (PD))

The ionic compound as the compound (D) is not particularly limited, but is preferably a compound represented by General Formula (PD).

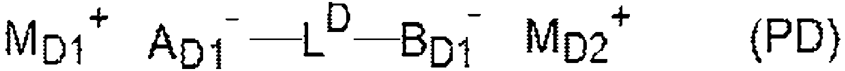

In General Formula (PD),
M_{D1}⁺ and M_{D2}⁺ each independently represent an organic cation,
L^{D} represents a divalent organic group, and
A_{D1}⁻ and B_{D1}⁻ each independently represent an acid anion group,
here, in a compound represented by HA_{D1}-L^{D}-B_{D1}H in which M_{D1}⁺ and M_{D2}⁺ of the compound represented by General Formula (PD) are each replaced with a hydrogen atom, a pKa of a group represented by HA_{D1} is lower than a pKa of a group represented by B_{D1}H.

Since the compound represented by General Formula (PD) includes both of a structure having a function corresponding to the photoacid generator (B) (a moiety corresponding to "M_{D1}⁺A_{D1}⁻-") and a structure having a function corresponding to the compound (C) (a moiety corresponding to "-B_{D1}⁻M_{D2}⁺") in one molecule, it is possible to keep a presence ratio of each of the structures constant in the resist film.

Therefore, the present inventors have presumed that, even in a case where the resist film is exposed, the amount and the diffusion of the acid generated in the resist film are likely to be uniform and the width of a pattern obtained after development is stabilized.

In General Formula (PD), M_{D1}⁺ and M_{D2}⁺ each independently represent an organic cation.

The organic cations of M_{D1}⁺ and M_{D2}⁺ are each independently preferably the cation represented by General Formula (ZcI) or General Formula (ZcII) described above.

In General Formula (PD), L^{D} represents a divalent organic group.

Examples of the divalent organic group include -COO-, -CONH-, -CO-, an alkylene group (which preferably has 1 to 6 carbon atoms, and may be linear or branched), a cycloalkylene group (preferably having 3 to 15 carbon atoms), an alkenylene group (preferably having 2 to 6 carbon atoms), and a divalent linking group formed by a combination of a plurality of these groups.

One or more of methylene groups constituting a cycloalkane ring of the above-described cycloalkylene group may be replaced with a carbonyl carbon and/or an oxygen atom.

It is also preferable that these divalent linking groups further have a group selected from the group consisting of -O-, -S-, -SO-, and -SO₂-.

Among these, L^{D} is preferably a group represented by General Formula (LD).

*A^{D}-L^{D}A-L^{D}B-L^{D}C-L^{D}D-L^{D}E-*B^{D} (LD)

In General Formula (LD), *A^{D} represents a bonding position to A_{D1}⁻ in General Formula (PD).

In General Formula (LD), *B^{D} represents a bonding position to B_{D1}⁻ in General Formula (PD).

In General Formula (LD), L^{D}A represents -(C(R^{D}_{LA1})(R^{D}_{LA2}))_{XA}^{D}- or an arylene group.

XA^{D} represents an integer of 1 or more, and is preferably 1 to 10 and more preferably 1 to 3.

R^{D}_{LA1} and R^{D}_{LA2} each independently represent a hydrogen atom or a substituent.

The substituents of R^{D}_{LA1} and R^{D}_{LA2} are each independently preferably a fluorine atom or a fluoroalkyl group, more preferably a fluorine atom or a perfluoroalkyl group, and still more preferably a fluorine atom or a perfluoromethyl group.

In a case where XA^{D} is 2 or more, XA^{D} pieces of R^{D}_{LA1}'s may be the same or different from each other.

In a case where XA^{D} is 2 or more, XA^{D} pieces of R^{D}_{LA2}'s may be the same or different from each other.
-(C(R^{D}_{LA1})(R^{D}_{LA2}))- is preferably -CH₂-, -CHF-, -CH(CF₃)-, or -CF₂-.

Among these, -(C(R^{D}_{LA1})(R^{D}_{LA2}))- which is directly bonded to A_{D1}⁻ in General Formula (PD) is preferably -CH₂-, -CHF-, -CH(CF₃)-, or -CF₂-.
-(C(R^{D}_{LA1})(R^{D}_{LA2}))-'s other than -(C(R^{D}_{LA1})(R^{D}_{LA2}))- which is directly bonded to A_{D1}⁻ in General Formula (PD) are each independently preferably -CH₂-, -CHF-, or -CF₂-.

The arylene group of L^{D}A is not particularly limited, but an arylene group having 6 to 14 carbon atoms is preferable, an arylene group having 6 to 10 carbon atoms, and a phenylene group is particularly preferable.

The arylene group may have a substituent. The substituent is not particularly limited, but a halogen atom is preferable and a fluorine atom is more preferable.

In General Formula (LD), L^{D}B represents a single bond, an ether group (-O-), an ester group (-COO-), or a sulfonyl group (-SO₂-).

In General Formula (LD), L^{D}C represents a single bond, an alkylene group, a cycloalkylene group, an arylene group, or a group formed by a combination thereof ("-alkylene group-cycloalkylene group-" and the like).

The above-described alkylene group may be linear or branched.

The number of carbon atoms in the alkylene group is preferably 1 to 5, more preferably 1 or 2, and still more preferably 1. The number of carbon atoms in the cycloalkylene group is preferably 3 to 15 and more preferably 5 to 10.

The above-described cycloalkylene group may be a monocycle or a polycycle.

Examples of the above-described cycloalkylene group include a norbornanediyl group and an adamantandiyl group.

As a substituent which may be included in the above-described cycloalkylene group, an alkyl group (may be linear or branched; preferably having 1 to 5 carbon atoms) is preferable.

One or more of methylene groups constituting a cycloalkane ring of the above-described cycloalkylene group may be replaced with a carbonyl carbon and/or a heteroatom (a nitrogen atom, an oxygen atom, and the like).

In a case where L^{D}C is "-alkylene group-cycloalkylene group-", an alkylene group moiety is preferably present on an L^{D}B side.

The arylene group of L^{D}C is not particularly limited, but an arylene group having 6 to 14 carbon atoms is preferable, an arylene group having 6 to 10 carbon atoms, and a phenylene group is particularly preferable.

The arylene group may have a substituent.

In a case where the L^{D}B is a single bond, L^{D}C is preferably a single bond or a cycloalkylene group.

In General Formula (LD), L^{D}D represents a single bond, an ether group (-O-), a carbonyl group (-CO-), or an ester group (-COO-).

In General Formula (LD), L^{D}E represents a single bond or -(C(R^{D}_{LE1})(R^{D}_{LE2}))_{XE}^{D}-.

XE^{D} in -(C(R^{D}_{LE1})(R^{D}_{LE2}))_{XE}^{D}- represents an integer of 1 or more, and is preferably 1 to 10 and more preferably 1 to 3.

R^{D}_{LE1} and R^{D}_{LE2} each independently represent a hydrogen atom or a substituent.

In a case where XE^{D} is 2 or more, XE^{D} pieces of R^{D}_{LE1}'s may be the same or different from each other.

In a case where XE^{D} is 2 or more, XE^{D} pieces of R^{D}_{LE2}'s may be the same or different from each other.

Among those, -(C(R^{D}_{LE1})(R^{D}_{LE2}))- is preferably -CH₂-. In General Formula (L^{D}), in a case where L^{D}B, L^{D}C, and L^{D}D are single bonds, it is preferable that L^{D}E is also a single bond.

In General Formula (PD), A_{D1}⁻ represents an acid anion group.

The acid anion group is a group having an anion atom.

Specifically, A_{D1}⁻ is preferably a group represented by General Formula (AD-1) or (AD-2).

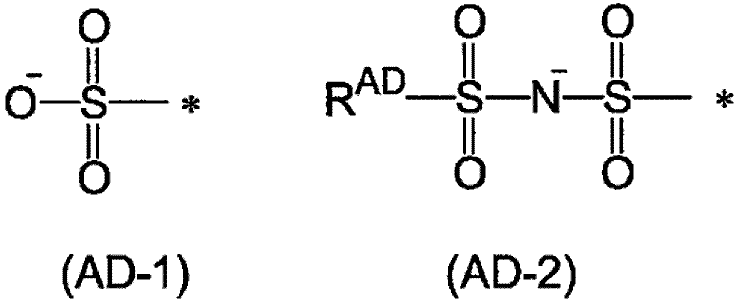

In General Formulae (AD-1) and (AD-2), * represents a bonding position.

In General Formula (AD-2), R^{AD} represents an organic group.

R^{AD} is not particularly limited, but is preferably an alkyl group.

The above-described alkyl group may be linear or branched.

The number of carbon atoms in the above-described alkyl group is preferably 1 to 10 and more preferably 1 to 5.

As a substituent which may be included in the above-described alkyl group, a fluorine atom is preferable.

The above-described alkyl group having a fluorine atom as a substituent may or may not be a perfluoroalkyl group.

In General Formula (PD), B_{D1}⁻ represents an acid anion group.

Specifically, B_{D1}⁻ is preferably a group represented by any of General Formulae (B-1) to (B-6).

B_{D1}⁻ is preferably a group represented by any of General Formulae (BD-1) to (BD-3), and more preferably a group represented by either of General Formula (BD-1) or (BD-2).

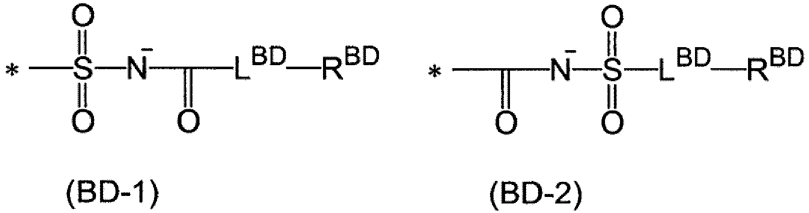

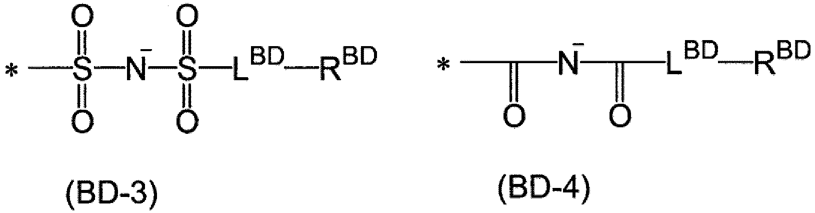

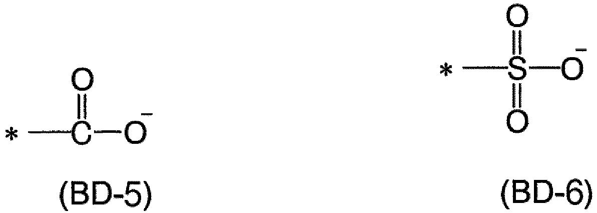

In General Formulae (BD-1) to (BD-6),
R^{BD}'s each independently represent an organic group,
L^{BD}'s each independently represent a single bond, an alkylene group, a cycloalkylene group, an arylene group, -O-, -C(=O)-, -SO₂-, or a divalent linking group of a combination of these groups, and
* represents a bonding position.

R^{BD} is preferably an alkyl group, a cycloalkyl group, or an aryl group.

As the alkyl group in a case where R^{BD} represents an alkyl group, a chain or branched alkyl group having 1 to 10 carbon atoms is preferable, and an alkyl group having 1 to 5 carbon atoms is more preferable.

Examples of the alkyl group include a methyl group, an ethyl group, an n-propyl group, and an i-propyl group, and a methyl group or an ethyl group is preferable and a methyl group is more preferable.

As the cycloalkyl group in a case where R^{BD} represents a cycloalkyl group, a cycloalkyl group having 3 to 15 carbon atoms is preferable, and a cycloalkyl group having 5 to 10 carbon atoms is more preferable. The cycloalkyl group may be a monocycle or a polycycle.

Examples of the cycloalkyl group include a cyclopentyl group, a norbornyl group, a decalinyl group, and an adamantyl group.

As the aryl group in a case where R^{BD} represents an aryl group, an aryl group having 6 to 15 carbon atoms is preferable.

Examples of the aryl group include a phenyl group and a naphthyl group, and a phenyl group is preferable.

R^{BD} may have a substituent.

As a preferred aspect, examples of the substituent in a case where R^{BD} has a substituent include the substituent as the above-described preferred aspect in the case where R^{C1} in General Formula (C1) described above has a substituent.

As a preferred aspect, examples of the substituent in a case where R^{BD} has a substituent include an alkoxycarbonyl group and SO₃⁻. An alkyl group in the alkoxycarbonyl group is not particularly limited, but is preferably a chain or branched alkyl group having 1 to 10 carbon atoms and more preferably an alkyl group having 1 to 5 carbon atoms.

L^{BD}'s each independently represent a single bond, an alkylene group, a cycloalkylene group, an arylene group, -O-, -C(=O)-, -SO₂-, or a divalent linking group of a combination of these groups.

Examples of the alkylene group in a case where L^{BD} represents an alkylene group include a chain or branched alkylene group having 1 to 20 carbon atoms.

Examples of the chain or branched alkylene group having 1 to 20 carbon atoms include a methylene group, an ethylene group, an n-propylene group, an i-propylene group, an n-butylene group, and an n-pentylene group, and a methylene group, an ethylene group, an n-propylene group, or an n-butylene group is preferable.

Examples of the cycloalkylene group in a case where L^{BD} represents a cycloalkylene group include a monocyclic or polycyclic cycloalkylene group having 3 to 20 carbon atoms.

Examples of the monocyclic or polycyclic cycloalkylene group having 3 to 20 carbon atoms include an adamantylene group, a cyclohexylene group, a cyclopentylene group, a cycloheptylene group, and a norbonylene group, and an adamantylene group, a cyclohexylene group, or a norbonylene group is preferable.

The arylene group in a case where L^{BD} is an arylene group is not particularly limited, but an arylene group having 6 to 14 carbon atoms is preferable, an arylene group having 6 to 10 carbon atoms, and a phenylene group is particularly preferable.

L^{BD} may have a substituent.

As a preferred aspect, examples of the substituent in a case where L^{BD} has a substituent include the substituent as the above-described preferred aspect in the case where R^{BD} has a substituent.

L^{BD} is preferably a single bond, a methylene group, -O-, -C(=O)-, or a divalent linking group of a combination of these groups, and more preferably a single bond or a methylene group.

In a compound represented by HA_{D1}-L^{D}-B_{D1}H in which M_{D1}⁺ and M_{D2}⁺ of the compound represented by General Formula (PD) are each replaced with a hydrogen atom, a pKa of a group represented by HA_{D1} is lower than a pKa of a group represented by B_{D1}H.

More specifically, in a case where an acid dissociation constant is determined for the compound represented by HA_{D1}-L^{D}-B_{D1}H, the pKa in a case where "HA_{D1}-L^{D}-B_{D1}H" serves as "A_{D1}⁻-L^{D}-B_{D1}H" is defined as the "pKa of a group represented by HA_{D1}", and the pKa in a case where "A_{D1}⁻-L^{D}-B_{D1}H" serves as "A_{D1}⁻-L^{D}-B_{D1}⁻" is defined as the "pKa of a group represented by B_{D1}H".

As described above, the "pKa of a group represented by HA_{D1}" and the "pKa of a group represented by B_{D1}H" are each determined using "Software Package 1" or "Gaussian 16".

Among these, the pKa of the group represented by HA_{D1} is preferably -12.00 to 1.00, more preferably -7.00 to 0.50, and still more preferably -5.00 to 0.00.

The pKa of the group represented by B_{D1}H is preferably -4.00 to 14.00, more preferably -2.00 to 12.00, and still more preferably -1.00 to 5.00.

A difference between the pKa of the group represented by B_{D1}H and the pKa of the group represented by HA_{D1} ("pKa of group represented by B_{D1}H" - "pKa of group represented by HA_{D1}") is preferably 0.10 to 20.00, more preferably 0.50 to 17.00, and still more preferably 2.00 to 15.00.

The followings are preferred specific examples of the compound corresponding to the compound (D).

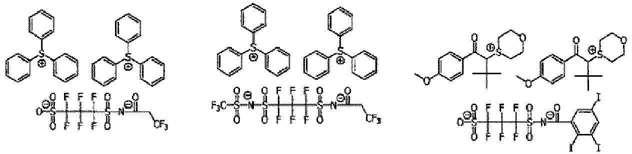

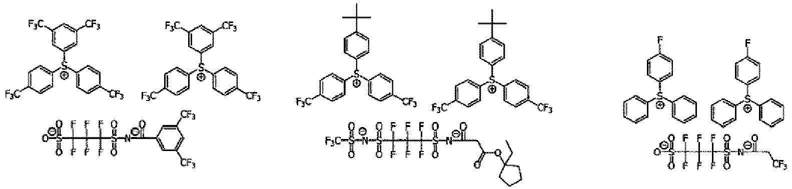

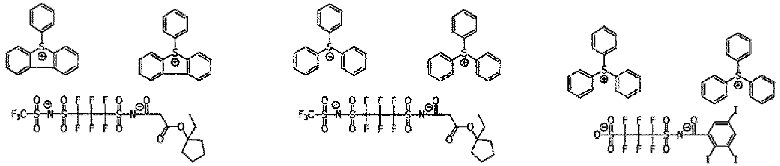

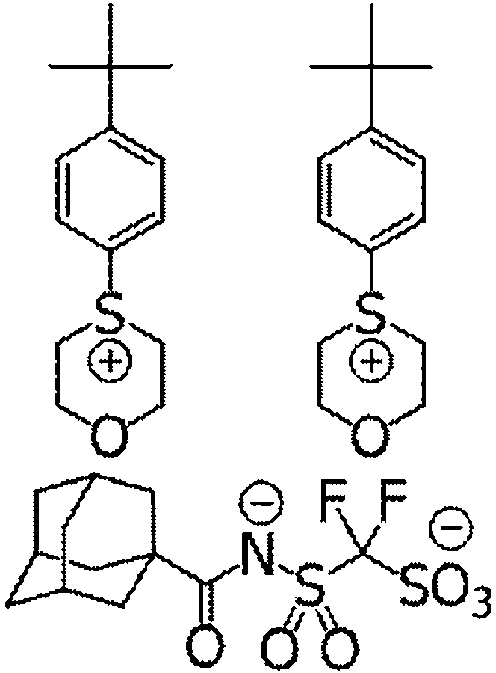

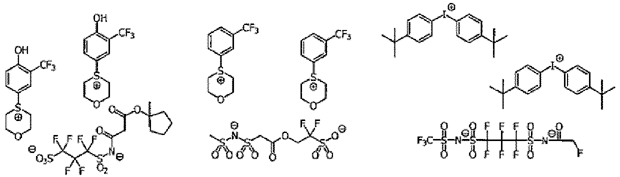

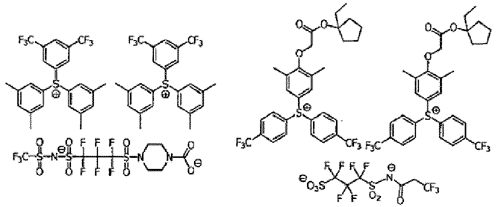

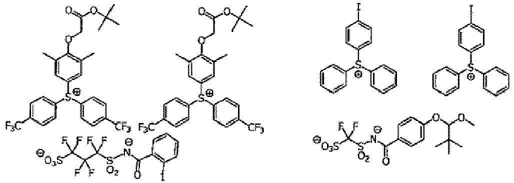

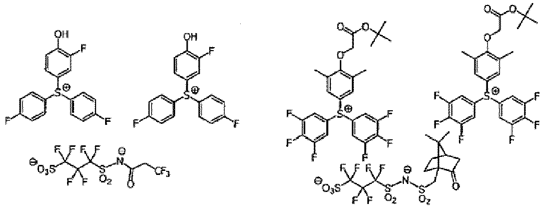

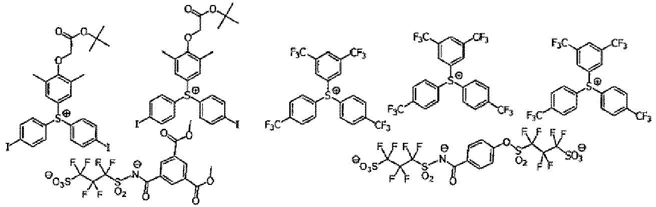

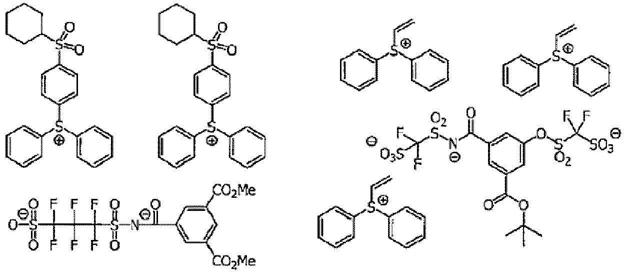

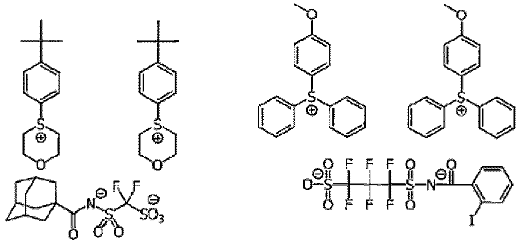

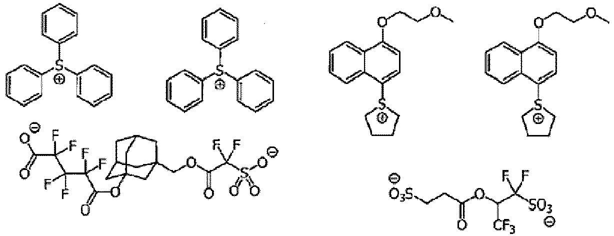

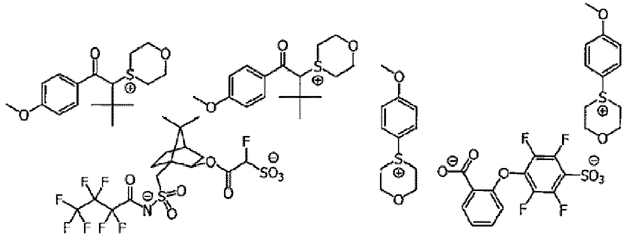

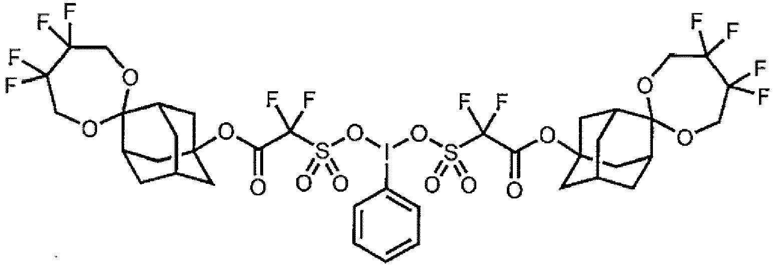

A content of the ionic compound (D) in the composition according to the embodiment of the present invention is not particularly limited, but from the viewpoint that the effects of the present invention are more excellent, the content is preferably 5% by mass or more, more preferably 9% by mass or more, and still more preferably 15% by mass or more with respect to the total solid content of the composition. In addition, the above-described content is preferably 90% by mass or less, more preferably 80% by mass or less, and still more preferably 70% by mass or less.

The compound (D) may be used alone or in combination of two or more kinds thereof.

It is also preferable that the composition according to the embodiment of the present invention contains at least one of the photoacid generator (B) or the compound (C), in addition to the compound (D).

Specific examples of a content aspect of the compound corresponding to the photoacid generator (B) or the compound (C) in the composition according to the embodiment of the present invention include the following aspects.
(i) photoacid generator (B) other than the compound (D) + ionic compound (C) other than the compound (D)
(ii) compound (D)
(iii) compound (D) + photoacid generator (B) other than the compound (D)
(iv) compound (D) + compound (C) other than the compound (D)
(v) compound (D) + photoacid generator (B) other than the compound (D) + compound (C) other than the compound (D)

In (iii), the compound (D) needs to function as the compound (C), and in this case, the pKa of the acid generated from the structure having a function corresponding to the compound (C) in the compound (D) is larger than the pKa of the acid generated from the photoacid generator (B).

In (iv), the compound (D) needs to function as the photoacid generator (B), and in this case, the pKa of the acid generated from the compound (C) is larger than the pKa of the acid generated from the structure having a function corresponding to the photoacid generator (B) in the compound (D).

In (v), the compound (D) may function as the photoacid generator (B) or as the compound (C). In a case of functioning as the photoacid generator (B), the pKa of the acid generated from the compound (C) is larger than the pKa of the acid generated from the structure having a function corresponding to the photoacid generator (B) in the compound (D). In a case of functioning as the compound (C), the pKa of the acid generated from the structure having a function corresponding to the compound (C) in the compound (D) is larger than the pKa of the acid generated from the photoacid generator (B).

### <(E) Other Acid Diffusion Control Agents>

The composition according to the embodiment of the present invention may further contain an acid diffusion control agent (also referred to as other acid diffusion control agent (E) or acid diffusion control agent (E)) other than the compound (C) and the compound (D) as long as the effects of the present invention are not impaired. The acid diffusion control agent acts as a quencher which traps an acid generated from the photoacid generator and functions to control the phenomenon of acid diffusion in the resist film.

The acid diffusion control agent (E) may be, for example, a basic compound.

The basic compound is preferably a compound having a structure represented by General Formula (A) to General Formula (E).

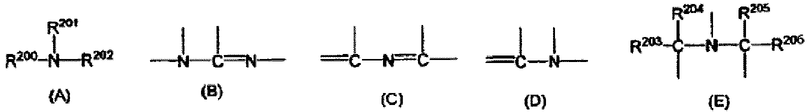

In General Formula (A) and General Formula (E), R²⁰⁰, R²⁰¹, and R²⁰² may be the same or different from each other, and each represent a hydrogen atom, an alkyl group (preferably having 1 to 20 carbon atoms), a cycloalkyl group (preferably having 3 to 20 carbon atoms), or an aryl group (preferably having 6 to 20 carbon atoms), in which R²⁰¹ and R²⁰² may be bonded to each other to form a ring.

With regard to the above-described alkyl group, an alkyl group having a substituent is preferably an aminoalkyl group having 1 to 20 carbon atoms, a hydroxyalkyl group having 1 to 20 carbon atoms, or a cyanoalkyl group having 1 to 20 carbon atoms.

R²⁰³, R²⁰⁴, R²⁰⁵, and R²⁰⁶ may be the same or different from each other, and each represent an alkyl group having 1 to 20 carbon atoms.

It is more preferable that the alkyl groups in General Formula (A) and General Formula (E) are unsubstituted.

As the basic compound, guanidine, aminopyrrolidine, pyrazole, pyrazoline, piperazine, aminomorpholine, aminoalkylmorpholine (in which an alkyl group moiety may be linear or branched, and may be partly substituted with an ether group and/or an ester group; a total number of all atoms other than hydrogen atoms in the alkyl group moiety is preferably 1 to 17), piperidine, or the like is preferable. Among these, a compound having an imidazole structure, a diazabicyclo structure, an onium hydroxide structure, an onium carboxylate structure, a trialkylamine structure, an aniline structure, or a pyridine structure; an alkylamine derivative having a hydroxyl group and/or an ether bond; an aniline derivative having a hydroxyl group and/or an ether bond; or the like is more preferable.

Examples of the compound having an imidazole structure include imidazole, 2,4,5-triphenylimidazole, and benzimidazole. Examples of the compound having a diazabicyclo structure include 1,4 -diazabicyclo [2,2,2] octane, 1,5-diazabicyclo[4,3,0]non-5-ene, and 1,8-diazabicyclo[5,4,0]undec-7-ene. Examples of the compound having an onium hydroxide structure include triarylsulfonium hydroxide, phenacylsulfonium hydroxide, and sulfonium hydroxide having a 2-oxoalkyl group. Specific examples thereof include triphenylsulfonium hydroxide, tris(t-butylphenyl)sulfonium hydroxide, bis(t-butylphenyl)iodonium hydroxide, phenacylthiophenium hydroxide, and 2-oxopropylthiophenium hydroxide. The compound having an onium carboxylate structure is formed by carboxylation of an anionic moiety of a compound having an onium hydroxide structure, and examples thereof include acetate, adamantane-1-carboxylate, and perfluoroalkyl carboxylate. Examples of the compound having a trialkylamine structure include tri(n-butyl)amine and tri(n-octyl)amine. Examples of the aniline compound include 2,6-diisopropylaniline, N,N-dimethylaniline, N,N-dibutylaniline, and N,N-dihexylaniline. Examples of the alkylamine derivative having a hydroxyl group and/or an ether bond include ethanolamine, diethanolamine, triethanolamine, tris(methoxyethoxyethyl)amine, and (HO-C₂H₄-O-C₂H₄)2N(-C₃H₆-O-CH₃). Examples of the aniline derivative having a hydroxyl group and/or an ether bond include N,N-bis(hydroxyethyl)aniline.

Preferred examples of the basic compound include an amine compound having a phenoxy group and an ammonium salt compound having a phenoxy group.

As the amine compound, for example, a primary, secondary, or tertiary amine compound can be used, and an amine compound in which at least one alkyl group is bonded to a nitrogen atom is preferable. The amine compound is more preferably a tertiary amine compound. As long as at least one alkyl group (preferably having 1 to 20 carbon atoms) is bonded to the nitrogen atom, the amine compound may have a cycloalkyl group (preferably having 3 to 20 carbon atoms) or an aryl group (preferably having 6 to 12 carbon atoms) bonded to the nitrogen atom in addition to the alkyl group.

In addition, the amine compound preferably has an oxyalkylene group. The number of oxyalkylene groups is preferably 1 or more, more preferably 3 to 9, and still more preferably 4 to 6, within the molecule. Among those, as the oxyalkylene group, an oxyethylene group (-CH₂CH₂O-) or an oxypropylene group (-CH(CH₃)CH₂O- or -CH₂CH₂CH₂O-) is preferable, and an oxyethylene group is more preferable.

Examples of the ammonium salt compound include primary, secondary, tertiary, and quaternary ammonium salt compounds, and an ammonium salt compound in which at least one alkyl group is bonded to a nitrogen atom is preferable. As long as at least one alkyl group (preferably having 1 to 20 carbon atoms) is bonded to the nitrogen atom, the ammonium salt compound may have a cycloalkyl group (preferably having 3 to 20 carbon atoms) or an aryl group (preferably having 6 to 12 carbon atoms) bonded to the nitrogen atom in addition to the alkyl group.

It is preferable that the ammonium salt compound has an oxyalkylene group. The number of oxyalkylene groups is preferably 1 or more, more preferably 3 to 9, and still more preferably 4 to 6, within the molecule. Among the oxyalkylene groups, an oxyethylene group (-CH₂CH₂O-) or an oxypropylene group (-CH(CH₃)CH₂O- or -CH₂CH₂CH₂O-) is preferable, and an oxyethylene group is more preferable.

Examples of the anion of the ammonium salt compound include a halogen atom, a sulfonate, a borate, and a phosphate, and among these, a halogen atom or a sulfonate is preferable. The halogen atom is preferably a chlorine atom, a bromine atom, or an iodine atom. The sulfonate is preferably an organic sulfonate having 1 to 20 carbon atoms. Examples of the organic sulfonate include an alkyl sulfonate and an aryl sulfonate, which have 1 to 20 carbon atoms. The alkyl group of the alkyl sulfonate may have a substituent, and examples of the substituent include a fluorine atom, a chlorine atom, a bromine atom, an alkoxy group, an acyl group, and an aromatic ring group. Examples of the alkyl sulfonate include methanesulfonate, ethanesulfonate, butanesulfonate, hexanesulfonate, octanesulfonate, benzyl sulfonate, trifluoromethanesulfonate, pentafluoroethanesulfonate, and nonafluorobutanesulfonate. Examples of an aryl group of the aryl sulfonate include a benzene ring group, a naphthalene ring group, and an anthracene ring group. The substituent which can be included in the benzene ring group, naphthalene ring group, and anthracene ring group is preferably a linear or branched alkyl group having 1 to 6 carbon atoms or a cycloalkyl group having 3 to 6 carbon atoms. Examples of the linear or branched alkyl group and the cycloalkyl group include a methyl group, an ethyl group, an n-propyl group, an isopropyl group, an n-butyl group, an i-butyl group, a t-butyl group, an n-hexyl group, and a cyclohexyl group. Examples of other substituents include an alkoxy group having 1 to 6 carbon atoms, a halogen atom, a cyano group, a nitro group, an acyl group, and an acyloxy group.

The amine compound having a phenoxy group and the ammonium salt compound having a phenoxy group are each a compound having a phenoxy group at the terminal on the opposite side to the nitrogen atom of the alkyl group which is included in the amine compound or the ammonium salt compound.

Examples of a substituent of the phenoxy group include an alkyl group, an alkoxy group, a halogen atom, a cyano group, a nitro group, a carboxylic acid group, a carboxylic acid ester group, a sulfonic acid ester group, an aryl group, an aralkyl group, an acyloxy group, and an aryloxy group. A substitution position of the substituent may be any of 2- to 6-positions. The number of substituents may be any of 1 to 5.

The compound preferably has at least one oxyalkylene group between the phenoxy group and the nitrogen atom. The number of oxyalkylene groups is preferably 1 or more, more preferably 3 to 9, and still more preferably 4 to 6, within the molecule. Among the oxyalkylene groups, an oxyethylene group (-CH₂CH₂O-) or an oxypropylene group (-CH(CH₃)CH₂O- or -CH₂CH₂CH₂O-) is preferable, and an oxyethylene group is more preferable.

The amine compound having a phenoxy group can be obtained by heating a mixture of a primary or secondary amine having a phenoxy group and a haloalkyl ether to perform a reaction, then adding an aqueous solution of a strong base (for example, sodium hydroxide, potassium hydroxide, tetraalkylammonium, and the like) to a reaction system, and extracting the reaction product with an organic solvent (for example, ethyl acetate, chloroform, and the like). Alternatively, the amine compound having a phenoxy group can also be obtained by heating a mixture of a primary or secondary amine and a haloalkyl ether having a phenoxy group at the terminal to perform a reaction, then adding an aqueous solution of the strong base to a reaction system, and extracting the reaction product with an organic solvent.

A low-molecular-weight compound having a nitrogen atom and a group which is eliminated by the action of acid can also be used as the acid diffusion control agent. The above-described low-molecular-weight compound is preferably an amine derivative having, on the nitrogen atom, a group which is eliminated by the action of acid.

The group which is eliminated by the action of acid is preferably an acetal group, a carbonate group, a carbamate group, a tertiary ester group, a tertiary hydroxyl group, or a hemiaminal ether group, and more preferably a carbamate group or a hemiaminal ether group.

A molecular weight of the low-molecular-weight compound is preferably 100 to 1000, more preferably 100 to 700, and still more preferably 100 to 500.

The low-molecular-weight compound may have a carbamate group having a protective group on the nitrogen atom.

Specific examples of the acid diffusion control agent (E) are shown below, but the present invention is not limited.

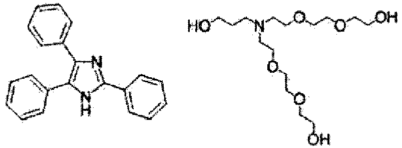

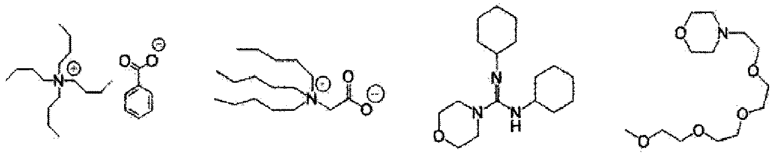

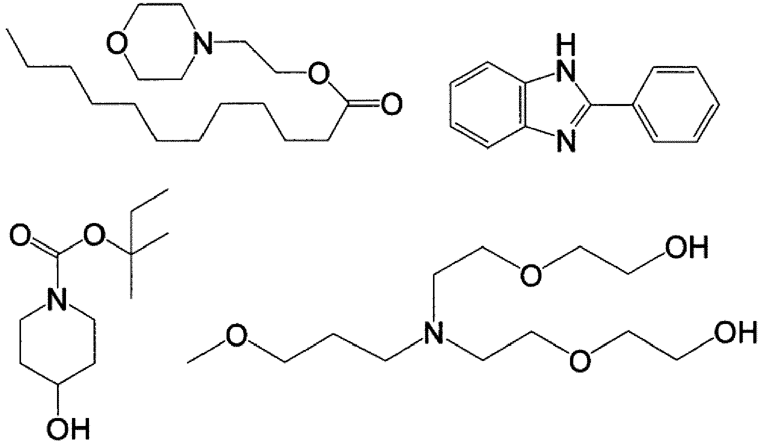

In a case where the composition according to the embodiment of the present invention contains the acid diffusion control agent (E), a content of the acid diffusion control agent (E) is preferably 0.001% to 20.0% by mass, more preferably 0.01% to 5.0% by mass, and still more preferably 0% by mass with respect to the total solid content of the composition.

The acid diffusion control agent (E) may be used alone or in combination of two or more kinds thereof.

Examples of the acid diffusion control agent (E) include compounds (amine compounds, amide group-containing compounds, urea compounds, nitrogen-containing heterocyclic compounds, and the like) described in paragraphs [0140] to [0144] of JP2013-11833A.

### <(F) Hydrophobic Resin>

The composition according to the embodiment of the present invention may contain a hydrophobic resin different from the resin (A), in addition to the above-described resin (A).

Although it is preferable that the hydrophobic resin is designed to be unevenly distributed on a surface of the resist film, it is not necessary to have a hydrophilic group in the molecule as different from a surfactant, and is not necessary to contribute to uniform mixing of polar materials and non-polar materials.

Examples of an effect caused by the addition of the hydrophobic resin include a control of static and dynamic contact angles of a surface of the resist film with respect to water and suppression of outgas.

From the viewpoint of uneven distribution on the film surface layer, the hydrophobic resin preferably has any one or more of a "fluorine atom", a "silicon atom", and a "CH₃ partial structure which is included in a side chain moiety of a resin", and more preferably has two or more kinds thereof. In addition, the above-described hydrophobic resin preferably has a hydrocarbon group having 5 or more carbon atoms. These groups may be included in the main chain of the resin or may be substituted in the side chain of the resin.

In a case where hydrophobic resin includes a fluorine atom and/or a silicon atom, the fluorine atom and/or the silicon atom in the hydrophobic resin may be included in the main chain or the side chain of the resin.

In a case where the hydrophobic resin includes a fluorine atom, as a partial structure having a fluorine atom, an alkyl group having a fluorine atom, a cycloalkyl group having a fluorine atom, or an aryl group having a fluorine atom is preferable.

The alkyl group having a fluorine atom (preferably having 1 to 10 carbon atoms and more preferably having 1 to 4 carbon atoms) is a linear or branched alkyl group in which at least one hydrogen atom is substituted with a fluorine atom, and the alkyl group may further have a substituent other than the fluorine atom.

The cycloalkyl group having a fluorine atom is a monocyclic or polycyclic cycloalkyl group in which at least one hydrogen atom is substituted with a fluorine atom, and may further have a substituent other than the fluorine atom.

Examples of the aryl group having a fluorine atom include an aryl group such as a phenyl group and a naphthyl group, in which at least one hydrogen atom is substituted with a fluorine atom, and the aryl group may further have a substituent other than the fluorine atom.

Examples of the repeating unit having a fluorine atom or a silicon atom include the repeating units exemplified in paragraph [0519] of US2012/0251948A1.

In addition, as described above, it is also preferable that the hydrophobic resin includes a CH₃ partial structure in the side chain moiety.

Here, the CH₃ partial structure included in the side chain moiety of the hydrophobic resin includes a CH₃ partial structure included in an ethyl group, a propyl group, and the like.

On the other hand, since a methyl group directly bonded to the main chain of the hydrophobic resin (for example, an α-methyl group in a repeating unit having a methacrylic acid structure) has only a small contribution of uneven distribution on the surface of the hydrophobic resin due to the effect of the main chain, the methyl group is not included in the CH₃ partial structure in the present invention.

With regard to the hydrophobic resin, reference can be made to the description in paragraphs [0348] to [0415] of JP2014-010245A, the contents of which are incorporated herein by reference.

For the hydrophobic resin, the resins described in JP2011-248019A, JP2010-175859A, and JP2012-032544A can also be preferably used, in addition to the resins described above.

In a case where the composition according to the embodiment of the present invention contains a hydrophobic resin, a content of the hydrophobic resin is preferably 0.01% to 20% by mass, and more preferably 0.1% to 15% by mass with respect to the total solid content of the composition.

### <Solvent>

The composition according to the embodiment of the present invention may contain a solvent.

The solvent preferably includes at least one solvent of (M1) propylene glycol monoalkyl ether carboxylate or (M2) at least one selected from the group consisting of propylene glycol monoalkyl ether, lactic acid ester, acetic acid ester, alkoxypropionic acid ester, chain ketone, cyclic ketone, lactone, and alkylene carbonate. The solvent may further include a component other than the components (M1) and (M2).

The present inventors have found that, by using such a solvent and the above-described resin in combination, a pattern having a small number of development defects can be formed while improving coating property of the composition. A reason for this is not always clear, but the present inventors have considered that, since these solvents have a good balance of solubility, boiling point, and viscosity of the above-described resin, unevenness of a film thickness of a composition film, generation of precipitates during spin coating, and the like can be suppressed.

As the component (M1), at least one selected from the group consisting of propylene glycol monomethyl ether acetate (PGMEA), propylene glycol monomethyl ether propionate, and propylene glycol monoethyl ether acetate is preferable, and propylene glycol monomethyl ether acetate (PGMEA) is more preferable.

The component (M2) is preferably the following solvent.

The propylene glycol monoalkyl ether is preferably propylene glycol monomethyl ether (PGME) or propylene glycol monoethyl ether (PGEE).

The lactic acid ester is preferably ethyl lactate, butyl lactate, or propyl lactate.

The acetic acid ester is preferably methyl acetate, ethyl acetate, butyl acetate, isobutyl acetate, propyl acetate, isoamyl acetate, methyl formate, ethyl formate, butyl formate, propyl formate, or 3-methoxybutyl acetate.

In addition, butyl butyrate is also preferable.

The alkoxypropionic acid ester is preferably methyl 3-methoxypropionate (MMP) or ethyl 3-ethoxypropionate (EEP).

The chain ketone is preferably 1-octanone, 2-octanone, 1-nonanone, 2-nonanone, acetone, 2-heptanone, 4-heptanone, 1-hexanone, 2-hexanone, diisobutyl ketone, phenyl acetone, methyl ethyl ketone, methyl isobutyl ketone, acetyl acetone, acetonyl acetone, ionone, diacetonyl alcohol, acetyl carbinol, acetophenone, methyl naphthyl ketone, or methyl amyl ketone.

The cyclic ketone is preferably methylcyclohexanone, isophorone, cyclopentanone, or cyclohexanone.

The lactone is preferably γ-butyrolactone.

The alkylene carbonate is preferably propylene carbonate.

The component (M2) is more preferably propylene glycol monomethyl ether (PGME), ethyl lactate, ethyl 3-ethoxypropionate, methyl amyl ketone, cyclohexanone, butyl acetate, pentyl acetate, y-butyrolactone, or propylene carbonate.

In addition to the above-described components, it is preferable to use an ester-based solvent having 7 or more carbon atoms (preferably 7 to 14 carbon atoms, more preferably 7 to 12 carbon atoms, and still more preferably 7 to 10 carbon atoms) and 2 or less heteroatoms.

Examples of the ester-based solvent having 7 or more carbon atoms and 2 or less heteroatoms include amyl acetate, 2-methylbutyl acetate, 1-methylbutyl acetate, hexyl acetate, pentyl propionate, hexyl propionate, butyl propionate, isobutyl isobutyrate, heptyl propionate, and butyl butanoate, and isoamyl acetate is preferable.

The component (M2) is preferably a solvent having a flash point (hereinafter, also referred to as fp) of 37°C or higher.

Such a component (M2) is preferably propylene glycol monomethyl ether (fp: 47°C), ethyl lactate (fp: 53°C), ethyl 3-ethoxypropionate (fp: 49°C), methyl amyl ketone (fp: 42°C), cyclohexanone (fp: 44°C), pentyl acetate (fp: 45°C), methyl 2-hydroxyisobutyrate (fp: 45°C), y-butyrolactone (fp: 101°C), or propylene carbonate (fp: 132°C). Among these, propylene glycol monoethyl ether, ethyl lactate, pentyl acetate, or cyclohexanone is more preferable, and propylene glycol monoethyl ether or ethyl lactate is still more preferable.

The "flash point" herein means a value described in a reagent catalog of Tokyo Chemical Industry Co., Ltd. or Sigma-Aldrich Co. LLC.

It is preferable that the solvent includes the component (M1). The solvent is more preferably composed of substantially only the component (M1) or is a mixed solvent of the component (M1) and other components. In the latter case, it is still more preferable that the solvent includes both the component (M1) and the component (M2).

A mass ratio (M1/M2) of the component (M1) to the component (M2) is preferably "100/0" to "0/10", more preferably "100/0" to "15/85", still more preferably "100/0" to "40/60", and particularly preferably "100/0" to "60/40".

That is, in a case where the solvent includes both the component (M1) and the component (M2), the mass ratio of the component (M1) to the component (M2) is preferably 15/85 or more, more preferably 40/60 or more, and still more preferably 60/40 or more. In a case where such a configuration is adopted, the number of development defects is reduced.

In a case where both of the component (M1) and the component (M2) are included in the solvent, the mass ratio of the component (M1) to the component (M2) is set to, for example, 99/1 or less.

As described above, the solvent may further include a component other than the components (M1) and (M2). In this case, a content of the component other than the components (M1) and (M2) is preferably 5% to 30% by mass with respect to the total amount of the solvent.

A content of the solvent in the composition according to the embodiment of the present invention is preferably set such that the concentration of solid contents is 0.5% to 30% by mass, and more preferably set such that the concentration of solid contents is 1% to 20% by mass. With this content, the coating property of the composition according to the embodiment of the present invention can be further improved.

The solid content means all components excluding the solvent.

The concentration of solid contents is a mass percentage of other components excluding the solvent with respect to the total mass of the composition according to the embodiment of the present invention.

The "total solid content" refers to a total mass of components obtained by removing the solvent from the whole composition of the composition according to the embodiment of the present invention. In addition, the "solid content" refers to components excluding the solvent as described above, and the components may be, for example, a solid or a liquid at 25°C.

### <Surfactant>

The composition according to the embodiment of the present invention may contain a surfactant. In a case where the surfactant is included, it is possible to form a pattern having more excellent adhesiveness and fewer development defects.

The surfactant is preferably a fluorine-based and/or silicon-based surfactant. Examples of the fluorine-based and/or silicon-based surfactant include the surfactants described in paragraph [0276] of the specification of US2008/0248425A. In addition, EFTOP EF301 or EF303 (manufactured by Shin-Akita Chemical Co., Ltd.); FLUORAD FC430, 431, and 4430 (manufactured by Sumitomo 3M inc.); MEGAFACE F 171, F-173, F-176, F-189, F-113, F-110, F-177, F-120, and R08 (manufactured by DIC Corporation); SURFLON S-382, SC101, 102, 103, 104, 105, or 106 (manufactured by Asahi Glass Co., Ltd.); TROYSOL S-366 (manufactured by Troy Corporation); GF-300 or GF-150 (manufactured by Toagosei Co., Ltd.); SURFLON S-393 (manufactured by AGC Seimi Chemical Co., Ltd.); EFTOP EF121, EF122A, EF122B, RF122C, EF125M, EF135M, EF351, EF352, EF801, EF802, or EF601 (manufactured by JEMCO Inc.); PF636, PF656, PF6320, and PF6520 (manufactured by OMNOVA Solutions Inc.); KH-20 (manufactured by Asahi Kasei Corporation); or FTX-204G, 208G, 218G, 230G, 204D, 208D, 212D, 218D, and 222D (manufactured by NEOS COMPANY LIMITED) may be used. A polysiloxane polymer KP-341 (manufactured by Shin-Etsu Chemical Co., Ltd.) can also be used as the silicon-based surfactant.

Moreover, in addition to the known surfactants as described above, the surfactant may be synthesized using a fluoroaliphatic compound produced by a telomerization method (also referred to as a telomer method) or an oligomerization method (also referred to as an oligomer method). Specifically, a polymer including a fluoroaliphatic group derived from the fluoroaliphatic compound may be used as the surfactant. The fluoroaliphatic compound can be synthesized, for example, by the method described in JP2002-90991A.

In addition, a surfactant other than the fluorine-based and/or the silicon-based surfactants described in paragraph [0280] of the specification of US2008/0248425A may be used.

The surfactant may be used alone or in combination of two or more kinds thereof.

In a case where the composition according to the embodiment of the present invention contains a surfactant, a content of the surfactant is preferably 0.0001% to 2% by mass, and more preferably 0.0005% to 1% by mass with respect to the total solid content of the composition.

### <Other Additives>

The composition according to the embodiment of the present invention may further contain a dissolution inhibiting compound, a dye, a plasticizer, a photosensitizer, a light absorbing agent, and/or a compound promoting a solubility in a developer (for example, a phenol compound having a molecular weight of 1,000 or less or an alicyclic or aliphatic compound including a carboxylic acid group), or the like.

The composition according to the embodiment of the present invention may further contain a dissolution inhibiting compound. Here, the "dissolution inhibiting compound" is intended to be a compound having a molecular weight of 3000 or less, in which solubility in an organic developer decreases by decomposition due to the action of acid.

### [Use]

The composition according to the embodiment of the present invention relates to an actinic ray-sensitive or radiation-sensitive resin composition having properties which change by undergoing a reaction by irradiation with actinic ray or radiation. More specifically, the composition according to the embodiment of the present invention relates to an actinic ray-sensitive or radiation-sensitive resin composition which is used in a step of manufacturing a semiconductor such as an integrated circuit (IC), for manufacturing of a circuit board for a liquid crystal, a thermal head, or the like, manufacturing of a mold structure for imprinting, other photofabrication steps, or production of a planographic printing plate or an acid-curable composition. A pattern formed in the present invention can be used in an etching step, an ion implanting step, a bump electrode forming step, a rewiring forming step, a microelectromechanical system (MEMS), or the like.

### [Actinic Ray-sensitive or Radiation-sensitive Film]

The present invention also relates to an actinic ray-sensitive or radiation-sensitive film (typically, "resist film") formed of the actinic ray or radiation-sensitive resin composition according to the present invention. Such a film is formed, for example, by applying the composition according to the embodiment of the present invention onto a support such as a substrate. A thickness of the film is preferably 0.02 to 0.1 µm.

As a method for applying the composition onto the substrate, the composition is applied to the substrate by an appropriate coating method such as a spin coating, a roll coating, a flow coating, a dip coating, a spray coating, and a doctor coating. Among these, a spin coating is preferable, and the rotation speed thereof is preferably 1000 to 3000 rotations per minute (rpm). The coating film is prebaked at 60°C to 150°C for 1 to 20 minutes, preferably at 80°C to 120°C for 1 to 10 minutes to form a thin film.

For a material constituting the substrate to be processed and an outermost layer thereof, for example, in a case of a semiconductor wafer, a silicon wafer can be used, and examples of the material forming the outermost layer include Si, SiO₂, SiN, SiON, and TiN, WSi, BPSG, SOG, and an organic antireflection film.

### [Pattern Forming Method]

A procedure of a pattern forming method using the above-described actinic ray-sensitive or radiation-sensitive resin composition is not particularly limited, but it is preferable to include the following steps.

Step 1: step of forming an actinic ray or radiation-sensitive film on a substrate using the actinic ray-sensitive or radiation-sensitive resin composition

Step 2: step of exposing the actinic ray or radiation-sensitive film (preferably, to EUV light)

Step 3: step of developing the exposed actinic ray or radiation-sensitive film with a developer to form a pattern

Hereinafter, the procedure of each of the above-described steps will be described in detail.

### <Step 1: Actinic Ray or Radiation-Sensitive Film Forming Step>

The step 1 is a step of forming an actinic ray or radiation-sensitive film on a substrate using the actinic ray-sensitive or radiation-sensitive resin composition.

Examples of the method of forming the actinic ray or radiation-sensitive film on the substrate using the actinic ray-sensitive or radiation-sensitive resin composition include a method of applying the actinic ray-sensitive or radiation-sensitive resin composition onto the substrate.

In addition, it is preferable that the actinic ray-sensitive or radiation-sensitive resin composition before the application is filtered through a filter, as desired. A pore size of the filter is preferably 0.1 µm or less, more preferably 0.05 µm or less, and still more preferably 0.03 µm or less. In addition, the filter is preferably a polytetrafluoroethylene-made filter, a polyethylene-made filter, or a nylon-made filter.

The actinic ray-sensitive or radiation-sensitive resin composition can be applied onto the substrate (for example, silicon and silicon dioxide coating) as used in the manufacture of integrated circuit elements by a suitable application method such as an application using a spinner or a coater. The coating method is preferably a spin application using a spinner. A rotation speed upon the spin application using a spinner is preferably 1000 to 3000 rpm.

After the application of the actinic ray-sensitive or radiation-sensitive resin composition, the substrate may be dried to form the actinic ray-sensitive or radiation-sensitive film. In addition, various underlying films (an inorganic film, an organic film, or an antireflection film) may be formed on an underlayer of the resist film.

Examples of the drying method include a method of heating and drying. The heating can be carried out using a unit included in an ordinary exposure machine and/or development machine, and may also be carried out using a hot plate or the like. A heating temperature is preferably 80°C to 150°C, more preferably 80°C to 140°C, and still more preferably 80°C to 130°C. A heating time is preferably 30 to 1000 seconds, more preferably 60 to 800 seconds, and still more preferably 60 to 600 seconds.

A film thickness of the actinic ray-sensitive or radiation-sensitive film is not particularly limited, but from the viewpoint that a fine pattern having higher accuracy can be formed, is preferably 10 to 65 nm and more preferably 15 to 50 nm.

A topcoat may be formed on an upper layer of the actinic ray-sensitive or radiation-sensitive film using a topcoat composition.

It is preferable that the topcoat composition is not mixed with the actinic ray-sensitive or radiation-sensitive film and can be uniformly applied onto the upper layer of the actinic ray-sensitive or radiation-sensitive film.

In addition, it is preferable that the actinic ray-sensitive or radiation-sensitive film is dried before forming the topcoat. Subsequently, the topcoat composition can be applied onto the obtained actinic ray-sensitive or radiation-sensitive film by the same unit as for the method for forming the actinic ray-sensitive or radiation-sensitive film described above, and further dried to form a topcoat.

A film thickness of the topcoat is preferably 10 to 200 nm, more preferably 20 to 100 nm, and still more preferably 40 to 80 nm.

The topcoat is not particularly limited, a topcoat known in the related art can be formed by the methods known in the related art, and for example, the topcoat can be formed based on the description in paragraphs [0072] to [0082] of JP2014-059543A.

For example, it is preferable that a topcoat including a basic compound as described in JP2013-61648A is formed on the actinic ray-sensitive or radiation-sensitive film. Specific examples of the basic compound which can be included in the topcoat include a basic compound which may be contained in the actinic ray-sensitive or radiation-sensitive resin composition described above.

In addition, it is preferable that the topcoat includes a compound which includes at least one group or bond selected from the group consisting of an ether bond, a thioether bond, a hydroxyl group, a thiol group, a carbonyl bond, and an ester bond.

### <Step 2: Exposing Step>

The step 2 is a step of exposing the actinic ray-sensitive or radiation-sensitive film (preferably, to EUV light).

Examples of an exposing method include a method in which the formed actinic ray-sensitive or radiation-sensitive film is irradiated with EUV light through a predetermined mask.

It is preferable to perform baking (heating) before performing development and after the exposure. The baking accelerates a reaction in the exposed portion, and the sensitivity and the pattern shape are improved.

A heating temperature is preferably 80°C to 150°C, more preferably 80°C to 140°C, and still more preferably 80°C to 130°C.

A heating time is preferably 10 to 1000 seconds, more preferably 10 to 180 seconds, and still more preferably 30 to 120 seconds.

The heating can be carried out using a unit included in an ordinary exposure machine and/or development machine, and may also be performed using a hot plate or the like.

This step is also referred to as a post-exposure baking.

### <Step 3: Developing Step>

The step 3 is a step of developing the exposed actinic ray-sensitive or radiation-sensitive film with a developer to form a pattern.

The developer may be either an alkali developer or a developer containing an organic solvent (hereinafter, also referred to as an organic developer).

Examples of a developing method include a method in which the substrate is immersed in a tank filled with a developer for a certain period of time (a dipping method), a method in which a development is performed by heaping a developer up onto the surface of the substrate by surface tension, and then leaving it to stand for a certain period of time (a puddle method), a method in which a developer is sprayed on the surface of the substrate (a spraying method), and a method in which a developer is continuously jetted onto the substrate rotating at a constant rate while scanning a developer jetting nozzle at a constant rate (a dynamic dispensing method).

In addition, after the step of performing the development, a step of stopping the development may be carried out while replacing the solvent with another solvent.

A developing time is not particularly limited as long as it is a period of time where the non-exposed portion of the resin is sufficiently dissolved, and is preferably 10 to 300 seconds and more preferably 20 to 120 seconds.

A temperature of the developer is preferably 0°C to 50°C and more preferably 15°C to 35°C.

As the alkali developer, it is preferable to use an aqueous alkali solution including an alkali. The type of the aqueous alkali solution is not particularly limited, and examples thereof include an aqueous alkali solution including a quaternary ammonium salt typified by tetramethylammonium hydroxide, an inorganic alkali, a primary amine, a secondary amine, a tertiary amine, an alcoholamine, a cyclic amine, or the like. Among these, the alkali developer is preferably aqueous solutions of the quaternary ammonium salts typified by tetramethylammonium hydroxide (TMAH). An appropriate amount of alcohols, a surfactant, or the like may be added to the alkali developer. An alkali concentration of the alkali developer is usually 0.1% to 20% by mass. In addition, a pH of the alkali developer is usually 10.0 to 15.0.

The organic developer is preferably a developer containing at least one organic solvent selected from the group consisting of a ketone-based solvent, an ester-based solvent, an alcohol-based solvent, an amide-based solvent, an ether-based solvent, and a hydrocarbon-based solvent.

Examples of the ketone-based solvent include 1-octanone, 2-octanone, 1-nonanone, 2-nonanone, acetone, 2-heptanone (methyl amyl ketone), 4-heptanone, 1-hexanone, 2-hexanone, diisobutyl ketone, cyclohexanone, methylcyclohexanone, phenyl acetone, methyl ethyl ketone, methyl isobutyl ketone, acetyl acetone, acetonyl acetone, ionone, diacetonyl alcohol, acetyl carbinol, acetophenone, methyl naphthyl ketone, isophorone, and propylene carbonate.

Examples of the ester-based solvent include methyl acetate, butyl acetate, ethyl acetate, isopropyl acetate, pentyl acetate, isopentyl acetate, propylene glycol monomethyl ether acetate, ethylene glycol monoethyl ether acetate, diethylene glycol monobutyl ether acetate, diethylene glycol monoethyl ether acetate, ethyl-3-ethoxypropionate, 3-methoxybutyl acetate, 3-methyl-3-methoxybutyl acetate, methyl formate, ethyl formate, butyl formate, propyl formate, ethyl lactate, butyl lactate, propyl lactate, butyl butyrate, methyl 2-hydroxyisobutyrate, isobutyl isobutyrate, and butyl propionate.

As the alcohol-based solvent, the amide-based solvent, the ether-based solvent, and the hydrocarbon-based solvent, for example, the solvents described in paragraphs [0715] to [0718] of the specification of US2016/0070167A1 can be used.

A plurality of the above-described solvents may be mixed, or the solvent may be used in admixture with a solvent other than those described above or water. A moisture content in the entire developer is preferably less than 50% by mass, more preferably less than 20% by mass, and still more preferably less than 10% by mass, and it is particularly preferable that the entire developer contains substantially no water.

A content of the organic solvent with respect to the organic developer is preferably 50% by mass to 100% by mass, more preferably 80% by mass to 100% by mass, still more preferably 90% by mass to 100% by mass, and particularly preferably 95% by mass to 100% by mass with respect to the total amount of the developer.

### <Other Steps>

It is preferable that the above-described pattern forming method includes a step of performing washing using a rinsing liquid after the step 3.

Examples of the rinsing liquid used in the rinsing step after the step of performing development using an alkali developer include pure water. An appropriate amount of a surfactant may be added to the pure water. An appropriate amount of a surfactant may be added to the rinsing liquid.

The rinsing liquid used in the rinsing step after the developing step with an organic developer is not particularly limited as long as the rinsing liquid does not dissolve the resist pattern, and a solution including a common organic solvent can be used. As the rinsing liquid, a rinsing liquid containing at least one organic solvent selected from the group consisting of a hydrocarbon-based solvent, a ketone-based solvent, an ester-based solvent, an alcohol-based solvent, an amide-based solvent, and an ether-based solvent is preferably used.

Examples of the hydrocarbon-based solvent, the ketone-based solvent, the ester-based solvent, the alcohol-based solvent, the amide-based solvent, and the ether-based solvent include the same as those described for the developer including an organic solvent.

A method for the rinsing step is not particularly limited, and examples thereof include a method in which the rinsing liquid is continuously jetted onto the substrate rotated at a constant rate (a spin coating method), a method in which the substrate is immersed in a tank filled with the rinsing liquid for a certain period of time (a dipping method), and a method in which the rinsing liquid is sprayed on the surface of the substrate (a spraying method).

In addition, the pattern forming method according to the embodiment of the present invention may include a heating step (postbaking) after the rinsing step. By this step, the developer and the rinsing liquid remaining between and inside the patterns are removed by baking. In addition, this step also has an effect that a resist pattern is annealed and the surface roughness of the pattern is improved. The heating step after the rinsing step is usually performed at 40°C to 250°C (preferably 90°C to 200°C) for usually 10 seconds to 3 minutes (preferably 30 seconds to 120 seconds).

In addition, an etching treatment on the substrate may be carried out using the formed pattern as a mask. That is, the substrate (or the underlayer film and the substrate) may be processed using the pattern formed in the step 3 as a mask to form a pattern on the substrate.

A method for processing the substrate (or the underlayer film and the substrate) is not particularly limited, but a method in which a pattern is formed on a substrate by subjecting the substrate (or the underlayer film and the substrate) to dry etching using the pattern formed in the step 3 as a mask is preferable.

The dry etching may be one-stage etching or multi-stage etching. In a case where the etching is an etching including a plurality of stages, etchings at the respective stages may be the same treatment or different treatments.

For etching, any of known methods can be used, and various conditions and the like are appropriately determined according to the type of the substrate, usage, and the like. The etching can be carried out, for example, in accordance with Journal of The International Society for Optical Engineering (Proc. of SPIE), Vol. 6924, 692420 (2008), JP2009-267112A, and the like. In addition, the etching can also be carried out in accordance with "Chapter 4 Etching" in "Semiconductor Process Text Book, 4th Ed., published in 2007, publisher: SEMI Japan".

Among these, oxygen plasma etching is preferable as the dry etching.

It is preferable that various materials (for example, the solvent, the developer, the rinsing liquid, a composition for forming the antireflection film, a composition for forming the topcoat, and the like) used in the composition according to the embodiment of the present invention and the pattern forming method according to the embodiment of the present invention do not include impurities such as metals. A content of the impurities included in these materials is preferably 1 ppm by mass or less, more preferably 10 ppb by mass or less, still more preferably 100 ppt by mass or less, particularly preferably 10 ppt by mass or less, and most preferably 1 ppt by mass or less. Here, examples of the metal impurities include Na, K, Ca, Fe, Cu, Mg, Al, Li, Cr, Ni, Sn, Ag, As, Au, Ba, Cd, Co, Pb, Ti, V, W, and Zn.

Examples of a method for removing the impurities such as metals from the various materials include filtration using a filter. As for a filter pore diameter, the pore size is preferably less than 100 nm, more preferably 10 nm or less, and still more preferably 5 nm or less. As a filter, a polytetrafluoroethylene-made filter, a polyethylene-made filter, or a nylon-made filter is preferable. The filter may be composed of a composite material in which the above-described filter material is combined with an ion exchange medium. As the filter, a filter which has been washed with an organic solvent in advance may be used. In the step of filter filtration, plural kinds of filters connected in series or in parallel may be used. In a case of using the plural kinds of filters, a combination of filters having different pore diameters and/or materials may be used. In addition, various materials may be filtered plural times, and the step of filtering plural times may be a circulatory filtration step.

In the production of the composition according to the embodiment of the present invention, for example, it is preferable to dissolve the respective components such as the resin and the photoacid generator in the solvent, and then perform a circulatory filtration using a plurality of filters having different materials. For example, it is preferable to connect a polyethylene-made filter having a pore diameter of 50 nm, a nylon-made filter having a pore diameter of 10 nm, and a polyethylene-made filter having a pore diameter of 3 nm in permuted connection, and then perform the circulatory filtration ten times or more. A smaller pressure difference between the filters is more preferable, and the pressure difference is generally 0.1 MPa or less, preferably 0.05 MPa or less, and more preferably 0.01 MPa or less. A smaller pressure difference between the filter and the charging nozzle is more preferable, and the pressure difference is generally 0.5 MPa or less, preferably 0.2 MPa or less, and more preferably 0.1 MPa or less.

It is preferable to subject the inside of a device for producing the composition according to the embodiment of the present invention to gas replacement with an inert gas such as nitrogen. As a result, it is possible to suppress the dissolution of an active gas such as oxygen in the composition.

After being filtered by a filter, the composition according to the embodiment of the present invention is charged into a clean container. It is preferable that the composition according to the embodiment of the present invention charged in the container is stored in a refrigerator. As a result, performance deterioration over time is suppressed. A shorter time from the completion of charging the composition into the container to the start of the storage in a refrigerator is more preferable, and the time is generally 24 hours or less, preferably 16 hours or less, more preferably 12 hours or less, and still more preferably 10 hours or less. A storage temperature is preferably 0°C to 15°C, more preferably 0°C to 10°C, and still more preferably 0°C to 5°C.

In addition, examples of a method for reducing the impurities such as metals included in the various materials include a method of selecting raw materials having a low content of metals as raw materials constituting the various materials, a method of subjecting raw materials constituting the various materials to filter filtration, and a method of performing distillation under the condition for suppressing the contamination as much as possible by, for example, lining the inside of a device with TEFLON (registered trademark).

In addition to the filter filtration, removal of the impurities by an adsorbing material may be performed, or a combination of filter filtration and an adsorbing material may be used. As the adsorbing material, known adsorbing materials can be used, and for example, inorganic adsorbing materials such as silica gel and zeolite and organic adsorbing materials such as activated carbon can be used. It is necessary to prevent the incorporation of impurities such as metals in the production process in order to reduce the metal impurities included in the above-described various materials. Sufficient removal of the metal impurities from a production device can be confirmed by measuring the content of metal components included in a washing solution used to wash the production device. A content of the metal components included in the washing solution after the use is preferably 100 parts per trillion (ppt) by mass or less, more preferably 10 ppt by mass or less, and still more preferably 1 ppt by mass or less.

A conductive compound may be added to an organic treatment liquid such as the rinsing liquid in order to prevent breakdown of chemical liquid pipes and various parts (a filter, an O-ring, a tube, or the like) due to electrostatic charging, and subsequently generated electrostatic discharging. The conductive compound is not particularly limited, and examples thereof include methanol. An addition amount is not particularly limited, but from the viewpoint that preferred development characteristics or rinsing characteristics are maintained, the addition amount is preferably 10% by mass or less and more preferably 5% by mass or less.

For members of the chemical liquid pipe, for example, various pipes coated with stainless steel (SUS), or a polyethylene, polypropylene, or a fluororesin (a polytetrafluoroethylene resin, a perfluoroalkoxy resin, or the like) that has been subjected to an antistatic treatment can be used. In the same manner, for the filter or the O-ring, polyethylene, polypropylene, or a fluororesin (polytetrafluoroethylene, a perfluoroalkoxy resin, or the like) that has been subjected to an antistatic treatment can be used.

A method for improving the surface roughness of the pattern may be adopted to the pattern formed by the method according to the embodiment of the present invention. Examples of the method for improving the surface roughness of the pattern include the method of treating a pattern by a plasma of a hydrogen-containing gas described in WO2014/002808A. Additional examples of the method include known methods as described in JP2004-235468A, US2010/0020297A, JP2008-83384A, and Proc. of SPIE Vol. 8328 83280N-1 "EUV Resist Curing Technique for LWR Reduction and Etch Selectivity Enhancement".

In a case where the formed pattern is in a form of a line, an aspect ratio determined by dividing a height of the pattern with a line width of the pattern is preferably 2.5 or less, more preferably 2.1 or less, and still more preferably 1.7 or less.

In a case where the formed pattern is in a form of a trench (groove) pattern or a contact hole pattern, an aspect ratio determined by dividing a height of the pattern with a trench width or hole diameter of the pattern is preferably 4.0 or less, more preferably 3.5 or less, and still more preferably 3.0 or less.

The pattern forming method according to the embodiment of the present invention can also be used for forming a guide pattern in a directed self-assembly (DSA) (see, for example, ACS Nano Vol. 4, No. 8, Pages 4815-4823).

In addition, a pattern formed by the above-described method can be used as a core material (core) of the spacer process described in, for example, JP1991-270227A (JP-H3-270227A) and JP2013-164509A.

In addition, the present invention further relates to a method for manufacturing an electronic device, including the above-described pattern forming method, and an electronic device manufactured by this manufacturing method.

The electronic device according to the embodiment of the present invention is suitably mounted on electric and electronic apparatus (for example, home appliances, office automation (OA)-related equipment, media-related equipment, optical equipment, telecommunication equipment, and the like).

### Examples

Hereinbelow, the present invention will be described in more detail with reference to Examples. The materials, the amounts of materials used, the proportions, the treatment details, the treatment procedure, and the like shown in Examples below may be modified as appropriate as long as the modifications do not depart from the spirit of the present invention. Therefore, the scope of the present invention should not be construed as being limited to Examples shown below.

[Various Components of Actinic Ray-Sensitive or Radiation-Sensitive Resin Composition]

### [Resin (A)]

Resins A (resins A-1 to A-69 and A'-1 and A'-2) shown in Table 3 are shown below.

As the resins A-1 to A-68 and A'-1 and A'-2, those synthesized according to a method for synthesizing a resin A-1 (Synthesis Example 2) which will be described later were used. A compositional ratio (ratio in % by mole; corresponding in order from the left) of each repeating unit shown below, a weight-average molecular weight (Mw), and a dispersity (Mw/Mn) are shown in Table 1.

The weight-average molecular weight (Mw) and the dispersity (Mw/Mn) of the resins A-1 to A-69 and A'-1 and A'-2 were measured by GPC (solvent: tetrahydrofuran (THF)). In addition, the compositional ratio (ratio in % by mole) of the resin was measured by means of ¹³C-nuclear magnetic resonance (NMR).

Although the resins A'-1 and A'-2 are not the resin (A), they are listed in the resin (A) in Table 1 for convenience.

**[Table 1-1]**

| Table 1 | Molar ratio of repeating unit 1 | | Molar ratio of repeating unit 2 | | Molar ratio of repeating unit 3 | | Molar ratio of repeating unit 4 | | Molar ratio of repeating unit 5 | | Mw | Mw/Mn |
|---|---|---|---|---|---|---|---|---|---|---|---|---|
| Resin A-1 | - | - | - | - | M-70 | 50 | M-31 | 50 | - | - | 6500 | 1.60 |
| Resin A-2 | - | - | - | - | M-71 | 35 | M-32 | 40 | M-33 | 25 | 8000 | 1.54 |
| Resin A-3 | - | - | - | - | M-72 | 30 | M-33 | 70 | - | - | 9000 | 1.67 |
| Resin A-4 | - | - | - | - | M-73 | 40 | M-34 | 60 | - | - | 7500 | 1.55 |
| Resin A-5 | - | - | - | - | M-74 | 20 | M-35 | 80 | - | - | 8500 | 1.59 |
| Resin A-6 | - | - | - | - | M-75 | 50 | M-36 | 30 | M-37 | 20 | 5500 | 1.68 |
| Resin A-7 | - | - | - | - | M-76 | 45 | M-37 | 30 | M-38 | 25 | 4500 | 1.77 |
| Resin A-8 | M-69 | 10 | - | - | M-77 | 40 | M-38 | 50 | - | - | 12000 | 1.84 |
| Resin A-9 | - | - | M-68 | 15 | M-78 | 25 | M-39 | 60 | - | - | 14000 | 1.65 |
| Resin A-10 | M-67 | 5 | - | - | M-79 | 35 | M-40 | 60 | - | - | 8800 | 1.55 |
| Resin A-11 | M-66 | 10 | - | - | M-80 | 30 | M-41 | 45 | M-32 | 15 | 22000 | 1.56 |
| Resin A-12 | - | - | M-1 | 35 | M-81 | 25 | M-42 | 40 | - | - | 14000 | 1.65 |
| Resin A-13 | - | - | M-2 | 15 | M-82 | 25 | M-43 | 60 | - | - | 6500 | 1.40 |
| Resin A-14 | - | - | M-3 | 15 | M-83 | 35 | M-44 | 50 | - | - | 6700 | 1.50 |
| Resin A-15 | - | - | M-4 | 10 | M-84 | 55 | M-45 | 15 | M-41 | 20 | 7200 | 1.54 |
| Resin A-16 | - | - | M-5 | 5 | M-85 | 40 | M-46 | 55 | | | 8000 | 1.29 |
| Resin A-17 | - | - | M-6 | 30 | M-70 | 10 | M-47 | 60 | - | - | 4500 | 1.35 |
| Resin A-18 | M-7 | 10 | - | - | M-71 | 35 | M-48 | 55 | - | - | 5000 | 1.25 |
| Resin A-19 | M-8 | 25 | - | - | M-72 | 25 | M-49 | 40 | M-40 | 10 | 5500 | 1.50 |
| Resin A-20 | M-9 | 10 | - | - | M-73 | 40 | M-50 | 50 | - | - | 5600 | 1.60 |
| Resin A-21 | M-10 | 10 | - | - | M-74 | 25 | M-51 | 35 | M-58 | 30 | 7800 | 1.56 |
| Resin A-22 | M-11 | 15 | - | - | M-75 | 40 | M-52 | 45 | - | - | 9000 | 1.54 |
| Resin A-23 | M-12 | 15 | - | - | M-76 | 35 | M-53 | 50 | - | - | 8000 | 1.74 |
| Resin A-24 | M-13 | 10 | - | - | M-77 | 25 | M-54 | 65 | - | - | 7500 | 1.55 |
| Resin A-25 | M-14 | 5 | - | - | M-78 | 35 | M-55 | 60 | - | - | 8600 | 1.72 |
| Resin A-26 | M-15 | 30 | - | - | M-79 | 10 | M-56 | 60 | - | - | 12500 | 1.80 |
| Resin A-27 | - | - | M-16 | 5 | M-80 | 25 | M-57 | 70 | - | - | 5500 | 1.33 |
| Resin A-28 | M-17 | 15 | - | - | M-81 | 40 | M-58 | 45 | - | - | 4500 | 1.40 |
| Resin A-29 | M-18 | 20 | - | - | M-82 | 25 | M-59 | 55 | - | - | 6500 | 1.55 |
| Resin A-30 | M-19 | 25 | - | - | M-83 | 15 | M-60 | 60 | - | - | 6000 | 1.65 |
| Resin A-31 | M-20 | 20 | - | - | M-84 | 10 | M-61 | 70 | - | - | 5900 | 1.56 |
| Resin A-32 | M-21 | 10 | - | - | M-85 | 40 | M-62 | 50 | - | - | 8900 | 1.55 |
| Resin A-33 | M-22 | 15 | - | - | M-70 | 25 | M-63 | 60 | - | - | 12000 | 1.65 |
| Resin A-34 | M-23 | 10 | - | - | M-71 | 20 | M-64 | 70 | - | - | 8000 | 1.55 |
| Resin A-35 | M-24 | 5 | - | - | M-72 | 20 | M-65 | 75 | - | - | 14000 | 1.56 |

**[Table 1-2]**

| Table 1 | Molar ratio of repeating unit 1 | | Molar ratio of repeating unit 2 | | Molar ratio of repeating unit 3 | | Molar ratio of repeating unit 4 | | Molar ratio of repeating unit 5 | | Mw | Mw/Mn |
|---|---|---|---|---|---|---|---|---|---|---|---|---|
| Resin A-36 | M-25 | 5 | - | - | M-73 | 30 | M-31 | 65 | - | - | 12000 | 1.65 |
| Resin A-37 | M-26 | 10 | - | - | M-74 | 30 | M-32 | 60 | - | - | 8000 | 1.40 |
| Resin A-38 | - | - | M-27 | 15 | M-75 | 30 | M-33 | 55 | - | - | 6500 | 1.54 |
| Resin A-39 | M-28 | 15 | - | - | M-76 | 35 | M-34 | 50 | - | - | 5500 | 1.29 |
| Resin A-40 | M-29 | 10 | - | - | M-77 | 30 | M-35 | 60 | - | - | 4000 | 1.35 |
| Resin A-41 | - | - | M-30 | 10 | M-78 | 35 | M-36 | 55 | - | - | 8800 | 1.25 |
| Resin A-42 | - | - | M-1 | 20 | M-79 | 10 | M-37 | 70 | - | - | 10000 | 1.50 |
| Resin A-43 | - | - | M-2 | 15 | M-80 | 20 | M-38 | 65 | - | - | 7100 | 1.67 |
| Resin A-44 | - | - | M-3 | 15 | M-81 | 15 | M-39 | 70 | - | - | 6600 | 1.65 |
| Resin A-45 | - | - | M-4 | 10 | M-82 | 45 | M-40 | 45 | - | - | 12200 | 1.55 |
| Resin A-46 | - | - | M-5 | 5 | M-83 | 45 | M-41 | 50 | - | - | 7700 | 1.56 |
| Resin A-47 | - | - | M-6 | 5 | M-84 | 40 | M-42 | 55 | - | - | 6700 | 1.65 |
| Resin A-48 | M-13 | 10 | - | - | M-85 | 25 | M-43 | 65 | - | - | 5400 | 1.40 |
| Resin A-49 | - | - | M-4 | 30 | M-70 | 15 | M-31 | 55 | - | - | 4300 | 1.54 |
| Resin A-50 | M-15 | 15 | - | - | M-71 | 45 | M-32 | 40 | - | - | 5400 | 1.29 |
| Resin A-51 | M-19 | 15 | M-1 | 5 | M-72 | 45 | M-33 | 35 | - | - | 6800 | 1.35 |
| Resin A-52 | M-22 | 15 | M-2 | 10 | M-73 | 20 | M-34 | 55 | - | - | 7100 | 1.25 |
| Resin A-53 | - | - | M-3 | 20 | M-74 | 30 | M-35 | 50 | - | - | 7400 | 1.50 |
| Resin A-54 | - | - | M-4 | 15 | M-75 | 25 | M-36 | 60 | - | - | 6900 | 1.60 |
| Resin A-55 | - | - | M-5 | 25 | M-76 | 10 | M-31 | 65 | - | - | 8500 | 1.55 |
| Resin A-56 | - | - | M-6 | 30 | M-77 | 20 | M-32 | 50 | - | - | 6500 | 1.55 |
| Resin A-57 | M-9 | 10 | - | - | M-78 | 45 | M-33 | 45 | - | - | 5500 | 1.55 |
| Resin A-58 | M-13 | 15 | - | - | M-79 | 35 | M-43 | 50 | - | - | 4000 | 1.55 |
| Resin A-59 | - | - | M-1 | 20 | M-80 | 15 | M-32 | 65 | - | - | 8800 | 1.55 |
| Resin A-60 | - | - | M-2 | 15 | M-81 | 25 | M-43 | 60 | - | - | 10000 | 1.55 |
| Resin A-61 | - | - | M-3 | 15 | M-82 | 15 | M-33 | 70 | - | - | 7100 | 1.55 |
| Resin A-62 | - | - | M-4 | 30 | M-83 | 15 | M-31 | 55 | - | - | 6600 | 1.55 |
| Resin A-63 | - | - | M-5 | 35 | M-84 | 30 | M-32 | 35 | - | - | 12200 | 1.55 |
| Resin A-64 | - | - | M-6 | 25 | M-85 | 35 | M-33 | 40 | - | - | 7700 | 1.55 |
| Resin A-65 | - | - | - | - | M-70 | 50 | M-33 | 50 | - | - | 6700 | 1.55 |
| Resin A-66 | - | - | - | - | M-86 | 50 | M-33 | 50 | - | - | 6700 | 1.55 |
| Resin A-67 | - | - | - | - | M-87 | 50 | M-33 | 50 | - | - | 6700 | 1.55 |
| Resin A-68 | - | - | - | - | M-88 | 50 | M-33 | 50 | - | - | 6700 | 1.55 |
| Resin A-69 | - | - | - | - | M-91 | 50 | M-33 | 50 | - | - | 6700 | 1.55 |
| Resin A'-1 | M-89 | 50 | - | - | - | - | M-31 | 50 | - | - | 6500 | 1.60 |
| Resin A'-2 | M-90 | 50 | - | - | - | - | M-31 | 50 | - | - | 6500 | 1.60 |

Structures of monomers M-1 to M-91 corresponding to respective repeating units constituting resins A-1 to A-69, A'-1, and A'-2 shown in Table 1 are shown below.

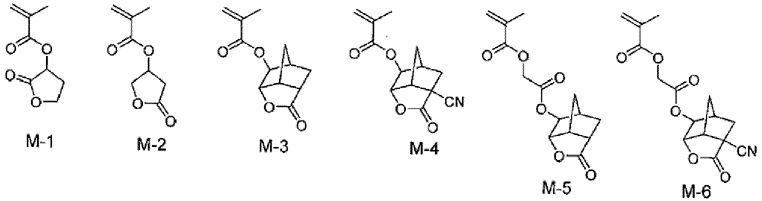

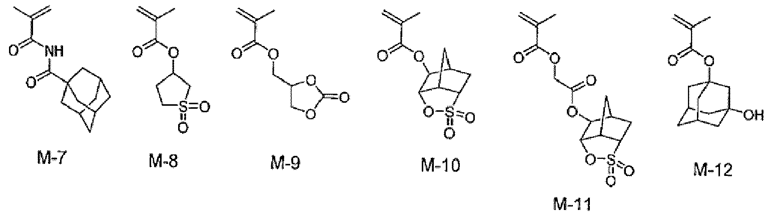

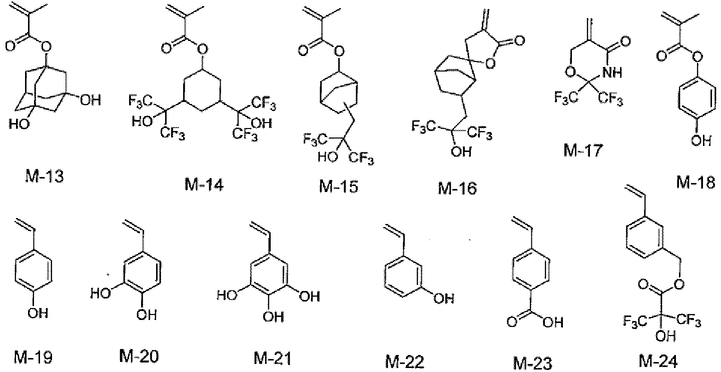

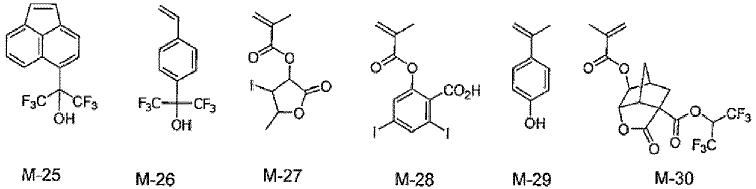

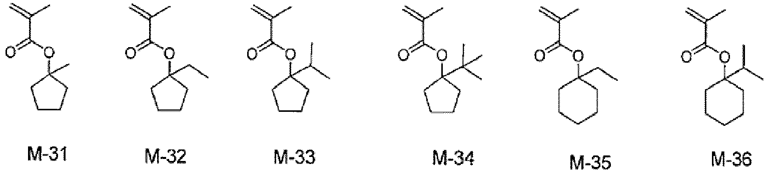

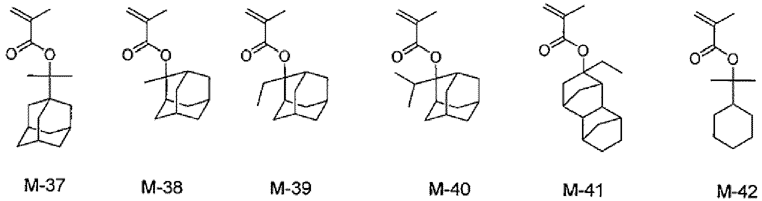

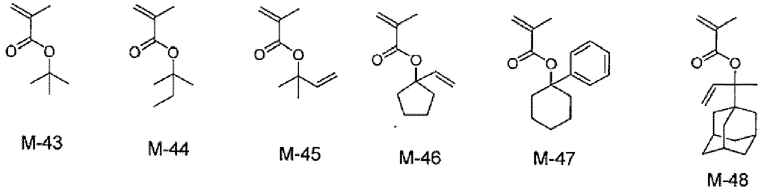

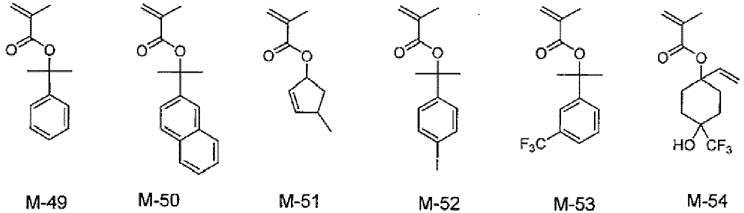

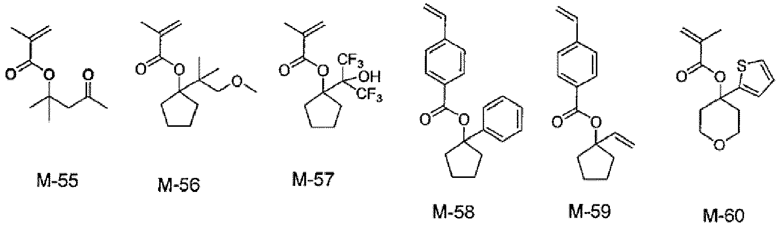

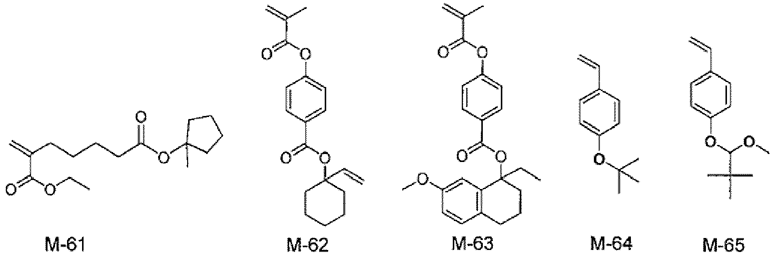

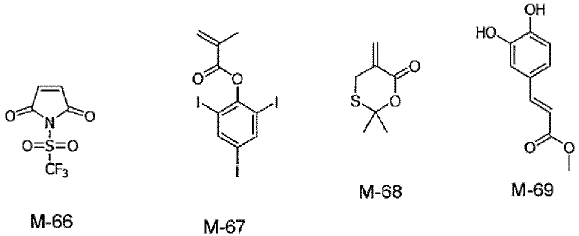

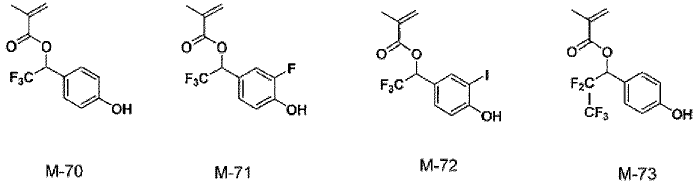

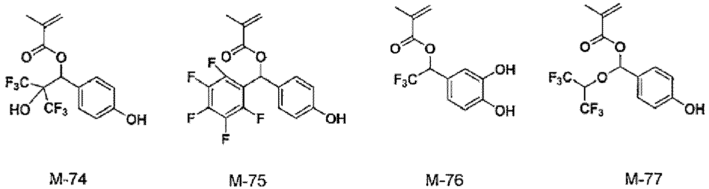

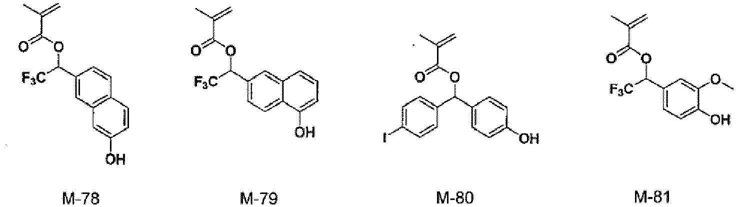

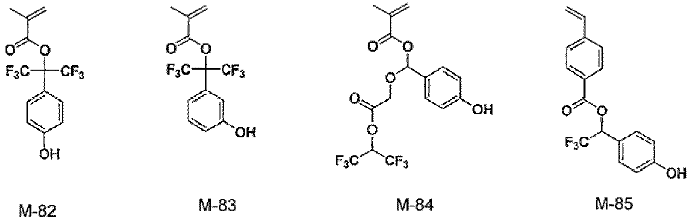

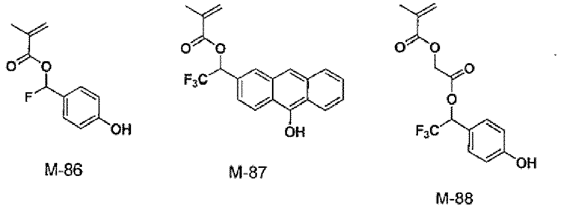

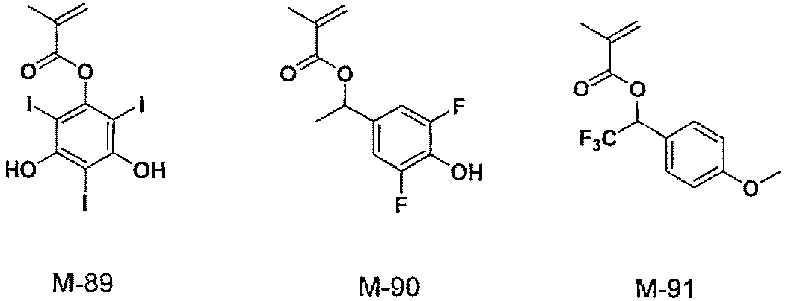

### <Synthesis Example 1: Synthesis of M-70>

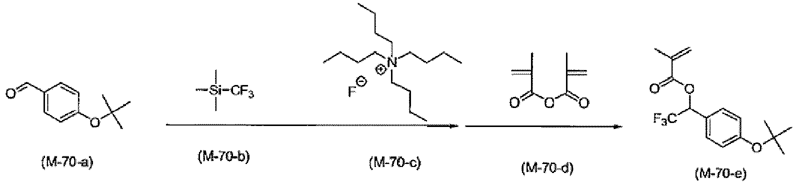

100 g of a compound represented by Formula (M-70-a) and 96 g of a compound represented by Formula (M-70-b) were dissolved in 1000 ml of tetrahydrofuran and stirred at -10°C. 28 ml of a tetrahydrofuran solution of a compound represented by Formula (M-70-c) (concentration: 1 M) was added dropwise to the obtained solution. After stirring at -10°C for 1 hour, 600 ml of a tetrahydrofuran solution of the compound represented by Formula (M-70-c) (concentration: 1 M) was further added dropwise thereto. The obtained solution was stirred at 25°C for 1 hour and cooled to 5°, and 346 g of a compound represented by Formula (M-70-d) was added dropwise thereto. The obtained solution was stirred at 50°C for 6 hours, 800 ml of saturated aqueous sodium bicarbonate was added thereto, and the mixture was further stirred for 2 hours. The obtained solution was concentrated, 1000 ml of ethyl acetate and 600 ml of 1 M hydrochloric acid water were added to the concentrated mixture, and the mixture was stirred at 25°C for 30 minutes and then separated to take out the organic layer. 600 ml of ion exchange water was added to the obtained organic layer, and the mixture was stirred at 25°C for 30 minutes and then separated to take out the organic layer. This washing operation with water was repeated 6 times. The obtained organic layer was concentrated to obtain 170 g of a compound represented by Formula (M-70-e).

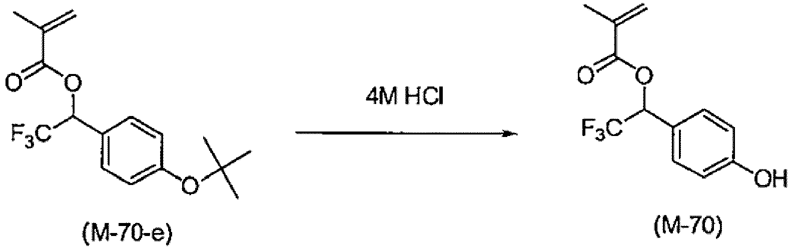

680 g of acetone and 93.5 g of a 4 M hydrochloric acid water were added to 170 g of the compound represented by Formula (M-70-e), and the mixture was stirred at 50°C for 24 hours. 2040 g of ethyl acetate was added to the obtained solution, and the mixture was stirred at 25°C for 30 minutes and then separated to take out the organic layer. 600 ml of ion exchange water was added to the obtained organic layer, and the mixture was stirred at 25°C for 30 minutes and then separated to take out the organic layer. This washing operation with water was repeated 3 times. Na2SO4 was added to the obtained organic layer, and the mixture was stirred at 25°C for 30 minutes, filtered, and concentrated. 700 g of hexane was added to the obtained solution for crystallization, and the solid was collected by filtration and dried to obtain 110 g of a compound represented by Formula (M-70). A measurement result of ¹H-NMR of the compound represented by Formula (M-70) is shown below.

¹H-NMR (CDCl₃): δ = 7.36 (m, 2H), 6.85 (m, 2H), 6.28 (s, 1H), 6.12 (q, 1H), 5.71 (m, 1H), 5.00 (s, 1H), 1.99 (s, 3H)

### <Synthesis Example 2: Synthesis of Resin A-1>

Cyclohexanone (226 g) was heated to 80°C under a nitrogen stream. While stirring this liquid, a mixed solution of a monomer (60 g) represented by Formula M-70, a monomer (40 g) represented by Formula M-31, cyclohexanone (420 g), and dimethyl 2,2'-azobisisobutyrate [V-601, manufactured by FUJIFILM Wako Pure Chemical Corporation] (12.42 g) was added dropwise thereto over 6 hours to obtain a reaction solution. After completion of dropwise addition, the reaction solution was further stirred at 80°C for 2 hours. The obtained reaction solution was cooled, then reprecipitated with a large amount of methanol/water (mass ratio: 6:4), and filtered, and the obtained solid was vacuum-dried to obtain 83 g of a resin A-1.

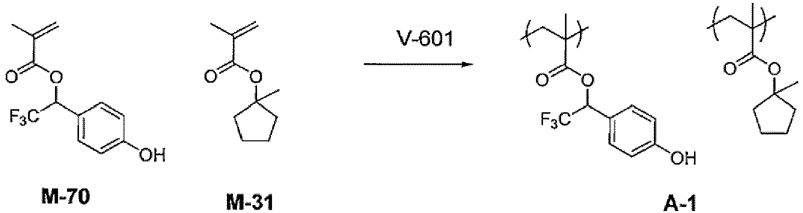

The obtained resin A-1 had a weight-average molecular weight (Mw; in terms of polystyrene) of 6500 and a dispersity (Mw/Mn) of 1.60, as determined from GPC (carrier: tetrahydrofuran (THF)). The compositional ratio measured by ¹³C-nuclear magnetic resonance (NMR) was 50/50 in a molar ratio.

Other resins were also synthesized in the same manner.

### [Photoacid Generator]

### <Photoacid Generator (B)>

Structures of photoacid generators (B) (compounds B-1 to B-39) shown in Table 3 are shown below.

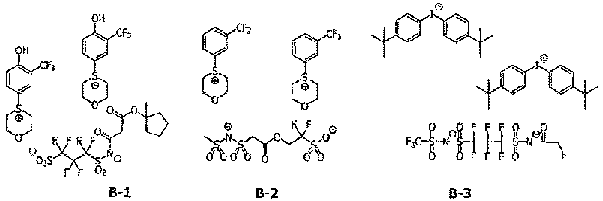

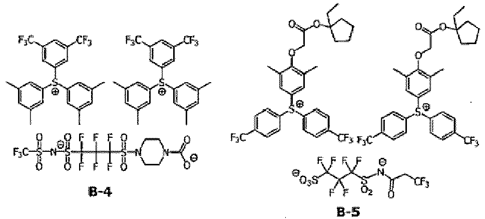

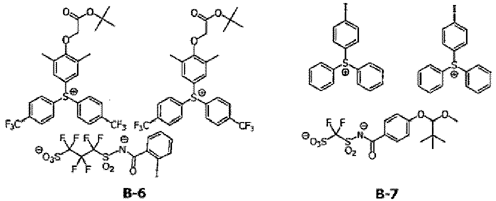

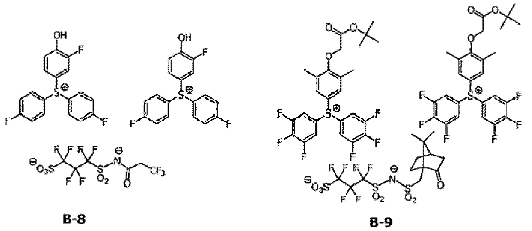

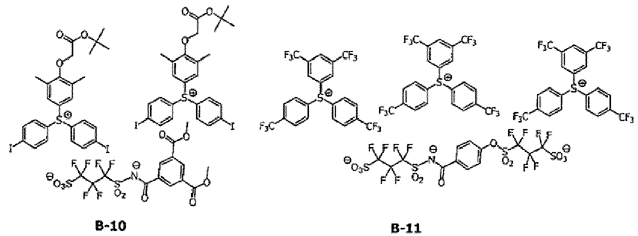

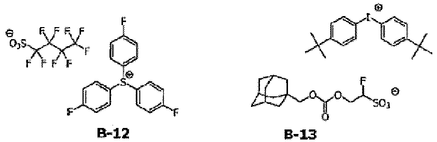

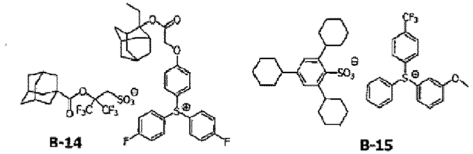

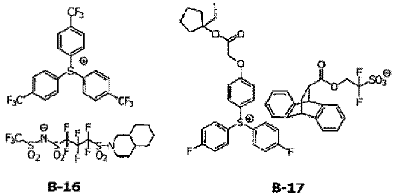

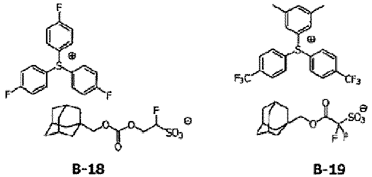

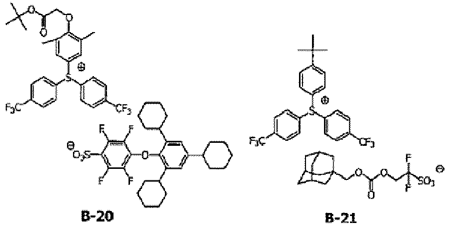

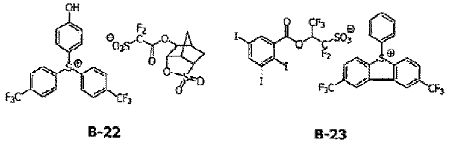

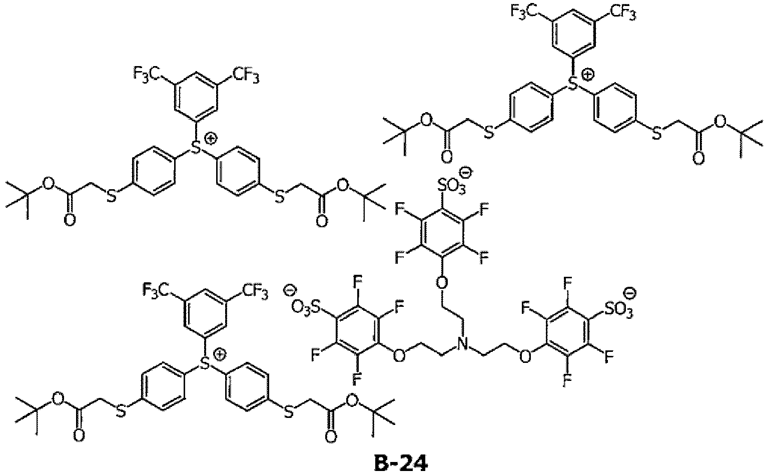

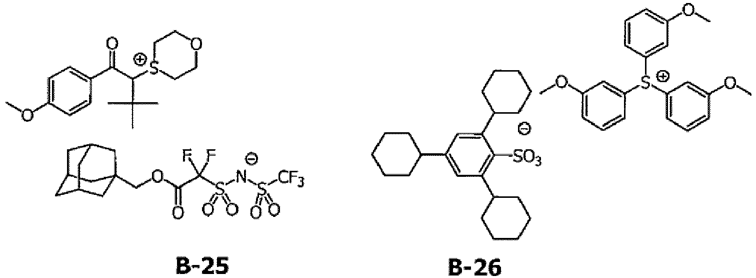

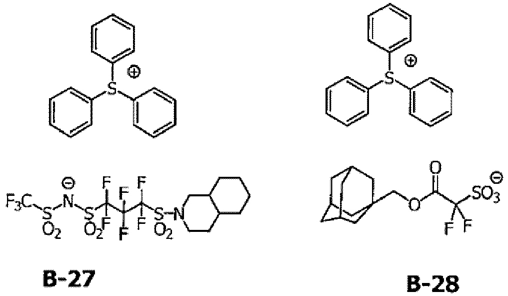

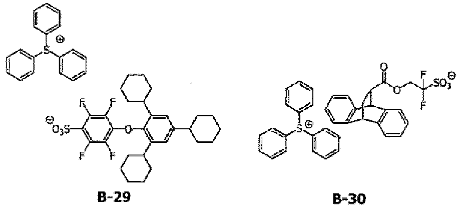

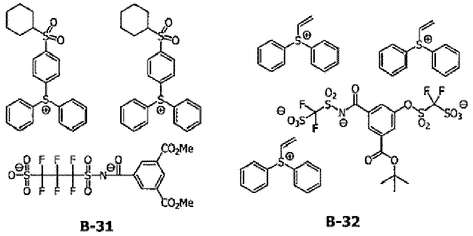

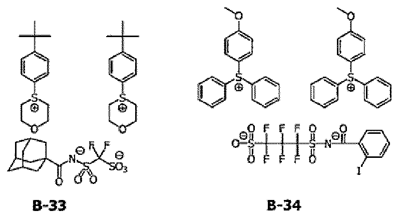

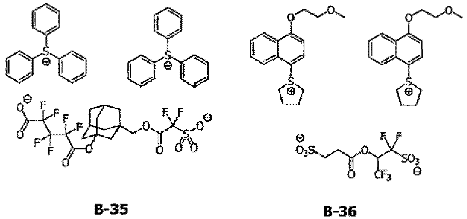

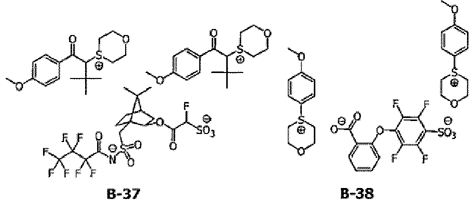

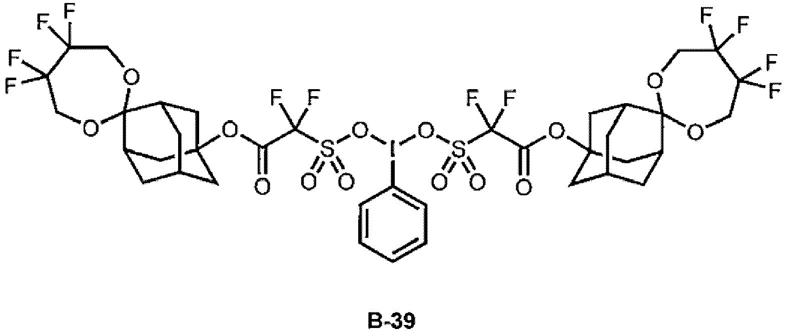

### [Photodegradable Quencher]

### <Photodegradable Quencher (C)>

Structures of photodegradable quenchers (C) (compounds C-1 to C-17) shown in Table 3 are shown below.

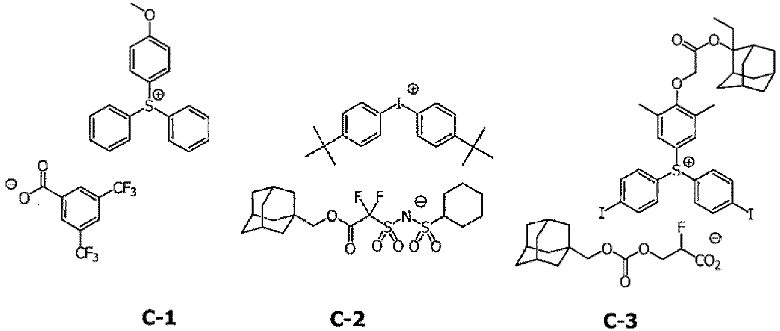

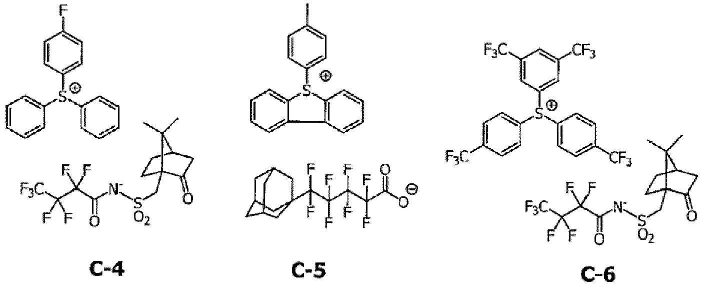

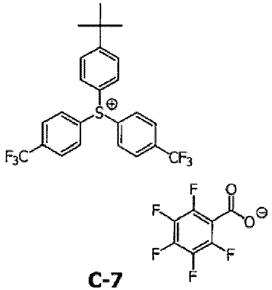

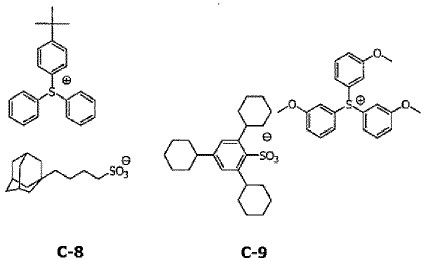

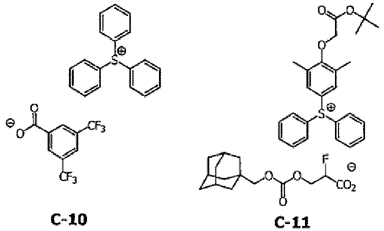

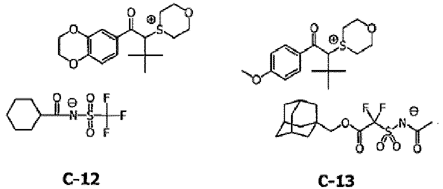

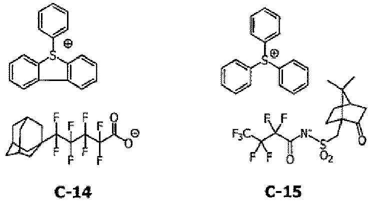

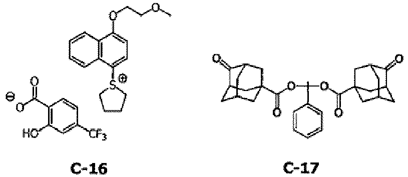

### [Photoacid Generator-linked Photodegradable Quencher]

### <Photoacid Generator-linked Photodegradable Quencher (D)>

Specific compounds of photoacid generator-linked photodegradable quencher (D) are described in the column of the photoacid generator (B) described above.

### [Acid Diffusion Control Agent]

Structures of acid diffusion control agents (E) (compounds G-1 to G-5) shown in Table 3 are shown below.

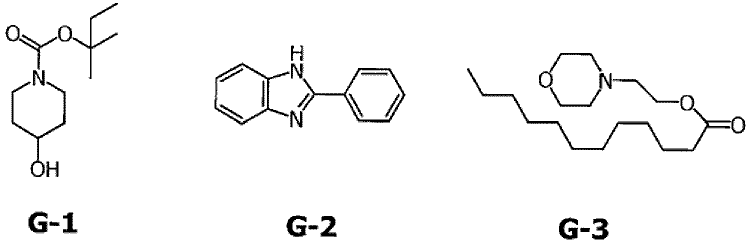

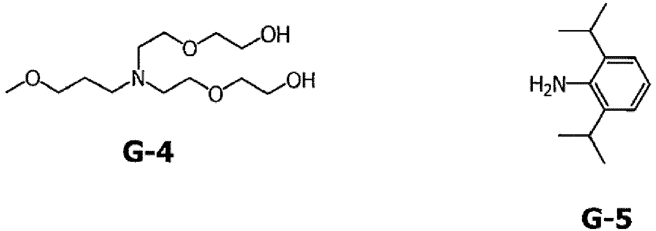

### [Resin F]

Resins (F) (resins I-1 to I-7) shown in Table 3 are shown below.

As the resins I-1 to I-7, those synthesized according to the method for synthesizing the resin A-1 (Synthesis Example 2) described above were used. A compositional ratio (ratio in % by mass; corresponding in order from the left) of each repeating unit shown below, a weight-average molecular weight (Mw), and a dispersity (Mw/Mn) are shown in Table 2.

The weight-average molecular weight (Mw) and the dispersity (Mw/Mn) of the resins I-1 to I-7 were measured by GPC (solvent carrier: tetrahydrofuran (THF)) (an amount expressed in terms of polystyrene). In addition, the compositional ratio (ratio in % by mass) of the resin was measured by means of ¹³C-nuclear magnetic resonance (NMR).

**[Table 2]**

| | Mass ratio of repeating unit | | | | | Mw | Mw/Mn |
|---|---|---|---|---|---|---|---|
| Resin I-1 | 50 | 45 | 5 | - | - | 6500 | 1.52 |
| Resin I-2 | 50 | 50 | - | - | - | 25000 | 1.65 |
| Resin I-3 | 30 | 65 | 5 | - | - | 22000 | 1.55 |
| Resin I-4 | 40 | 40 | 20 | - | - | 12000 | 1.68 |
| Resin I-5 | 40 | 50 | 5 | 5 | - | 5500 | 1.49 |
| Resin I-6 | 90 | 8 | 2 | - | - | 12000 | 1.63 |
| Resin I-7 | 20 | 30 | 40 | 10 | - | 13000 | 1.55 |

Structural formulae of the resins I-1 to I-7 shown in Table 2 are shown below.

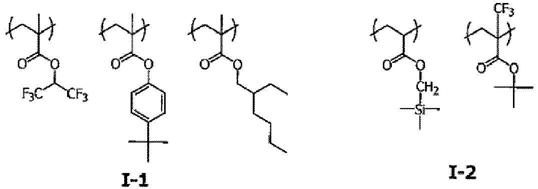

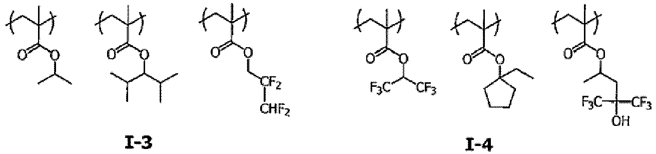

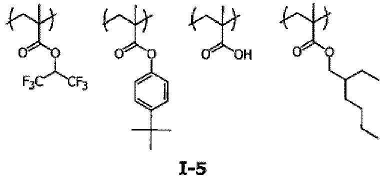

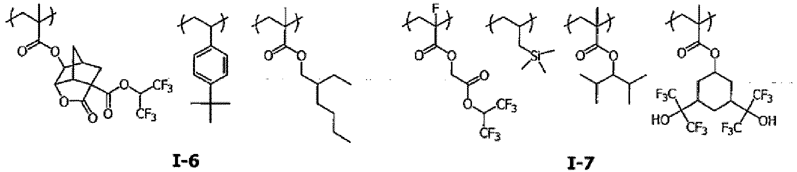

### [Surfactant]

Surfactants shown in Table 3 are shown below.
H-1: MEGAFACE F176 (manufactured by DIC Corporation, fluorine-based surfactant)
H-2: MEGAFACE R08 (manufactured by DIC Corporation, fluorine- and silicon-based surfactant)
H-3: PF656 (manufactured by OMNOVA Solutions Inc., fluorine-based surfactant)

### [Solvent]

Solvents shown in Table 3 are shown below.
F-1: Propylene glycol monomethyl ether acetate (PGMEA)
F-2: Propylene glycol monomethyl ether (PGME)
F-3: Propylene glycol monoethyl ether (PGEE)
F-4: Cyclohexanone
F-5: Cyclopentanone
F-6: 2-Heptanone
F-7: Ethyl lactate
F-8: y-Butyrolactone
F-9: Propylene carbonate

### [Preparation of Resist Composition]

The respective components shown in Table 3 were mixed so that the concentration of solid contents was 2.0% by mass. Next, the obtained mixed liquid was filtered initially through a polyethylene-made filter having a pore diameter of 50 nm, then through a nylon-made filter having a pore diameter of 10 nm, and lastly through a polyethylene-made filter having a pore diameter of 5 nm in this order to prepare resist compositions (Re-1 to Re-69 and Re'-1 and Re'-2).

The solid content means all components excluding the solvent. The obtained resist compositions were used in Examples and Comparative Examples.

In addition, in the tables, the column of "Amount" indicates a content (% by mass) of each component with respect to the total solid content in the resist composition.

**[Table 3-1]**

| Table 3 | Solid content | | | | | | | | | | | | Solvent | |
|---|---|---|---|---|---|---|---|---|---|---|---|---|---|---|
| | Resin A | | Photoacid generator B | | Photodegradable base C | | Acid diffusion control agent E | | Resin F | | Surfactant | | | |
| | Type | Amount | Type | Amount | Type | Amount | Type | Amount | Type | Amount | Type | Amount | Type | Mixed ratio (mass ratio) |
| Re-1 | A-1 | 65.0 | B-28 | 25.0 | C-10 | 10 | - | - | - | - | - | - | F-1/F-2 | 80/20 |
| Re-2 | A-2 | 60.0 | B-1 | 40.0 | - | - | - | - | - | - | - | - | F-1/F-5 | 85/15 |
| Re-3 | A-3 | 56.0 | B-2 | 44.0 | - | - | - | - | - | - | - | - | F-1/F-2 | 80/20 |
| Re-4 | A-4 | 55.0 | B-3 | 45.0 | - | - | - | - | - | - | - | - | F-1/F-2/F-8 | 40/20/40 |
| Re-5 | A-5 | 60.0 | B-4 | 40.0 | - | - | - | - | - | - | - | - | F-1/F-2 | 80/20 |
| Re-6 | A-6 | 65.0 | B-5 | 35.0 | - | - | - | - | - | - | - | - | F-I/F-9 | 85/15 |
| Re-7 | A-7 | 54.0 | B-6 | 45.9 | - | - | - | - | - | - | H-1 | 0.1 | F-1/F-7 | 90/10 |
| Re-8 | A-8 | 45.0 | B-7 | 55.0 | - | - | - | - | - | - | - | - | F-1/F-2 | 80/20 |
| Re-9 | A-9 | 50.0 | B-8 | 35.0 | C-1 | 15.0 | - | - | - | - | - | - | F-1/F-2 | 80/20 |
| Re-10 | A-10 | 50.0 | B-9 | 40.0 | C-2 | 10.0 | - | - | - | - | - | - | F-1/F-2/F-6 | 70/20/10 |
| Re-11 | A-11 | 55.0 | B-10 | 30.0 | C-3 | 15.0 | - | - | - | - | - | - | F-1/F-2 | 80/20 |
| Re-12 | A-12 | 60.0 | B-11 | 30.0 | C-4 | 10.0 | - | - | - | - | - | - | F-1/F-4 | 80/20 |
| Re-13 | A-13 | 90.0 | B-12 | 6.0 | C-5 | 20 | - | - | 1-1 | 2.0 | - | - | F-1/F-2 | 80/20 |
| Re-14 | A-14 | 70.0 | B-13 | 20.0 | C-6 | 7.0 | - | - | I-2 | 2.9 | H-2 | 0.1 | F-4 | 100 |
| Re-15 | A-15 | 65.0 | B-14 | 32.0 | - | - | G-1 | 3.0 | - | - | - | - | F-1/F-2 | 85/15 |
| Re-16 | A-16 | 80.0 | B-15 | 15.0 | - | - | G-2 | 5.0 | - | - | - | - | F-1/F-5 | 80/20 |
| Re-17 | A-17 | 63.5 | B-16 | 26.0 | C-7 | 6.5 | - | - | I-3 | 4.0 | - | - | F-1/F-2 | 75/25 |
| Re-18 | A-18 | 90.0 | B-17 | 8.0 | - | - | G-3 | 2.0 | - | - | - | - | F-1/F-2/F-8 | 34/33/33 |
| Re-19 | A-19 | 65.5 | B-18 | 20.0 | C-8 | 12.5 | - | - | I-4 | 2.0 | - | - | F-1/F-2 | 80/20 |
| Re-20 | A-20 | 88.5 | B-19 | 6.5 | - | - | G-4 | 5.0 | - | - | - | - | F-I/F-9 | 80/20 |
| Re-21 | A-21 | 74.0 | B-20 | 22.0 | - | - | G-5 | 4.0 | - | - | - | - | F-1/F-7 | 80/20 |
| Re-22 | A-22 | 80.0 | B-21 | 7.0 | C-9 | 4.0 | - | - | I-5 | 8.9 | H-3 | 0.1 | F-1/F-2 | 85/15 |
| Re-23 | A-23 | 80.0 | B-22 | 120 | C-10 | 5.0 | - | - | I-6 | 3.0 | - | - | F-1/F-2 | 80/20 |
| Re-24 | A-24 | 85.0 | B-23 | 8.0 | C-11 | 20 | - | - | I-7 | 5.0 | - | - | F-1/F-2/F-6 | 80/15/5 |
| Re-25 | A-25 | 70.0 | B-24 | 20.0 | C-12 | 10.0 | - | - | - | - | - | - | F-1/F-2 | 90/10 |

**[Table 3-2]**

| Table 3 | Solid content | | | | | | | | | | | | Solvent | |
|---|---|---|---|---|---|---|---|---|---|---|---|---|---|---|
| | Resin A | | Photoacid generator B | | Photodegradable base C | | Acid diffusion control agent E | | Resin F | | Surfactant | | | |
| | Type | Amount | Type | Amount | Type | Amount | Type | Amount | Type | Amount | Type | Amount | Type | Mixed ratio (mass ratio) |
| Re-26 | A-26 | 65.0 | B-25 | 25.0 | C-13 | 10.0 | - | - | - | - | - | - | F-1/F-4 | 80/20 |
| Re-27 | A-27 | 55.0 | B-26 | 30.0 | C-14 | 15.0 | - | - | - | - | - | - | F-1/F-2 | 80/20 |
| Re-28 | A-28 | 45.0 | B-27 | 35.0 | C-15 | 19.9 | - | - | - | - | H-3 | 0.1 | F-4 | 100 |
| Re-29 | A-29 | 40.0 | B-28 | 45.0 | C-16 | 15.0 | - | - | - | - | - | - | F-1/F-3 | 85/15 |
| Re-30 | A-30 | 75.0 | B-29 | 20.0 | C-1 | 5.0 | - | - | - | - | - | - | F-1/F-5 | 80/20 |
| Re-31 | A-31 | 75.0 | B-30 | 15.0 | C-2 | 9.9 | - | - | - | - | H-1 | 0.1 | F-1/F-2 | 75/25 |
| Re-32 | A-32 | 75.0 | B-31 | 15.0 | C-3 | 10.0 | - | - | - | - | - | - | F-1/F-2/F-8 | 40/20/40 |
| Re-33 | A-33 | 70.0 | B-32 | 15.0 | C-4 | 15.0 | - | - | - | - | - | - | F-1/F-2 | 80/20 |
| Re-34 | A-34 | 70.0 | B-33 | 20.0 | C-5 | 10.0 | - | - | - | - | - | - | F-1/F-9 | 80/20 |
| Re-35 | A-35 | 60.0 | B-34 | 30.0 | C-6 | 10.0 | - | - | - | - | - | - | F-1/F-7 | 80/20 |
| Re-36 | A-36 | 65.0 | B-35 | 35.0 | - | - | - | - | - | - | - | - | F-1/F-2 | 85/15 |
| Re-37 | A-37 | 70.0 | B-36 | 30.0 | - | - | - | - | - | - | - | - | F-1/F-2 | 90/10 |
| Re-38 | A-38 | 55.0 | B-37 | 45.0 | - | - | - | - | - | - | - | - | F-1/F-2/F-6 | 80/10/10 |
| Re-39 | A-39 | 55.0 | B-38 | 45.0 | - | - | - | - | - | - | - | - | F-1/F-2 | 90/10 |
| Re-40 | A-40 | 55.0 | B-1/B-12 | 30.0/15.0 | - | - | - | - | - | - | - | - | F-1/F-4 | 80/20 |
| Re-41 | A-41 | 60.0 | B-39 | 30.0 | C-17 | 10.0 | - | - | - | - | - | - | F-1/F-2 | 80/20 |
| Re-42 | A-42 | 65.0 | B-3 | 35.0 | - | - | - | - | - | - | - | - | F-4 | 100 |
| Re-43 | A-43 | 60.0 | B-4 | 40.0 | - | - | - | - | - | - | - | - | F-1/F-2 | 80/20 |
| Re-44 | A-44 | 70.0 | B-5 | 30.0 | - | - | - | - | - | - | - | - | F-1/F-5 | 80/20 |
| Re-45 | A-45 | 65.0 | B-6 | 35.0 | - | - | - | - | - | - | - | - | F-1/F-2 | 85/15 |
| Re-46 | A-46 | 65.0 | B-7 | 35.0 | - | - | - | - | - | - | - | - | F-1/F-2/F-8 | 80/10/10 |
| Re-47 | A-47 | 60.0 | B-8 | 40.0 | - | - | | | | | | | F-1/F-2 | 80/20 |
| Re-48 | A-48 | 55.0 | B-9 | 45.0 | - | - | - | - | - | - | - | - | F-1/F-9 | 80/20 |
| Re-49 | A-49 | 45.0 | B-10 | 55.0 | - | - | - | - | - | - | - | - | F-1/F-7 | 70/30 |
| Re-50 | A-50 | 50.0 | B-11 | 50.0 | - | - | - | - | - | - | - | - | F-1/F-2 | 90/10 |

**[Table 3-3]**

| Table 3 | Solid content | | | | | | | | | | | | Solvent | |
|---|---|---|---|---|---|---|---|---|---|---|---|---|---|---|
| | Resin A | | Photoacid generator B | | Photodegradable base C | | Acid diffusion control agent E | | Resin F | | Surfactant | | | |
| | Type | Amount | Type | Amount | Type | Amount | Type | Amount | Type | Amount | Type | Amount | Type | Mixed ratio (mass ratio) |
| Re-51 | A-51 | 55.0 | B-12 | 35.0 | C-9 | 10.0 | - | - | - | - | - | - | F-1/F-2 | 80/20 |
| Re-52 | A-52 | 70.0 | B-13 | 25.0 | C-10 | 5.0 | - | - | - | - | - | - | F-1/F-2/F-6 | 80/10/10 |
| Re-53 | A-53 | 80.0 | B-14 | 10.0 | C-11 | 10.0 | - | - | - | - | - | - | F-1/F-2 | 80/20 |
| Re-54 | A-54 | 55.0 | B-15 | 35.0 | C-12 | 10.0 | - | - | - | - | - | - | F-1/F-4 | 80/20 |
| Re-55 | A-55 | 60.0 | B-16 | 30.0 | C-13 | 10.0 | - | - | - | - | - | - | F-1/F-2 | 80/20 |
| Re-56 | A-56 | 75.0 | B-17 | 20.0 | C-14 | 5.0 | - | - | - | - | - | - | F-4 | 100 |
| Re-57 | A-57 | 75.0 | B-18 | 15.0 | C-15 | 10.0 | - | - | - | - | - | - | F-1/F-2 | 80/20 |
| Re-58 | A-58 | 65.0 | B-19 | 30.0 | C-16 | 5.0 | - | - | - | - | - | - | F-1/F-5 | 80/20 |
| Re-59 | A-59 | 80.0 | B-20 | 15.0 | C-7 | 5.0 | - | - | - | - | - | - | F-1/F-2 | 80/20 |
| Re-60 | A-60 | 65.0 | B-21 | 25.0 | C-8 | 10.0 | - | - | - | - | - | - | F-1/F-2/F-8 | 34/33/33 |
| Re-61 | A-61 | 45.0 | B-22 | 45.0 | C-1 | 10.0 | - | - | - | - | - | - | F-1/F-2 | 80/20 |
| Re-62 | A-62 | 65.0 | B-23 | 30.0 | C-2 | 5.0 | - | - | - | - | - | - | F-1/F-2 | 85/15 |
| Re-63 | A-63 | 25.0 | B-24 | 70.0 | C-3 | 5.0 | - | - | - | - | - | - | F-1/F-7 | 90/10 |
| Re-64 | A-64 | 55.0 | B-25 | 40.0 | C-4 | 5.0 | - | - | - | - | - | - | F-1/F-2 | 80/20 |
| Re-65 | A-65 | 65.0 | B-26 | 30.0 | C-5 | 5.0 | - | - | - | - | - | - | F-1/F-2 | 80/20 |
| Re-66 | A-66 | 55.0 | B-27 | 30.0 | C-6 | 15.0 | | | | | | | F-1/F-7 | 90/10 |
| Re-67 | A-67 | 70.0 | B-28 | 20.0 | C-7 | 10.0 | | | | | | | F-1/F-2 | 80/20 |
| Re-68 | A-68 | 55.0 | B-29 | 30.0 | C-8 | 15.0 | | | | | | | F-1/F-2 | 80/20 |
| Re-69 | A-69 | 55.0 | B-29 | 30.0 | C-8 | 15.0 | | | | | | | F-1/F-2 | 80/20 |
| Re'-1 | A'-1 | 65.0 | B-28 | 25.0 | C-10 | 10.0 | - | - | - | - | - | - | F-1/F-2 | 80/20 |
| Re'-2 | A'-2 | 65.0 | B-28 | 25.0 | C-10 | 10.0 | - | - | - | - | - | - | F-1/F-2 | 80/20 |

### [Pattern Formation]

### [EUV Exposure and Organic Solvent Development]

A composition for forming an underlayer film, AL412 (manufactured by Brewer Science, Inc.), was applied to a silicon wafer having a diameter of 12 inches and baked at 205°C for 60 seconds to form an underlying film having a film thickness of 20 nm. A resist composition shown in Table 3 was applied thereto and baked at 100°C for 60 seconds to form a resist film having a film thickness of 30 nm.

Using an EUV exposure device (manufactured by Exitech Ltd., Micro Exposure Tool, NA 0.3, Quadrupole, outer sigma: 0.68, inner sigma: 0.36), the silicon wafer having the obtained resist film was subjected to a pattern irradiation so that an average line width of the obtained pattern was 20 nm. As a reticle, a mask having a line size = 20 nm and a line:space = 1:1 was used.

The resist film after the exposure was baked at 90°C for 60 seconds, developed with n-butyl acetate for 30 seconds, and spin-dried to obtain a negative tone pattern.

### [Evaluation]

### <Defect Evaluation (Defect Suppression Property)>

Using UVision5 (manufactured by Applied Materials, Inc.) and SEMVisionG4 (manufactured by Applied Materials, Inc.), the pattern obtained by the above-described method was evaluated according to the following evaluation standard by counting the number of defects per one silicon wafer. As the number of defects is smaller, defect suppression property is better.
"A": The number of defects was 50 or less.
"B": The number of defects was more than 50 and 100 or less.
"C": The number of defects was more than 100 and 200 or less.
"D": The number of defects was more than 200 and 300 or less.
"E": The number of defects was more than 300 and 400 or less.
"F": The number of defects was more than 400 and 500 or less.
"G": The number of defects was more than 500 and 600 or less.

### <Roughness Performance (LWR Performance, nm)>

The pattern obtained by the above-described method was observed from an upper part of the pattern using a length-measuring scanning electron microscope (SEM (S-9380II of Hitachi, Ltd.)). A line width of the pattern was observed at 250 points, and a measurement variation thereof was evaluated with 3σ and taken as a line width roughness (LWR) (nm). As the value of LWR is smaller, the roughness performance is better.

In addition, the LWR performance (nm) was preferably 4.1 nm or less, 3.8 nm or less, 3.5 nm or less, and 3.2 nm or less in this order.

### The evaluation results are shown in Table 4 below.

**[Table 4-1]**

| Table 4 | Resist composition | Evaluation item 1 defect suppression property | Evaluation item 2 roughness performance (nm) |
|---|---|---|---|
| Example 1-1 | Re-1 | A | 3.2 |
| Example 1-2 | Re-2 | A | 3.1 |
| Example 1-3 | Re-3 | A | 3.1 |
| Example 1-4 | Re-4 | A | 3.0 |
| Example 1-5 | Re-5 | A | 2.9 |
| Example 1-6 | Re-6 | A | 3.0 |
| Example 1-7 | Re-7 | A | 2.8 |
| Example 1-8 | Re-8 | A | 2.8 |
| Example 1-9 | Re-9 | A | 2.9 |
| Example 1-10 | Re-10 | A | 3.0 |
| Example 1-11 | Re-11 | A | 3.1 |
| Example 1-12 | Re-12 | A | 2.9 |
| Example 1-13 | Re-13 | A | 2.8 |
| Example 1-14 | Re-14 | A | 2.8 |
| Example 1-15 | Re-15 | A | 2.8 |
| Example 1-16 | Re-16 | C | 3.6 |
| Example 1-17 | Re-17 | B | 3.4 |
| Example 1-18 | Re-18 | B | 3.3 |
| Example 1-19 | Re-19 | B | 3.4 |
| Example 1-20 | Re-20 | B | 3.3 |
| Example 1-21 | Re-21 | B | 3.3 |
| Example 1-22 | Re-22 | B | 3.3 |
| Example 1-23 | Re-23 | B | 3.4 |
| Example 1-24 | Re-24 | B | 3.4 |
| Example 1-25 | Re-25 | A | 2.9 |
| Example 1-26 | Re-26 | A | 3.2 |
| Example 1-27 | Re-27 | A | 3.2 |
| Example 1-28 | Re-28 | B | 3.5 |
| Example 1-29 | Re-29 | B | 3.4 |
| Example 1-30 | Re-30 | B | 3.5 |
| Example 1-31 | Re-31 | B | 3.3 |
| Example 1-32 | Re-32 | C | 3.6 |
| Example 1-33 | Re-33 | B | 3.3 |
| Example 1-34 | Re-34 | B | 3.5 |
| Example 1-35 | Re-35 | B | 3.3 |

**[Table 4-2]**

| Table 4 | Resist composition | Evaluation item 1 defect suppression property | Evaluation item 2 roughness performance (nm) |
|---|---|---|---|
| Example 1-36 | Re-36 | A | 3.2 |
| Example 1-37 | Re-37 | A | 3.2 |
| Example 1-38 | Re-38 | A | 3.2 |
| Example 1-39 | Re-39 | A | 3.2 |
| Example 1-40 | Re-40 | A | 3.0 |
| Example 1-41 | Re-41 | B | 3.5 |
| Example 1-42 | Re-42 | A | 3.0 |
| Example 1-43 | Re-43 | A | 2.9 |
| Example 1-44 | Re-44 | A | 3.0 |
| Example 1-45 | Re-45 | A | 3.0 |
| Example 1-46 | Re-46 | A | 2.9 |
| Example 1-47 | Re-47 | A | 2.8 |
| Example 1-48 | Re-48 | B | 3.3 |
| Example 1-49 | Re-49 | A | 2.8 |
| Example 1-50 | Re-50 | A | 3.0 |
| Example 1-51 | Re-51 | A | 2.9 |
| Example 1-52 | Re-52 | A | 2.8 |
| Example 1-53 | Re-53 | A | 2.9 |
| Example 1-54 | Re-54 | A | 3.0 |
| Example 1-55 | Re-55 | A | 3.0 |
| Example 1-56 | Re-56 | A | 3.0 |
| Example 1-57 | Re-57 | A | 2.9 |
| Example 1-58 | Re-58 | A | 2.9 |
| Example 1-59 | Re-59 | A | 3.0 |
| Example 1-60 | Re-60 | A | 2.9 |
| Example 1-61 | Re-61 | A | 3.0 |
| Example 1-62 | Re-62 | A | 3.0 |
| Example 1-63 | Re-63 | A | 3.0 |
| Example 1-64 | Re-64 | B | 3.3 |
| Example 1-65 | Re-65 | A | 3.2 |
| Example 1-66 | Re-66 | A | 3.0 |
| Example 1-67 | Re-67 | A | 3.1 |
| Example 1-68 | Re-68 | A | 3.2 |
| Example 1-69 | Re-69 | A | 3.2 |
| Comparative Example 1-1 | Re'-1 | G | 4.9 |
| Comparative Example 1-2 | Re'-2 | G | 5.1 |

As shown in Table 4 above, it was confirmed that the resist composition according to the embodiment of the present invention has excellent roughness performance and can suppress the occurrence of pattern defects in a case where a pattern is formed by the organic solvent development. On the other hand, the resist compositions of Comparative Examples were insufficient in these performances.

A resist composition Re-70 was produced in the same manner as in Example 1-1, except that the resin A-1 used in the resist composition Re-1 was changed to an equivalent amount of the resin A-1 and the resin A-2 (specifically, the mass ratio of the resin A-1 and the resin A-2 was 1:1, and the total "amount" thereof was 65 parts). As a result of performing the same evaluations as in Example 1-1, the same results as in Example 1-1 were obtained.

A resist composition Re-71 was produced in the same manner as in Example 1-1, except that the resin A-1 used in the resist composition Re-1 was changed to a mixture of the resin A-1 and the resin A'-1 (specifically, (Resin A-1):(Resin A'-1) = 9:1 (mass ratio), and the total "amount" thereof was 65 parts). As a result of performing the same evaluations as in Example 1-1, the same results as in Example 1-1 were obtained.

### [EUV Exposure and Aqueous Alkali Solution Development]

A composition for forming an underlayer film, AL412 (manufactured by Brewer Science, Inc.), was applied to a silicon wafer having a diameter of 12 inches and baked at 205°C for 60 seconds to form an underlying film having a film thickness of 20 nm. A resist composition shown in Table 4 was applied thereto and baked at 100°C for 60 seconds to form a resist film having a film thickness of 30 nm.

Using an EUV exposure device (manufactured by Exitech Ltd., Micro Exposure Tool, NA 0.3, Quadrupole, outer sigma: 0.68, inner sigma: 0.36), the silicon wafer having the obtained resist film was subjected to a pattern irradiation so that an average line width of the obtained pattern was 20 nm. As a reticle, a mask having a line size = 20 nm and a line:space = 1:1 was used.

The resist film after the exposure was baked at 90°C for 60 seconds, developed with an aqueous tetramethylammonium hydroxide solution (2.38% by mass) for 30 seconds, and then rinsed with pure water for 30 seconds. Thereafter, the resist film was spin-dried to obtain a positive tone pattern.

Using the obtained positive tone pattern, the occurrence of pattern defects and the roughness performance were evaluated in the same manner as described above.

The evaluation results are shown in Table 5 below.

**[Table 5-1]**

| Table 5 | Resist composition | Evaluation item 1 defect suppression property | Evaluation item 2 roughness performance (nm) |
|---|---|---|---|
| Example 2-1 | Re-1 | A | 3.2 |
| Example 2-2 | Re-2 | A | 3.1 |
| Example 2-3 | Re-3 | A | 3.0 |
| Example 2-4 | Re-4 | A | 3.0 |
| Example 2-5 | Re-5 | A | 3.0 |
| Example 2-6 | Re-6 | A | 2.9 |
| Example 2-7 | Re-7 | A | 2.8 |
| Example 2-8 | Re-8 | A | 2.9 |
| Example 2-9 | Re-9 | A | 3.0 |
| Example 2-10 | Re-10 | A | 3.0 |
| Example 2-11 | Re-11 | A | 3.1 |
| Example 2-12 | Re-12 | A | 2.9 |
| Example 2-13 | Re-13 | A | 2.8 |
| Example 2-14 | Re-14 | A | 2.8 |
| Example 2-15 | Re-15 | A | 2.8 |
| Example 2-16 | Re-16 | C | 3.6 |
| Example 2-17 | Re-17 | B | 3.4 |
| Example 2-18 | Re-18 | B | 3.3 |
| Example 2-19 | Re-19 | B | 3.4 |
| Example 2-20 | Re-20 | B | 3.3 |
| Example 2-21 | Re-21 | B | 3.4 |
| Example 2-22 | Re-22 | B | 3.3 |
| Example 2-23 | Re-23 | B | 3.4 |
| Example 2-24 | Re-24 | B | 3.4 |
| Example 2-25 | Re-25 | A | 2.9 |
| Example 2-26 | Re-26 | A | 3.2 |
| Example 2-27 | Re-27 | A | 3.2 |
| Example 2-28 | Re-28 | B | 3.5 |
| Example 2-29 | Re-29 | B | 3.4 |
| Example 2-30 | Re-30 | B | 3.5 |
| Example 2-31 | Re-31 | B | 3.3 |
| Example 2-32 | Re-32 | C | 3.6 |
| Example 2-33 | Re-33 | B | 3.3 |
| Example 2-34 | Re-34 | B | 3.5 |
| Example 2-35 | Re-35 | B | 3.3 |

**[Table 5-2]**

| Table 5 | Resist composition | Evaluation item 1 defect suppression property | Evaluation item 2 roughness performance (nm) |
|---|---|---|---|
| Example 2-36 | Re-36 | A | 3.2 |
| Example 2-37 | Re-37 | A | 3.2 |
| Example 2-38 | Re-38 | A | 3.2 |
| Example 2-39 | Re-39 | A | 3.2 |
| Example 2-40 | Re-40 | A | 3.0 |
| Example 2-41 | Re-41 | B | 3.5 |
| Example 2-42 | Re-42 | A | 3.0 |
| Example 2-43 | Re-43 | A | 2.9 |
| Example 2-44 | Re-44 | A | 3.0 |
| Example 2-45 | Re-45 | A | 3.0 |
| Example 2-46 | Re-46 | A | 2.8 |
| Example 2-47 | Re-47 | A | 3.0 |
| Example 2-48 | Re-48 | B | 3.3 |
| Example 2-49 | Re-49 | A | 2.8 |
| Example 2-50 | Re-50 | A | 3.0 |
| Example 2-51 | Re-51 | A | 2.9 |
| Example 2-52 | Re-52 | A | 2.8 |
| Example 2-53 | Re-53 | A | 2.9 |
| Example 2-54 | Re-54 | A | 2.9 |
| Example 2-55 | Re-55 | A | 3.0 |
| Example 2-56 | Re-56 | A | 3.0 |
| Example 2-57 | Re-57 | A | 2.9 |
| Example 2-58 | Re-58 | A | 2.9 |
| Example 2-59 | Re-59 | A | 3.0 |
| Example 2-60 | Re-60 | A | 3.0 |
| Example 2-61 | Re-61 | A | 3.0 |
| Example 2-62 | Re-62 | A | 3.0 |
| Example 2-63 | Re-63 | A | 3.0 |
| Example 2-64 | Re-64 | B | 3.3 |
| Example 2-65 | Re-65 | A | 3.2 |
| Example 2-66 | Re-66 | A | 3.0 |
| Example 2-67 | Re-67 | A | 3.0 |
| Example 2-68 | Re-68 | A | 3.2 |
| Example 2-69 | Re-69 | A | 3.2 |
| Comparative Example 2-1 | Re'-1 | G | 5.0 |
| Comparative Example 2-2 | Re'-2 | G | 4.9 |

As shown in Table 5 above, it was confirmed that the resist composition according to the embodiment of the present invention has excellent roughness performance and can suppress the occurrence of pattern defects even in a case of forming a pattern by the alkali development. On the other hand, the resist compositions of Comparative Examples were insufficient in these performances.

In a case where the above-described resist composition Re-1 was changed to the above-described resist composition Re-70 and the same evaluations as in Example 2-1 were performed, the same results as in Example 2-1 were obtained.

In a case where the above-described resist composition Re-1 was changed to the above-described resist composition Re-71 and the same evaluations as in Example 2-1 were performed, the same results as in Example 2-1 were obtained.

## Claims

1. An actinic ray-sensitive or radiation-sensitive resin composition comprising:
(A) a resin which has a repeating unit derived from a compound represented by the following Formula (1); and
(B) a compound which generates an acid by irradiation with an actinic ray or a radiation, 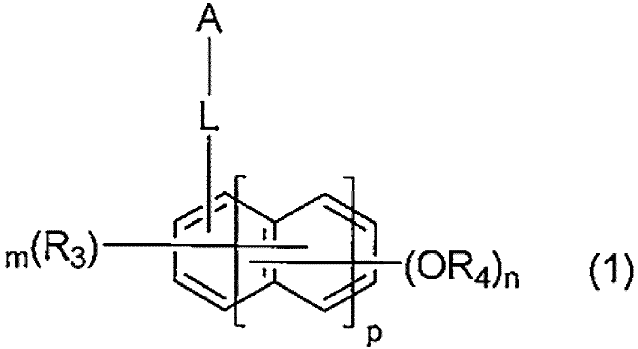 wherein in the Formula (1),
A represents a polymerizable group,
L represents a divalent linking group having a halogen atom,
R₃ represents a halogen atom, an amino group, a thiol group, or an organic group,
R₄ represents a hydrogen atom or an organic group,
n represents an integer of 1 or more, m represents an integer of 0 or more, and p represents an integer of 0 or more, n, m, and p satisfy a relationship of n + m ≤ 5 + 2p, and
in a case where m represents an integer of 2 or more, a plurality of R₃'s may be the same or different from each other, and in a case where n represents an integer of 2 or more, a plurality of R₄'s may be the same or different from each other.

2. The actinic ray-sensitive or radiation-sensitive resin composition according to claim 1,
wherein the compound represented by the Formula (1) is a compound represented by the following Formula (2), 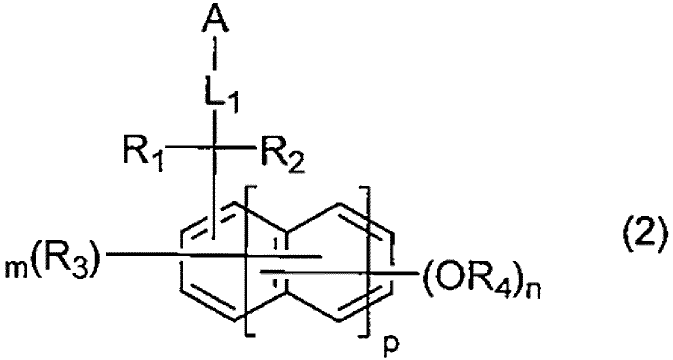 in the Formula (2),
A has the same meaning as A in the Formula (1),
L₁ represents a single bond or a divalent linking group,
R₁ represents a halogen atom or an organic group having a halogen atom,
R₂ represents a hydrogen atom, a halogen atom, a hydroxy group, an amino group, a thiol group, or an organic group, and
R₃, R₄, n, m, and p have the same meanings as R₃, R₄, n, m, and p in the Formula (1), respectively.

3. The actinic ray-sensitive or radiation-sensitive resin composition according to claim 2,
wherein the compound represented by the Formula (2) is a compound represented by the following Formula (3), 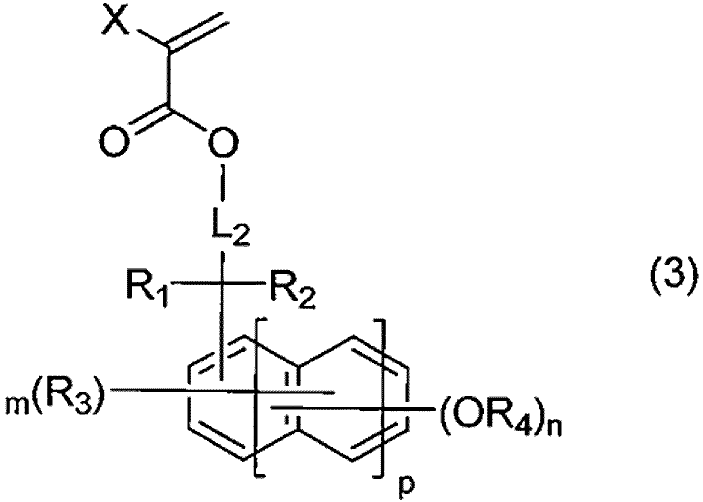 in the Formula (3),
X represents a hydrogen atom or an organic group,
R₁ and R₂ have the same meanings as R₁ and R₂ in the Formula (2), respectively,
L₂ represents a single bond or a divalent linking group, and
R₃, R₄, n, m, and p have the same meanings as R₃, R₄, n, m, and p in the Formula (1), respectively.

4. The actinic ray-sensitive or radiation-sensitive resin composition according to any one of claims 1 to 3,
wherein the compound (B) is an onium salt.

5. The actinic ray-sensitive or radiation-sensitive resin composition according to any one of claims 1 to 4,
wherein the resin (A) further has a repeating unit represented by the following Formula (4), 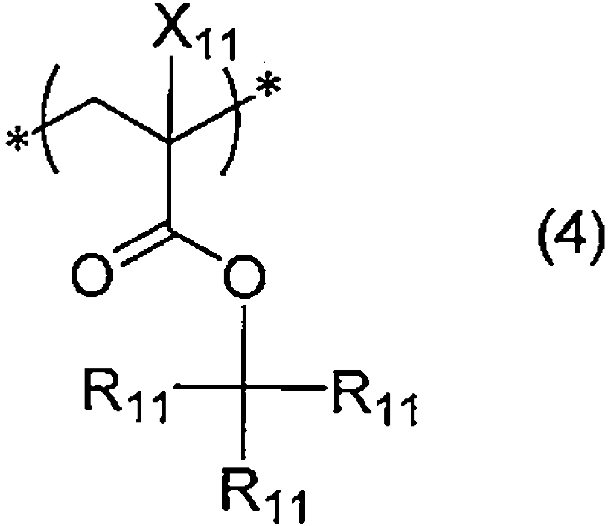 in the Formula (4),
X₁₁ represents a hydrogen atom, a halogen atom, a hydroxy group, or an organic group, and
R₁₁'s each independently represent an alkyl group, and two R₁₁'s may be bonded to each other to form a ring.

6. An actinic ray-sensitive or radiation-sensitive film formed of the actinic ray-sensitive or radiation-sensitive resin composition according to any one of claims 1 to 5.

7. A pattern forming method comprising:
a step of forming an actinic ray-sensitive or radiation-sensitive film on a substrate using the actinic ray-sensitive or radiation-sensitive resin composition according to any one of claims 1 to 5;
a step of exposing the actinic ray-sensitive or radiation-sensitive film; and
a step of developing the exposed actinic ray-sensitive or radiation-sensitive film with a developer to form a pattern.

8. A method for manufacturing an electronic device, comprising:
the pattern forming method according to claim 7.

9. A compound represented by the following Formula (2), 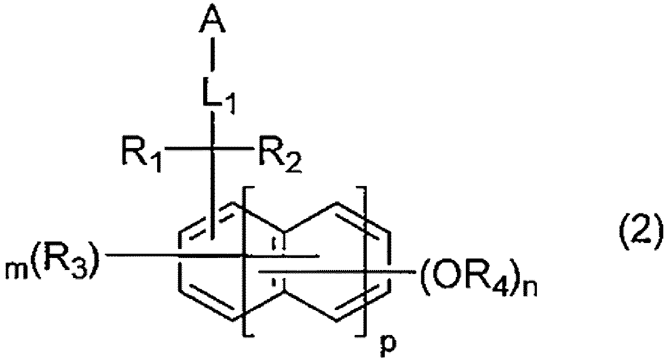 wherein in the Formula (2),
A represents a polymerizable group,
L₁ represents a single bond or a divalent organic group,
R₁ represents a halogen atom or an organic group having a halogen atom,
R₂ represents a hydrogen atom, a halogen atom, a hydroxy group, an amino group, a thiol group, or an organic group,
R₃ represents a halogen atom, an amino group, a thiol group, or an organic group,
R₄ represents a hydrogen atom or an organic group,
n represents an integer of 1 or more, m represents an integer of 0 or more, and p represents an integer of 0 or more, n, m, and p satisfy a relationship of n + m ≤ 5 + 2p, and
in a case where m represents an integer of 2 or more, a plurality of R₃'s may be the same or different from each other, and in a case where n represents an integer of 2 or more, a plurality of R₄'s may be the same or different from each other.

10. The compound according to claim 9,
wherein the compound represented by the Formula (2) is a compound represented by the following Formula (3), 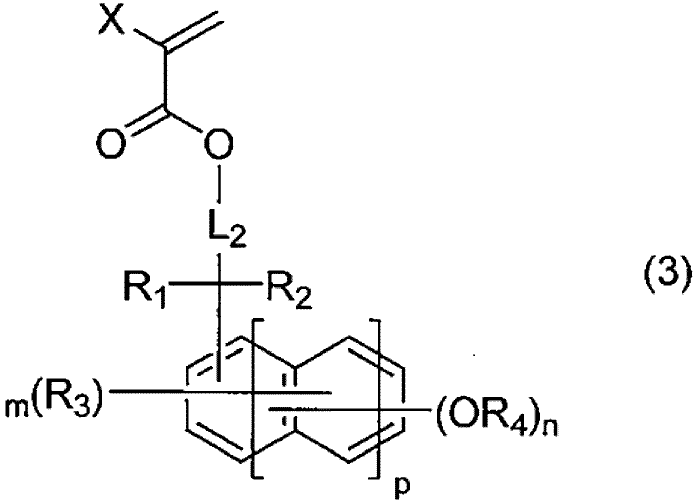 in the Formula (3),
X represents a hydrogen atom or an organic group,
R₁ and R₂ have the same meanings as R₁ and R₂ in the Formula (2), respectively,
L₂ represents a single bond or a divalent linking group, and
R₃, R₄, n, m, and p have the same meanings as R₃, R₄, n, m, and p in the Formula (2), respectively.

11. A resin having a repeating unit derived from the compound according to claim 9 or 10.
